# EUROPEAN PATENT SPECIFICATION

(11) **EP 3 505 632 B1**
(45) Date of publication and mention of the grant of the patent: **03.08.2022**
(21) Application number: 18205786.9
(22) Date of filing: 28.12.2010
(51) Int. Cl.: A61P 31/12, A61P 31/16, A61P 37/04, C12N 1/12, C12P 21/02

(54) **PRODUCTION OF HETEROLOGOUS POLYPEPTIDES IN MICROALGAE, MICROALGAL EXTRACELLULAR BODIES, COMPOSITIONS, AND METHODS OF MAKING AND USES THEREOF**
HERSTELLUNG HETEROLOGER POLYPEPTIDE IN MIKROALGEN, EXTRAZELLULÄRE ALGENKÖRPER, ZUSAMMENSETZUNGEN UND VERFAHREN ZUR HERSTELLUNG SOWIE VERWENDUNGEN DAVON
PRODUCTION DE POLYPEPTIDES HÉTÉROLOGUES DANS DES MICRO-ALGUES, CORPS EXTRACELLULAIRE DE MICRO-ALGUES, COMPOSITIONS ET PROCÉDÉS DE FABRICATION ET LEURS UTILISATIONS

(30) Priority: 28.12.2009 US 29046909 P; 28.12.2009 US 29044109 P; 12.11.2010 US 41335310 P
(43) Date of publication of application: 03.07.2019
(62) Divisional of application: 10844276.5
(73) Proprietor: Sanofi Vaccine Technologies, S.A.S., 69007 Lyon (FR)
(72) Inventor: BAYNE, Anne-Cecile V., Ellicott City, MD 21043 (US); LIPPMEIER, James Casey, Columbia, MD 21045 (US); APT, Kirk Emil, deceased (US); ZIRKLE, Ross Eric, Mt. Airy, MD 21771 (US)
(74) Representative: Wilding, James Roger

(56) References cited:
- EP-A1- 2 090 648
- WO-A1-2005/047501
- WO-A1-2009/009876
- WO-A2-02/083869
- WO-A2-2006/013572
- WO-A2-2008/024844
- US-A1- 2006 275 904
- US-A1- 2006 286 650
- US-A1- 2008 022 422
- NWE NITAR ET AL: "Expression of hemagglutinin protein from the avian influenza virus H5N1 in a baculovirus/insect cell system significantly enhanced by suspension culture", BMC MICROBIOLOGY, BIOMED CENTRAL LTD, GB, vol. 6, no. 1, 24 February 2006 (2006-02-24), page 16, XP021014845, ISSN: 1471-2180, DOI: 10.1186/1471-2180-6-16
- MENG SUN ET AL: "Foot-and-mouth disease virus VP1 protein fused with cholera toxin B subunit expressed in Chlamydomonas reinhardtii chloroplast.", BIOTECHNOLOGY LETTERS, vol. 25, no. 13, 1 July 2003 (2003-07-01), pages 1087-1092, XP055062669, ISSN: 0141-5492
- SIRIPORNADULSIL SURASAK ET AL: "Microalgal vaccines", ADVANCES IN EXPERIMENTAL MEDICINE AND BIOLOGY,, 1 January 2007 (2007-01-01), pages 122-128, XP009097595, ISSN: 0065-2598, DOI: 10.1007/978-0-387-75532-8_11
- PRASAD V ET AL: "Expression of biologically active Hemagglutinin-neuraminidase protein of Peste des petits ruminants virus in transgenic pigeonpea [Cajanus cajan (L) Millsp.]", PLANT SCI, ELSEVIER IRELAND LTD, IE, vol. 166, no. 1, 1 January 2004 (2004-01-01), pages 199-205, XP008161290, ISSN: 0168-9452, DOI: 10.1016/J.PLANTSCI.2003.08.020
- GOMEZ E ET AL: "Expression of Hemagglutinin-Neuraminidase glycoprotein of Newcastle Disease Virus in agroinfiltrated Nicotiana benthamiana plants", JOURNAL OF BIOTECHNOLOGY, ELSEVIER, AMSTERDAM, NL, vol. 144, no. 4, 1 December 2009 (2009-12-01), pages 337-340, XP026778485, ISSN: 0168-1656, DOI: 10.1016/J.JBIOTEC.2009.09.015 [retrieved on 2009-09-30]
- TANABAYASHI K ET AL: "Expression of mumps virus glycoproteins in mammalian cells from cloned cDNAs: Both F and HN proteins are required for cell fusion", VIROLOGY, ELSEVIER, AMSTERDAM, NL, vol. 187, no. 2, 1 April 1992 (1992-04-01) , pages 801-804, XP026462300, ISSN: 0042-6822, DOI: 10.1016/0042-6822(92)90482-5 [retrieved on 1992-04-01]
- ANNE-CÉCILE V. BAYNE ET AL: "Vaccination against Influenza with Recombinant Hemagglutinin Expressed by Schizochytrium sp. Confers Protective Immunity", PLOS ONE, vol. 8, no. 4, 1 January 2013 (2013-01-01) , pages e61790-e61790, XP055062273, ISSN: 1932-6203, DOI: 10.1371/journal.pone.0061790
- DATABASE BIOSIS [Online] BIOSCIENCES INFORMATION SERVICE, PHILADELPHIA, PA, US; 22 July 2010 (2010-07-22), AGUILAR-YANES JOSE M ET AL: "An Influenza A/H1N1/2009 Hemagglutinin Vaccine Produced in Escherichia coli", Database accession no. PREV201000479780 & PLOS ONE, vol. 5, no. 7, 22 July 2010 (2010-07-22), page Article No.: e11694, ISSN: 1932-6203(print), DOI: 10.1371/JOURNAL.PONE.0011694

### SEQUENCE LISTING

<110> Sanofi Vaccine Technologies, S.A.S.
<120> Production of Heterologous Polypeptides in Microalgae, Microalgal
   Extracellular Bodies, Compositions, and Methods of Making and Uses Thereof
<130> 2715.238PC01/JUK/SAS/BNC
<140> To Be Assigned
   <141> Herewith
<150> 61/413,353
   <151> 2010-11-12
<150> 61/290,469
   <151> 2009-12-28
<150> 61/290,441
   <151> 2009-12-28
<160> 103
<170> PatentIn version 3.5
<210> 1
   <211> 35
   <212> PRT
   <213> Schizochytrium
<400> 1
   Met Ala Asn Ile Met Ala Asn Val Thr Pro Gln Gly Val Ala Lys Gly
   1               5                   10                  15
   Phe Gly Leu Phe Val Gly Val Leu Phe Phe Leu Tyr Trp Phe Leu Val
               20                  25                  30
   Gly Leu Ala
           35
<210> 2
   <211> 105
   <212> DNA
   <213> Schizochytrium
<400> 2
   atggccaaca tcatggccaa cgtcacgccc cagggcgtcg ccaagggctt tggcctcttt      60
   gtcggcgtgc tcttctttct ctactggttc cttgtcggcc tcgcc                     105
<210> 3
   <211> 1999
   <212> DNA
   <213> Schizochytrium
<400> 3
   ccgcgaatca agaaggtagg cgcgctgcga ggcgcggcgg cggagcggag cgagggagag      60
   ggagagggag agagagggag ggagacgtcg ccgcggcggg gcctggcctg gcctggtttg     120
   gcttggtcag cgcggccttg tccgagcgtg cagctggagt tgggtggatt catttggatt     180
   ttcttttgtt tttgtttttc tctctttccc ggaaagtgtt ggccggtcgg tgttctttgt     240
   tttgatttct tcaaaagttt tggtggttgg ttctctctct tggctctctg tcaggcggtc     300
   cggtccacgc cccggcctct cctctcctct cctctcctct cctctccgtg cgtatacgta     360
   cgtacgtttg tatacgtaca tacatcccgc ccgccgtgcc ggcgagggtt tgctcagcct     420
   ggagcaatgc gatgcgatgc gatgcgatgc gacgcgacgc gacgcgagtc actggttcgc     480
   gctgtggctg tggcttgctt gcttacttgc tttcgagctc tcccgctttc ttctttcctt     540
   ctcacgccac caccaacgaa agaagatcgg ccccggcacg ccgctgagaa gggctggcgg     600
   cgatgacggc acgcgcgccc gctgccacgt tggcgctcgc tgctgctgct gctgctgctg     660
   ctgctgctgc tgctgctgct gctgctgctt ctgcgcgcag gctttgccac gaggccggcg     720
   tgctggccgc tgccgcttcc agtccgcgtg gagagatcga atgagagata aactggatgg     780
   attcatcgag ggatgaatga acgatggttg gatgcctttt tcctttttca ggtccacagc     840
   gggaagcagg agcgcgtgaa tctgccgcca tccgcatacg tctgcatcgc atcgcatcgc     900
   atgcacgcat cgctcgccgg gagccacaga cgggcgacag ggcggccagc cagccaggca     960
   gccagccagg caggcaccag agggccagag agcgcgcctc acgcacgcgc cgcagtgcgc    1020
   gcatcgctcg cagtgcagac cttgattccc cgcgcggatc tccgcgagcc cgaaacgaag    1080
   agcgccgtac gggcccatcc tagcgtcgcc tcgcaccgca tcgcatcgca tcgcgttccc    1140
   tagagagtag tactcgacga aggcaccatt tccgcgctcc tcttcggcgc gatcgaggcc    1200
   cccggcgccg cgacgatcgc ggcggccgcg gcgctggcgg cggccctggc gctcgcgctg    1260
   gcggccgccg cgggcgtctg gccctggcgc gcgcgggcgc cgcaggagga gcggcagcgg    1320
   ctgctcgccg ccagagaagg agcgcgccgg gcccggggag ggacggggag gagaaggaga    1380
   aggcgcgcaa ggcggccccg aaagagaaga ccctggactt gaacgcgaag aagaagaaga    1440
   aggagaagaa gttgaagaag aagaagaaga aggagaggaa gttgaagaag acgaggagca    1500
   ggcgcgttcc aaggcgcgtt ctcttccgga ggcgcgttcc agctgcggcg gcggggcggg    1560
   ctgcggggcg ggcgcgggcg cgggtgcggg cagaggggac gcgcgcgcgg aggcggaggg    1620
   ggccgagcgg gagcccctgc tgctgcgggg cgcccgggcc gcaggtgtgg cgcgcgcgac    1680
   gacggaggcg acgacgccag cggccgcgac gacaaggccg gcggcgtcgg cgggcggaag    1740
   gccccgcgcg gagcaggggc gggagcagga caaggcgcag gagcaggagc agggccggga    1800
   gcgggagcgg gagcgggcgg cggagcccga ggcagaaccc aatcgagatc cagagcgagc    1860
   agaggccggc cgcgagcccg agcccgcgcc gcagatcact agtaccgctg cggaatcaca 1920
   gcagcagcag cagcagcagc agcagcagca gcagcagcag cagccacgag agggagataa 1980
   agaaaaagcg gcagagacg 1999
<210> 4
   <211> 325
   <212> DNA
   <213> Schizochytrium
<400> 4
   gatccgaaag tgaaccttgt cctaacccga cagcgaatgg cgggaggggg cgggctaaaa 60
   gatcgtatta catagtattt ttcccctact ctttgtgttt gtcttttttt ttttttgaac 120
   gcattcaagc cacttgtctt ggtttacttg tttgtttgct tgcttgcttg cttgcttgcc 180
   tgcttcttgg tcagacggcc caaaaaaggg aaaaaattca ttcatggcac agataagaaa 240
   aagaaaaagt ttgtcgacca ccgtcatcag aaagcaagag aagagaaaca ctcgcgctca 300
   cattctcgct cgcgtaagaa tctta 325
<210> 5
   <211> 372
   <212> DNA
   <213> Streptoalloteichus hindustanus
<400> 5
   atggccaagt tgaccagtgc cgttccggtg ctcaccgcgc gcgacgtcgc cggagcggtc 60
   gagttctgga ccgaccggct cgggttctcc cgggacttcg tggaggacga cttcgccggt 120
   gtggtccggg acgacgtgac cctgttcatc agcgcggtcc aggaccaggt ggtgccggac 180
   aacaccctgg cctgggtgtg ggtgcgcggc ctggacgagc tgtacgccga gtggtcggag 240
   gtcgtgtcca cgaacttccg ggacgcctcc gggccggcca tgaccgagat cggcgagcag 300
   ccgtgggggc gggagttcgc cctgcgcgac ccggccggca actgcgtgca cttcgtggcc 360
   gaggagcagg ac 372
<210> 6
   <211> 2055
   <212> DNA
   <213> Schizochytrium
<400> 6
   atgagcgcga cccgcgcggc gacgaggaca gcggcggcgc tgtcctcggc gctgacgacg     60
   cctgtaaagc agcagcagca gcagcagctg cgcgtaggcg cggcgtcggc acggctggcg    120
   gccgcggcgt tctcgtccgg cacgggcgga gacgcggcca agaaggcggc cgcggcgagg    180
   gcgttctcca cgggacgcgg ccccaacgcg acacgcgaga agagctcgct ggccacggtc    240
   caggcggcga cggacgatgc gcgcttcgtc ggcctgaccg gcgcccaaat ctttcatgag    300
   ctcatgcgcg agcaccaggt ggacaccatc tttggctacc ctggcggcgc cattctgccc    360
   gtttttgatg ccatttttga gagtgacgcc ttcaagttca ttctcgctcg ccacgagcag    420
   ggcgccggcc acatggccga gggctacgcg cgcgccacgg gcaagcccgg cgttgtcctc    480
   gtcacctcgg gccctggagc caccaacacc atcaccccga tcatggatgc ttacatggac    540
   ggtacgccgc tgctcgtgtt caccggccag gtgcccacct ctgctgtcgg cacggacgct    600
   ttccaggagt gtgacattgt tggcatcagc cgcgcgtgca ccaagtggaa cgtcatggtc    660
   aaggacgtga aggagctccc gcgccgcatc aatgaggcct ttgagattgc catgagcggc    720
   cgcccgggtc ccgtgctcgt cgatcttcct aaggatgtga ccgccgttga gctcaaggaa    780
   atgcccgaca gctcccccca ggttgctgtg cgccagaagc aaaaggtcga gcttttccac    840
   aaggagcgca ttggcgctcc tggcacggcc gacttcaagc tcattgccga gatgatcaac    900
   cgtgcggagc gacccgtcat ctatgctggc cagggtgtca tgcagagccc gttgaatggc    960
   ccggctgtgc tcaaggagtt cgcggagaag gccaacattc ccgtgaccac caccatgcag   1020
   ggtctcggcg gctttgacga gcgtagtccc ctctccctca agatgctcgg catgcacggc   1080
   tctgcctacg ccaactactc gatgcagaac gccgatctta tcctggcgct cggtgcccgc   1140
   tttgatgatc gtgtgacggg ccgcgttgac gcctttgctc cggaggctcg ccgtgccgag   1200
   cgcgagggcc gcggtggcat cgttcacttt gagatttccc ccaagaacct ccacaaggtc   1260
   gtccagccca ccgtcgcggt cctcggcgac gtggtcgaga acctcgccaa cgtcacgccc   1320
   cacgtgcagc gccaggagcg cgagccgtgg tttgcgcaga tcgccgattg gaaggagaag   1380
   cacccttttc tgctcgagtc tgttgattcg gacgacaagg ttctcaagcc gcagcaggtc    1440
   ctcacggagc ttaacaagca gattctcgag attcaggaga aggacgccga ccaggaggtc   1500
   tacatcacca cgggcgtcgg aagccaccag atgcaggcag cgcagttcct tacctggacc   1560
   aagccgcgcc agtggatctc ctcgggtggc gccggcacta tgggctacgg ccttccctcg   1620
   gccattggcg ccaagattgc caagcccgat gctattgtta ttgacatcga tggtgatgct   1680
   tcttattcga tgaccggtat ggaattgatc acagcagccg aattcaaggt tggcgtgaag   1740
   attcttcttt tgcagaacaa ctttcagggc atggtcaaga actggcagga tctcttttac   1800
   gacaagcgct actcgggcac cgccatgttc aacccgcgct tcgacaaggt cgccgatgcg   1860
   atgcgtgcca agggtctcta ctgcgcgaaa cagtcggagc tcaaggacaa gatcaaggag   1920
   tttctcgagt acgatgaggg tcccgtcctc ctcgaggttt tcgtggacaa ggacacgctc   1980
   gtcttgccca tggtccccgc tggctttccg ctccacgaga tggtcctcga gcctcctaag   2040
   cccaaggacg cctaa                                                    2055
<210> 7
   <211> 684
   <212> PRT
   <213> Schizochytrium
<400> 7
   Met Ser Ala Thr Arg Ala Ala Thr Arg Thr Ala Ala Ala Leu Ser Ser
   1               5                   10                  15
   Ala Leu Thr Thr Pro Val Lys Gln Gln Gln Gln Gln Gln Leu Arg Val
               20                  25                  30
   Gly Ala Ala Ser Ala Arg Leu Ala Ala Ala Ala Phe Ser Ser Gly Thr
           35                  40                  45
   Gly Gly Asp Ala Ala Lys Lys Ala Ala Ala Ala Arg Ala Phe Ser Thr
       50                  55                  60
   Gly Arg Gly Pro Asn Ala Thr Arg Glu Lys Ser Ser Leu Ala Thr Val
   65                  70                  75                  80
   Gln Ala Ala Thr Asp Asp Ala Arg Phe Val Gly Leu Thr Gly Ala Gln
                   85                  90                  95
   Ile Phe His Glu Leu Met Arg Glu His Gln Val Asp Thr Ile Phe Gly
               100                 105                 110
   Tyr Pro Gly Gly Ala Ile Leu Pro Val Phe Asp Ala Ile Phe Glu Ser
           115                 120                 125
   Asp Ala Phe Lys Phe Ile Leu Ala Arg His Glu Gln Gly Ala Gly His
       130                 135                 140
   Met Ala Glu Gly Tyr Ala Arg Ala Thr Gly Lys Pro Gly Val Val Leu
   145                 150                 155                 160
   Val Thr Ser Gly Pro Gly Ala Thr Asn Thr Ile Thr Pro Ile Met Asp
                   165                 170                 175
   Ala Tyr Met Asp Gly Thr Pro Leu Leu Val Phe Thr Gly Gln Val Pro
               180                 185                 190
   Thr Ser Ala Val Gly Thr Asp Ala Phe Gln Glu Cys Asp Ile Val Gly
           195                 200                 205
   Ile Ser Arg Ala Cys Thr Lys Trp Asn Val Met Val Lys Asp Val Lys
       210                 215                 220
   Glu Leu Pro Arg Arg Ile Asn Glu Ala Phe Glu Ile Ala Met Ser Gly
   225 230 235 240
   Arg Pro Gly Pro Val Leu Val Asp Leu Pro Lys Asp Val Thr Ala Val
   245 250 255
   Glu Leu Lys Glu Met Pro Asp Ser Ser Pro Gln Val Ala Val Arg Gln
   260 265 270
   Lys Gln Lys Val Glu Leu Phe His Lys Glu Arg Ile Gly Ala Pro Gly
   275 280 285
   Thr Ala Asp Phe Lys Leu Ile Ala Glu Met Ile Asn Arg Ala Glu Arg
   290 295 300
   Pro Val Ile Tyr Ala Gly Gln Gly Val Met Gln Ser Pro Leu Asn Gly
   305 310 315 320
   Pro Ala Val Leu Lys Glu Phe Ala Glu Lys Ala Asn Ile Pro Val Thr
   325 330 335
   Thr Thr Met Gln Gly Leu Gly Gly Phe Asp Glu Arg Ser Pro Leu Ser
   340 345 350
   Leu Lys Met Leu Gly Met His Gly Ser Ala Tyr Ala Asn Tyr Ser Met
   355 360 365
   Gln Asn Ala Asp Leu Ile Leu Ala Leu Gly Ala Arg Phe Asp Asp Arg
   370 375 380
   Val Thr Gly Arg Val Asp Ala Phe Ala Pro Glu Ala Arg Arg Ala Glu
   385 390 395 400
   Arg Glu Gly Arg Gly Gly Ile Val His Phe Glu Ile Ser Pro Lys Asn
   405 410 415
   Leu His Lys Val Val Gln Pro Thr Val Ala Val Leu Gly Asp Val Val
   420 425 430
   Glu Asn Leu Ala Asn Val Thr Pro His Val Gln Arg Gln Glu Arg Glu
   435 440 445
   Pro Trp Phe Ala Gln Ile Ala Asp Trp Lys Glu Lys His Pro Phe Leu
   450 455 460
   Leu Glu Ser Val Asp Ser Asp Asp Lys Val Leu Lys Pro Gln Gln Val
   465 470 475 480
   Leu Thr Glu Leu Asn Lys Gln Ile Leu Glu Ile Gln Glu Lys Asp Ala
                   485                 490                 495
   Asp Gln Glu Val Tyr Ile Thr Thr Gly Val Gly Ser His Gln Met Gln
               500                 505                 510
   Ala Ala Gln Phe Leu Thr Trp Thr Lys Pro Arg Gln Trp Ile Ser Ser
           515                 520                 525
   Gly Gly Ala Gly Thr Met Gly Tyr Gly Leu Pro Ser Ala Ile Gly Ala
       530                 535                 540
   Lys Ile Ala Lys Pro Asp Ala Ile Val Ile Asp Ile Asp Gly Asp Ala
   545                 550                 555                 560
   Ser Tyr Ser Met Thr Gly Met Glu Leu Ile Thr Ala Ala Glu Phe Lys
                   565                 570                 575
   Val Gly Val Lys Ile Leu Leu Leu Gln Asn Asn Phe Gln Gly Met Val
               580                 585                 590
   Lys Asn Trp Gln Asp Leu Phe Tyr Asp Lys Arg Tyr Ser Gly Thr Ala
           595                 600                 605
   Met Phe Asn Pro Arg Phe Asp Lys Val Ala Asp Ala Met Arg Ala Lys
       610                 615                 620
   Gly Leu Tyr Cys Ala Lys Gln Ser Glu Leu Lys Asp Lys Ile Lys Glu
   625                 630                 635                 640
   Phe Leu Glu Tyr Asp Glu Gly Pro Val Leu Leu Glu Val Phe Val Asp
                   645                 650                 655
   Lys Asp Thr Leu Val Leu Pro Met Val Pro Ala Gly Phe Pro Leu His
               660                 665                 670
   Glu Met Val Leu Glu Pro Pro Lys Pro Lys Asp Ala
           675                 680
<210> 8
   <211> 684
   <212> PRT
   <213> Artificial Sequence
<220>
   <223> Mutated ALS 1
<400> 8
   Met Ser Ala Thr Arg Ala Ala Thr Arg Thr Ala Ala Ala Leu Ser Ser
   1               5                   10                  15
   Ala Leu Thr Thr Pro Val Lys Gln Gln Gln Gln Gln Gln Leu Arg Val
               20                  25                  30
   Gly Ala Ala Ser Ala Arg Leu Ala Ala Ala Ala Phe Ser Ser Gly Thr
           35                  40                  45
   Gly Gly Asp Ala Ala Lys Lys Ala Ala Ala Ala Arg Ala Phe Ser Thr
       50                  55                  60
   Gly Arg Gly Pro Asn Ala Thr Arg Glu Lys Ser Ser Leu Ala Thr Val
   65                  70                  75                  80
   Gln Ala Ala Thr Asp Asp Ala Arg Phe Val Gly Leu Thr Gly Ala Gln
                   85                  90                  95
   Ile Phe His Glu Leu Met Arg Glu His Gln Val Asp Thr Ile Phe Gly
               100                 105                 110
   Tyr Pro Gly Gly Ala Ile Leu Pro Val Phe Asp Ala Ile Phe Glu Ser
           115                 120                 125
   Asp Ala Phe Lys Phe Ile Leu Ala Arg His Glu Gln Gly Ala Gly His
       130                 135                 140
   Met Ala Glu Gly Tyr Ala Arg Ala Thr Gly Lys Pro Gly Val Val Leu
   145                 150                 155                 160
   Val Thr Ser Gly Pro Gly Ala Thr Asn Thr Ile Thr Pro Ile Met Asp
                   165                 170                 175
   Ala Tyr Met Asp Gly Thr Pro Leu Leu Val Phe Thr Gly Gln Val Pro
               180                 185                 190
   Thr Ser Ala Val Gly Thr Asp Ala Phe Gln Glu Cys Asp Ile Val Gly
           195                 200                 205
   Ile Ser Arg Ala Cys Thr Lys Trp Asn Val Met Val Lys Asp Val Lys
       210                 215                 220
   Glu Leu Pro Arg Arg Ile Asn Glu Ala Phe Glu Ile Ala Met Ser Gly
   225                 230                 235                 240
   Arg Pro Gly Pro Val Leu Val Asp Leu Pro Lys Asp Val Thr Ala Val
                   245                 250                 255
   Glu Leu Lys Glu Met Pro Asp Ser Ser Pro Gln Val Ala Val Arg Gln
   260 265 270
   Lys Gln Lys Val Glu Leu Phe His Lys Glu Arg Ile Gly Ala Pro Gly
   275 280 285
   Thr Ala Asp Phe Lys Leu Ile Ala Glu Met Ile Asn Arg Ala Glu Arg
   290 295 300
   Pro Val Ile Tyr Ala Gly Gln Gly Val Met Gln Ser Pro Leu Asn Gly
   305 310 315 320
   Pro Ala Val Leu Lys Glu Phe Ala Glu Lys Ala Asn Ile Pro Val Thr
   325 330 335
   Thr Thr Met Gln Gly Leu Gly Gly Phe Asp Glu Arg Ser Pro Leu Ser
   340 345 350
   Leu Lys Met Leu Gly Met His Gly Ser Ala Tyr Ala Asn Tyr Ser Met
   355 360 365
   Gln Asn Ala Asp Leu Ile Leu Ala Leu Gly Ala Arg Phe Asp Asp Arg
   370 375 380
   Val Thr Gly Arg Val Asp Ala Phe Ala Pro Glu Ala Arg Arg Ala Glu
   385 390 395 400
   Arg Glu Gly Arg Gly Gly Ile Val His Phe Glu Ile Ser Pro Lys Asn
   405 410 415
   Leu His Lys Val Val Gln Pro Thr Val Ala Val Leu Gly Asp Val Val
   420 425 430
   Glu Asn Leu Ala Asn Val Thr Pro His Val Gln Arg Gln Glu Arg Glu
   435 440 445
   Pro Trp Phe Ala Gln Ile Ala Asp Trp Lys Glu Lys His Pro Phe Leu
   450 455 460
   Leu Glu Ser Val Asp Ser Asp Asp Lys Val Leu Lys Pro Gln Gln Val
   465 470 475 480
   Leu Thr Glu Leu Asn Lys Gln Ile Leu Glu Ile Gln Glu Lys Asp Ala
   485 490 495
   Asp Gln Glu Val Tyr Ile Thr Thr Gly Val Gly Ser His Gln Met Gln
   500 505 510
   Ala Ala Gln Phe Leu Thr Trp Thr Lys Pro Arg Gln Trp Ile Ser Ser
   515 520 525
   Gly Gly Ala Gly Thr Met Gly Tyr Gly Leu Pro Ser Ala Ile Gly Ala
   530 535 540
   Lys Ile Ala Lys Pro Asp Ala Ile Val Ile Asp Ile Asp Gly Asp Ala
   545 550 555 560
   Ser Tyr Ser Met Thr Gly Met Glu Leu Ile Thr Ala Ala Glu Phe Lys
   565 570 575
   Val Gly Val Lys Ile Leu Leu Leu Gln Asn Asn Phe Gln Gly Met Val
   580 585 590
   Lys Asn Val Gln Asp Leu Phe Tyr Asp Lys Arg Tyr Ser Gly Thr Ala
   595 600 605
   Met Phe Asn Pro Arg Phe Asp Lys Val Ala Asp Ala Met Arg Ala Lys
   610 615 620
   Gly Leu Tyr Cys Ala Lys Gln Ser Glu Leu Lys Asp Lys Ile Lys Glu
   625 630 635 640
   Phe Leu Glu Tyr Asp Glu Gly Pro Val Leu Leu Glu Val Phe Val Asp
   645 650 655
   Lys Asp Thr Leu Val Leu Pro Met Val Pro Ala Gly Phe Pro Leu His
   660 665 670
   Glu Met Val Leu Glu Pro Pro Lys Pro Lys Asp Ala
   675 680
<210> 9
   <211> 684
   <212> PRT
   <213> Artificial Sequence
<220>
   <223> Mutated ALS 2
<400> 9
   Met Ser Ala Thr Arg Ala Ala Thr Arg Thr Ala Ala Ala Leu Ser Ser
   1 5 10 15
   Ala Leu Thr Thr Pro Val Lys Gln Gln Gln Gln Gln Gln Leu Arg Val
   20 25 30
   Gly Ala Ala Ser Ala Arg Leu Ala Ala Ala Ala Phe Ser Ser Gly Thr
   35 40 45
   Gly Gly Asp Ala Ala Lys Lys Ala Ala Ala Ala Arg Ala Phe Ser Thr
   50 55 60
   Gly Arg Gly Pro Asn Ala Thr Arg Glu Lys Ser Ser Leu Ala Thr Val
   65 70 75 80
   Gln Ala Ala Thr Asp Asp Ala Arg Phe Val Gly Leu Thr Gly Ala Gln
   85 90 95
   Ile Phe His Glu Leu Met Arg Glu His Gln Val Asp Thr Ile Phe Gly
   100 105 110
   Tyr Pro Gly Gly Ala Ile Leu Pro Val Phe Asp Ala Ile Phe Glu Ser
   115 120 125
   Asp Ala Phe Lys Phe Ile Leu Ala Arg His Glu Gln Gly Ala Gly His
   130 135 140
   Met Ala Glu Gly Tyr Ala Arg Ala Thr Gly Lys Pro Gly Val Val Leu
   145 150 155 160
   Val Thr Ser Gly Pro Gly Ala Thr Asn Thr Ile Thr Pro Ile Met Asp
   165 170 175
   Ala Tyr Met Asp Gly Thr Pro Leu Leu Val Phe Thr Gly Gln Val Gln
   180 185 190
   Thr Ser Ala Val Gly Thr Asp Ala Phe Gln Glu Cys Asp Ile Val Gly
   195 200 205
   Ile Ser Arg Ala Cys Thr Lys Trp Asn Val Met Val Lys Asp Val Lys
   210 215 220
   Glu Leu Pro Arg Arg Ile Asn Glu Ala Phe Glu Ile Ala Met Ser Gly
   225 230 235 240
   Arg Pro Gly Pro Val Leu Val Asp Leu Pro Lys Asp Val Thr Ala Val
   245 250 255
   Glu Leu Lys Glu Met Pro Asp Ser Ser Pro Gln Val Ala Val Arg Gln
   260 265 270
   Lys Gln Lys Val Glu Leu Phe His Lys Glu Arg Ile Gly Ala Pro Gly
   275 280 285
   Thr Ala Asp Phe Lys Leu Ile Ala Glu Met Ile Asn Arg Ala Glu Arg
       290                 295                 300
   Pro Val Ile Tyr Ala Gly Gln Gly Val Met Gln Ser Pro Leu Asn Gly
   305                 310                 315                 320
   Pro Ala Val Leu Lys Glu Phe Ala Glu Lys Ala Asn Ile Pro Val Thr
                   325                 330                 335
   Thr Thr Met Gln Gly Leu Gly Gly Phe Asp Glu Arg Ser Pro Leu Ser
               340                 345                 350
   Leu Lys Met Leu Gly Met His Gly Ser Ala Tyr Ala Asn Tyr Ser Met
           355                 360                 365
   Gln Asn Ala Asp Leu Ile Leu Ala Leu Gly Ala Arg Phe Asp Asp Arg
       370                 375                 380
   Val Thr Gly Arg Val Asp Ala Phe Ala Pro Glu Ala Arg Arg Ala Glu
   385                 390                 395                 400
   Arg Glu Gly Arg Gly Gly Ile Val His Phe Glu Ile Ser Pro Lys Asn
                   405                 410                 415
   Leu His Lys Val Val Gln Pro Thr Val Ala Val Leu Gly Asp Val Val
               420                 425                 430
   Glu Asn Leu Ala Asn Val Thr Pro His Val Gln Arg Gln Glu Arg Glu
           435                 440                 445
   Pro Trp Phe Ala Gln Ile Ala Asp Trp Lys Glu Lys His Pro Phe Leu
       450                 455                 460
   Leu Glu Ser Val Asp Ser Asp Asp Lys Val Leu Lys Pro Gln Gln Val
   465                 470                 475                 480
   Leu Thr Glu Leu Asn Lys Gln Ile Leu Glu Ile Gln Glu Lys Asp Ala
                   485                 490                 495
   Asp Gln Glu Val Tyr Ile Thr Thr Gly Val Gly Ser His Gln Met Gln
               500                 505                 510
   Ala Ala Gln Phe Leu Thr Trp Thr Lys Pro Arg Gln Trp Ile Ser Ser
           515                 520                 525
   Gly Gly Ala Gly Thr Met Gly Tyr Gly Leu Pro Ser Ala Ile Gly Ala
       530                 535                 540
   Lys Ile Ala Lys Pro Asp Ala Ile Val Ile Asp Ile Asp Gly Asp Ala
   545                 550                 555                 560
   Ser Tyr Ser Met Thr Gly Met Glu Leu Ile Thr Ala Ala Glu Phe Lys
                   565                 570                 575
   Val Gly Val Lys Ile Leu Leu Leu Gln Asn Asn Phe Gln Gly Met Val
               580                 585                 590
   Lys Asn Trp Gln Asp Leu Phe Tyr Asp Lys Arg Tyr Ser Gly Thr Ala
           595                 600                 605
   Met Phe Asn Pro Arg Phe Asp Lys Val Ala Asp Ala Met Arg Ala Lys
       610                 615                 620
   Gly Leu Tyr Cys Ala Lys Gln Ser Glu Leu Lys Asp Lys Ile Lys Glu
   625                 630                 635                 640
   Phe Leu Glu Tyr Asp Glu Gly Pro Val Leu Leu Glu Val Phe Val Asp
                   645                 650                 655
   Lys Asp Thr Leu Val Leu Pro Met Val Pro Ala Gly Phe Pro Leu His
               660                 665                 670
   Glu Met Val Leu Glu Pro Pro Lys Pro Lys Asp Ala
           675                 680
<210> 10
   <211> 684
   <212> PRT
   <213> Artificial Sequence
<220>
   <223> Mutated ALS 3
<400> 10
   Met Ser Ala Thr Arg Ala Ala Thr Arg Thr Ala Ala Ala Leu Ser Ser
   1               5                   10                  15
   Ala Leu Thr Thr Pro Val Lys Gln Gln Gln Gln Gln Gln Leu Arg Val
               20                  25                  30
   Gly Ala Ala Ser Ala Arg Leu Ala Ala Ala Ala Phe Ser Ser Gly Thr
           35                  40                  45
   Gly Gly Asp Ala Ala Lys Lys Ala Ala Ala Ala Arg Ala Phe Ser Thr
       50                  55                  60
   Gly Arg Gly Pro Asn Ala Thr Arg Glu Lys Ser Ser Leu Ala Thr Val
   65                  70                  75                  80
   Gln Ala Ala Thr Asp Asp Ala Arg Phe Val Gly Leu Thr Gly Ala Gln
                   85                  90                  95
   Ile Phe His Glu Leu Met Arg Glu His Gln Val Asp Thr Ile Phe Gly
               100                 105                 110
   Tyr Pro Gly Gly Ala Ile Leu Pro Val Phe Asp Ala Ile Phe Glu Ser
           115                 120                 125
   Asp Ala Phe Lys Phe Ile Leu Ala Arg His Glu Gln Gly Ala Gly His
       130                 135                 140
   Met Ala Glu Gly Tyr Ala Arg Ala Thr Gly Lys Pro Gly Val Val Leu
   145                 150                 155                 160
   Val Thr Ser Gly Pro Gly Ala Thr Asn Thr Ile Thr Pro Ile Met Asp
                   165                 170                 175
   Ala Tyr Met Asp Gly Thr Pro Leu Leu Val Phe Thr Gly Gln Val Gln
               180                 185                 190
   Thr Ser Ala Val Gly Thr Asp Ala Phe Gln Glu Cys Asp Ile Val Gly
           195                 200                 205
   Ile Ser Arg Ala Cys Thr Lys Trp Asn Val Met Val Lys Asp Val Lys
       210                 215                 220
   Glu Leu Pro Arg Arg Ile Asn Glu Ala Phe Glu Ile Ala Met Ser Gly
   225                 230                 235                 240
   Arg Pro Gly Pro Val Leu Val Asp Leu Pro Lys Asp Val Thr Ala Val
                   245                 250                 255
   Glu Leu Lys Glu Met Pro Asp Ser Ser Pro Gln Val Ala Val Arg Gln
               260                 265                 270
   Lys Gln Lys Val Glu Leu Phe His Lys Glu Arg Ile Gly Ala Pro Gly
           275                 280                 285
   Thr Ala Asp Phe Lys Leu Ile Ala Glu Met Ile Asn Arg Ala Glu Arg
       290                 295                 300
   Pro Val Ile Tyr Ala Gly Gln Gly Val Met Gln Ser Pro Leu Asn Gly
   305                 310                 315                 320
   Pro Ala Val Leu Lys Glu Phe Ala Glu Lys Ala Asn Ile Pro Val Thr
   325 330 335
   Thr Thr Met Gln Gly Leu Gly Gly Phe Asp Glu Arg Ser Pro Leu Ser
   340 345 350
   Leu Lys Met Leu Gly Met His Gly Ser Ala Tyr Ala Asn Tyr Ser Met
   355 360 365
   Gln Asn Ala Asp Leu Ile Leu Ala Leu Gly Ala Arg Phe Asp Asp Arg
   370 375 380
   Val Thr Gly Arg Val Asp Ala Phe Ala Pro Glu Ala Arg Arg Ala Glu
   385 390 395 400
   Arg Glu Gly Arg Gly Gly Ile Val His Phe Glu Ile Ser Pro Lys Asn
   405 410 415
   Leu His Lys Val Val Gln Pro Thr Val Ala Val Leu Gly Asp Val Val
   420 425 430
   Glu Asn Leu Ala Asn Val Thr Pro His Val Gln Arg Gln Glu Arg Glu
   435 440 445
   Pro Trp Phe Ala Gln Ile Ala Asp Trp Lys Glu Lys His Pro Phe Leu
   450 455 460
   Leu Glu Ser Val Asp Ser Asp Asp Lys Val Leu Lys Pro Gln Gln Val
   465 470 475 480
   Leu Thr Glu Leu Asn Lys Gln Ile Leu Glu Ile Gln Glu Lys Asp Ala
   485 490 495
   Asp Gln Glu Val Tyr Ile Thr Thr Gly Val Gly Ser His Gln Met Gln
   500 505 510
   Ala Ala Gln Phe Leu Thr Trp Thr Lys Pro Arg Gln Trp Ile Ser Ser
   515 520 525
   Gly Gly Ala Gly Thr Met Gly Tyr Gly Leu Pro Ser Ala Ile Gly Ala
   530 535 540
   Lys Ile Ala Lys Pro Asp Ala Ile Val Ile Asp Ile Asp Gly Asp Ala
   545 550 555 560
   Ser Tyr Ser Met Thr Gly Met Glu Leu Ile Thr Ala Ala Glu Phe Lys
                   565                 570                 575
   Val Gly Val Lys Ile Leu Leu Leu Gln Asn Asn Phe Gln Gly Met Val
               580                 585                 590
   Lys Asn Val Gln Asp Leu Phe Tyr Asp Lys Arg Tyr Ser Gly Thr Ala
           595                 600                 605
   Met Phe Asn Pro Arg Phe Asp Lys Val Ala Asp Ala Met Arg Ala Lys
       610                 615                 620
   Gly Leu Tyr Cys Ala Lys Gln Ser Glu Leu Lys Asp Lys Ile Lys Glu
   625                 630                 635                 640
   Phe Leu Glu Tyr Asp Glu Gly Pro Val Leu Leu Glu Val Phe Val Asp
                   645                 650                 655
   Lys Asp Thr Leu Val Leu Pro Met Val Pro Ala Gly Phe Pro Leu His
               660                 665                 670
   Glu Met Val Leu Glu Pro Pro Lys Pro Lys Asp Ala
           675                 680
<210> 11
   <211> 2052
   <212> DNA
   <213> Artificial Sequence
<220>
   <223> Mutated ALS 1
<400> 11
   atgagcgcga cccgcgcggc gacgaggaca gcggcggcgc tgtcctcggc gctgacgacg     60
   cctgtaaagc agcagcagca gcagcagctg cgcgtaggcg cggcgtcggc acggctggcg    120
   gccgcggcgt tctcgtccgg cacgggcgga gacgcggcca agaaggcggc cgcggcgagg    180
   gcgttctcca cgggacgcgg ccccaacgcg acacgcgaga agagctcgct ggccacggtc    240
   caggcggcga cggacgatgc gcgcttcgtc ggcctgaccg gcgcccaaat ctttcatgag    300
   ctcatgcgcg agcaccaggt ggacaccatc tttggctacc ctggcggcgc cattctgccc    360
   gtttttgatg ccatttttga gagtgacgcc ttcaagttca ttctcgctcg ccacgagcag    420
   ggcgccggcc acatggccga gggctacgcg cgcgccacgg gcaagcccgg cgttgtcctc    480
   gtcacctcgg gccctggagc caccaacacc atcaccccga tcatggatgc ttacatggac    540
   ggtacgccgc tgctcgtgtt caccggccag gtgcccacct ctgctgtcgg cacggacgct    600
   ttccaggagt gtgacattgt tggcatcagc cgcgcgtgca ccaagtggaa cgtcatggtc    660
   aaggacgtga aggagctccc gcgccgcatc aatgaggcct ttgagattgc catgagcggc    720
   cgcccgggtc ccgtgctcgt cgatcttcct aaggatgtga ccgccgttga gctcaaggaa 780
   atgcccgaca gctcccccca ggttgctgtg cgccagaagc aaaaggtcga gcttttccac 840
   aaggagcgca ttggcgctcc tggcacggcc gacttcaagc tcattgccga gatgatcaac 900
   cgtgcggagc gacccgtcat ctatgctggc cagggtgtca tgcagagccc gttgaatggc 960
   ccggctgtgc tcaaggagtt cgcggagaag gccaacattc ccgtgaccac caccatgcag 1020
   ggtctcggcg gctttgacga gcgtagtccc ctctccctca agatgctcgg catgcacggc 1080
   tctgcctacg ccaactactc gatgcagaac gccgatctta tcctggcgct cggtgcccgc 1140
   tttgatgatc gtgtgacggg ccgcgttgac gcctttgctc cggaggctcg ccgtgccgag 1200
   cgcgagggcc gcggtggcat cgttcacttt gagatttccc ccaagaacct ccacaaggtc 1260
   gtccagccca ccgtcgcggt cctcggcgac gtggtcgaga acctcgccaa cgtcacgccc 1320
   cacgtgcagc gccaggagcg cgagccgtgg tttgcgcaga tcgccgattg gaaggagaag 1380
   cacccttttc tgctcgagtc tgttgattcg gacgacaagg ttctcaagcc gcagcaggtc 1440
   ctcacggagc ttaacaagca gattctcgag attcaggaga aggacgccga ccaggaggtc 1500
   tacatcacca cgggcgtcgg aagccaccag atgcaggcag cgcagttcct tacctggacc 1560
   aagccgcgcc agtggatctc ctcgggtggc gccggcacta tgggctacgg ccttccctcg 1620
   gccattggcg ccaagattgc caagcccgat gctattgtta ttgacatcga tggtgatgct 1680
   tcttattcga tgaccggtat ggaattgatc acagcagccg aattcaaggt tggcgtgaag 1740
   attcttcttt tgcagaacaa ctttcagggc atggtcaaga acgttcagga tctcttttac 1800
   gacaagcgct actcgggcac cgccatgttc aacccgcgct tcgacaaggt cgccgatgcg 1860
   atgcgtgcca agggtctcta ctgcgcgaaa cagtcggagc tcaaggacaa gatcaaggag 1920
   tttctcgagt acgatgaggg tcccgtcctc ctcgaggttt tcgtggacaa ggacacgctc 1980
   gtcttgccca tggtccccgc tggctttccg ctccacgaga tggtcctcga gcctcctaag 2040
   cccaaggacg cc 2052
<210> 12
   <211> 2052
   <212> DNA
   <213> Artificial Sequence
<220>
   <223> Mutated ALS 2
<400> 12
   atgagcgcga cccgcgcggc gacgaggaca gcggcggcgc tgtcctcggc gctgacgacg      60
   cctgtaaagc agcagcagca gcagcagctg cgcgtaggcg cggcgtcggc acggctggcg     120
   gccgcggcgt tctcgtccgg cacgggcgga gacgcggcca agaaggcggc cgcggcgagg     180
   gcgttctcca cgggacgcgg ccccaacgcg acacgcgaga agagctcgct ggccacggtc 240
   caggcggcga cggacgatgc gcgcttcgtc ggcctgaccg gcgcccaaat ctttcatgag 300
   ctcatgcgcg agcaccaggt ggacaccatc tttggctacc ctggcggcgc cattctgccc 360
   gtttttgatg ccatttttga gagtgacgcc ttcaagttca ttctcgctcg ccacgagcag 420
   ggcgccggcc acatggccga gggctacgcg cgcgccacgg gcaagcccgg cgttgtcctc 480
   gtcacctcgg gccctggagc caccaacacc atcaccccga tcatggatgc ttacatggac 540
   ggtacgccgc tgctcgtgtt caccggccag gtgcagacct ctgctgtcgg cacggacgct 600
   ttccaggagt gtgacattgt tggcatcagc cgcgcgtgca ccaagtggaa cgtcatggtc 660
   aaggacgtga aggagctccc gcgccgcatc aatgaggcct ttgagattgc catgagcggc 720
   cgcccgggtc ccgtgctcgt cgatcttcct aaggatgtga ccgccgttga gctcaaggaa 780
   atgcccgaca gctcccccca ggttgctgtg cgccagaagc aaaaggtcga gcttttccac 840
   aaggagcgca ttggcgctcc tggcacggcc gacttcaagc tcattgccga gatgatcaac 900
   cgtgcggagc gacccgtcat ctatgctggc cagggtgtca tgcagagccc gttgaatggc 960
   ccggctgtgc tcaaggagtt cgcggagaag gccaacattc ccgtgaccac caccatgcag 1020
   ggtctcggcg gctttgacga gcgtagtccc ctctccctca agatgctcgg catgcacggc 1080
   tctgcctacg ccaactactc gatgcagaac gccgatctta tcctggcgct cggtgcccgc 1140
   tttgatgatc gtgtgacggg ccgcgttgac gcctttgctc cggaggctcg ccgtgccgag 1200
   cgcgagggcc gcggtggcat cgttcacttt gagatttccc ccaagaacct ccacaaggtc 1260
   gtccagccca ccgtcgcggt cctcggcgac gtggtcgaga acctcgccaa cgtcacgccc 1320
   cacgtgcagc gccaggagcg cgagccgtgg tttgcgcaga tcgccgattg gaaggagaag 1380
   cacccttttc tgctcgagtc tgttgattcg gacgacaagg ttctcaagcc gcagcaggtc 1440
   ctcacggagc ttaacaagca gattctcgag attcaggaga aggacgccga ccaggaggtc 1500
   tacatcacca cgggcgtcgg aagccaccag atgcaggcag cgcagttcct tacctggacc 1560
   aagccgcgcc agtggatctc ctcgggtggc gccggcacta tgggctacgg ccttccctcg 1620
   gccattggcg ccaagattgc caagcccgat gctattgtta ttgacatcga tggtgatgct 1680
   tcttattcga tgaccggtat ggaattgatc acagcagccg aattcaaggt tggcgtgaag 1740
   attcttcttt tgcagaacaa ctttcagggc atggtcaaga actggcagga tctcttttac 1800
   gacaagcgct actcgggcac cgccatgttc aacccgcgct tcgacaaggt cgccgatgcg 1860
   atgcgtgcca agggtctcta ctgcgcgaaa cagtcggagc tcaaggacaa gatcaaggag 1920
   tttctcgagt acgatgaggg tcccgtcctc ctcgaggttt tcgtggacaa ggacacgctc 1980
   gtcttgccca tggtccccgc tggctttccg ctccacgaga tggtcctcga gcctcctaag 2040
   cccaaggacg cc 2052
<210> 13
   <211> 2055
   <212> DNA
   <213> Artificial Sequence
<220>
   <223> Mutated ALS 3
<400> 13
   atgagcgcga cccgcgcggc gacgaggaca gcggcggcgc tgtcctcggc gctgacgacg      60
   cctgtaaagc agcagcagca gcagcagctg cgcgtaggcg cggcgtcggc acggctggcg     120
   gccgcggcgt tctcgtccgg cacgggcgga gacgcggcca agaaggcggc cgcggcgagg     180
   gcgttctcca cgggacgcgg ccccaacgcg acacgcgaga agagctcgct ggccacggtc     240
   caggcggcga cggacgatgc gcgcttcgtc ggcctgaccg gcgcccaaat ctttcatgag     300
   ctcatgcgcg agcaccaggt ggacaccatc tttggctacc ctggcggcgc cattctgccc     360
   gtttttgatg ccatttttga gagtgacgcc ttcaagttca ttctcgctcg ccacgagcag     420
   ggcgccggcc acatggccga gggctacgcg cgcgccacgg gcaagcccgg cgttgtcctc     480
   gtcacctcgg gccctggagc caccaacacc atcaccccga tcatggatgc ttacatggac     540
   ggtacgccgc tgctcgtgtt caccggccag gtgcagacct ctgctgtcgg cacggacgct     600
   ttccaggagt gtgacattgt tggcatcagc cgcgcgtgca ccaagtggaa cgtcatggtc     660
   aaggacgtga aggagctccc gcgccgcatc aatgaggcct ttgagattgc catgagcggc     720
   cgcccgggtc ccgtgctcgt cgatcttcct aaggatgtga ccgccgttga gctcaaggaa     780
   atgcccgaca gctcccccca ggttgctgtg cgccagaagc aaaaggtcga gcttttccac     840
   aaggagcgca ttggcgctcc tggcacggcc gacttcaagc tcattgccga gatgatcaac     900
   cgtgcggagc gacccgtcat ctatgctggc cagggtgtca tgcagagccc gttgaatggc     960
   ccggctgtgc tcaaggagtt cgcggagaag gccaacattc ccgtgaccac caccatgcag    1020
   ggtctcggcg gctttgacga gcgtagtccc ctctccctca agatgctcgg catgcacggc    1080
   tctgcctacg ccaactactc gatgcagaac gccgatctta tcctggcgct cggtgcccgc    1140
   tttgatgatc gtgtgacggg ccgcgttgac gcctttgctc cggaggctcg ccgtgccgag    1200
   cgcgagggcc gcggtggcat cgttcacttt gagatttccc ccaagaacct ccacaaggtc    1260
   gtccagccca ccgtcgcggt cctcggcgac gtggtcgaga acctcgccaa cgtcacgccc    1320
   cacgtgcagc gccaggagcg cgagccgtgg tttgcgcaga tcgccgattg gaaggagaag    1380
   cacccttttc tgctcgagtc tgttgattcg gacgacaagg ttctcaagcc gcagcaggtc    1440
   ctcacggagc ttaacaagca gattctcgag attcaggaga aggacgccga ccaggaggtc    1500
   tacatcacca cgggcgtcgg aagccaccag atgcaggcag cgcagttcct tacctggacc    1560
   aagccgcgcc agtggatctc ctcgggtggc gccggcacta tgggctacgg ccttccctcg 1620
   gccattggcg ccaagattgc caagcccgat gctattgtta ttgacatcga tggtgatgct 1680
   tcttattcga tgaccggtat ggaattgatc acagcagccg aattcaaggt tggcgtgaag 1740
   attcttcttt tgcagaacaa ctttcagggc atggtcaaga acgttcagga tctcttttac 1800
   gacaagcgct actcgggcac cgccatgttc aacccgcgct tcgacaaggt cgccgatgcg 1860
   atgcgtgcca agggtctcta ctgcgcgaaa cagtcggagc tcaaggacaa gatcaaggag 1920
   tttctcgagt acgatgaggg tcccgtcctc ctcgaggttt tcgtggacaa ggacacgctc 1980
   gtcttgccca tggtccccgc tggctttccg ctccacgaga tggtcctcga gcctcctaag 2040
   cccaaggacg cctaa 2055
<210> 14
   <211> 12
   <212> DNA
   <213> Schizochytrium
<400> 14
   cacgacgagt tg 12
<210> 15
   <211> 53
   <212> PRT
   <213> Schizochytrium
<400> 15
   Met Ala Asn Ile Met Ala Asn Val Thr Pro Gln Gly Val Ala Lys Gly
   1 5 10 15
   Phe Gly Leu Phe Val Gly Val Leu Phe Phe Leu Tyr Trp Phe Leu Val
   20 25 30
   Gly Leu Ala Leu Leu Gly Asp Gly Phe Lys Val Ile Ala Gly Asp Ser
   35 40 45
   Ala Gly Thr Leu Phe
   50
<210> 16
   <211> 21
   <212> DNA
   <213> Artificial Sequence
<220>
   <223> Synthetic Primer S4termF
<400> 16
   gatcccatgg cacgtgctac g 21
<210> 17
   <211> 22
   <212> DNA
   <213> Artificial Sequence
<220>
   <223> Synthetic Primer S4termR
<400> 17
   ggcaacatgt atgataagat ac 22
<210> 18
   <211> 21
   <212> DNA
   <213> Artificial Sequence
<220>
   <223> Synthetic Primer C2mcsSmaF
<400> 18
   gatccccggg ttaagcttgg t 21
<210> 19
   <211> 20
   <212> DNA
   <213> Artificial Sequence
<220>
   <223> Synthetic Primer C2mcsSmaR
<400> 19
   actggggccc gtttaaactc 20
<210> 20
   <211> 34
   <212> DNA
   <213> Artificial Sequence
<220>
   <223> Synthetic Primer 5'tubMCS_BglI
<400> 20
   gactagatct caattttagg ccccccactg accg 34
<210> 21
   <211> 34
   <212> DNA
   <213> Artificial Sequence
<220>
   <223> Synthetic Primer 3'SV40MCS_Sal
<400> 21
   gactgtcgac catgtatgat aagatacatt gatg 34
<210> 22
   <211> 28
   <212> DNA
   <213> Artificial Sequence
<220>
   <223> Synthetic Primer 5'ALSproNde3
<400> 22
   gactcatatg gcccaggcct actttcac 28
<210> 23
   <211> 34
   <212> DNA
   <213> Artificial Sequence
<220>
   <223> Synthetic Primer 3'ALStermBglII
<400> 23
   gactagatct gggtcaaggc agaagaattc cgcc 34
<210> 24
   <211> 97
   <212> DNA
   <213> Artificial Sequence
<220>
   <223> Synthetic Primer sec.Gfp5'1b
<400> 24
   tactggttcc ttgtcggcct cgcccttctc ggcgatggct tcaaggtcat cgccggtgac 60
tccgccggta cgctcttcat ggtgagcaag ggcgagg 97
<210> 25
   <211> 32
   <212> DNA
   <213> Artificial Sequence
<220>
   <223> Synthetic Primer sec.Gfp3'Spe
<400> 25
   gatcggtacc ggtgttcttt gttttgattt ct 32
<210> 26
   <211> 105
   <212> DNA
   <213> Artificial Sequence
<220>
   <223> Synthetic Primer sec.Gfp5'Bam
<400> 26
   taatggatcc atggccaaca tcatggccaa cgtcacgccc cagggcgtcg ccaagggctt 60
tggcctcttt gtcggcgtgc tcttctttct ctactggttc cttgt 105
<210> 27
   <211> 40
   <212> DNA
   <213> Artificial Sequence
<220>
   <223> Synthetic Primer ss.eGfpHELD3'RV
<400> 27
   cctgatatct tacaactcgt cgtggttgta cagctcgtcc 40
<210> 28
   <211> 105
   <212> DNA
   <213> Artificial Sequence
<220>
   <223> Synthetic Primer sec.Gfp5'Bam2
<400> 28
   taatggatcc atggccaaca tcatggccaa cgtcacgccc cagggcgtcg ccaagggctt 60
   tggcctcttt gtcggcgtgc tcttctttct ctactggttc cttgt 105
<210> 29
   <211> 27
   <212> DNA
   <213> Artificial Sequence
<220>
   <223> Synthetic Primer prREZ15
<400> 29
   cggtacccgc gaatcaagaa ggtaggc 27
<210> 30
   <211> 27
   <212> DNA
   <213> Artificial Sequence
<220>
   <223> Synthetic Primer prREZ16
<400> 30
   cggatcccgt ctctgccgct ttttctt 27
<210> 31
   <211> 30
   <212> DNA
   <213> Artificial Sequence
<220>
   <223> Synthetic Primer prREZ17
<400> 31
   cggatccgaa agtgaacctt gtcctaaccc 30
<210> 32
   <211> 27
   <212> DNA
   <213> Artificial Sequence
<220>
   <223> Synthetic Primer prREZ18
<400> 32
   ctctagacag atccgcacca tcggccg 27
<210> 33
   <211> 32
   <212> DNA
   <213> Artificial Sequence
<220>
   <223> Synthetic Primer 5'eGFP_kpn
<400> 33
   gactggtacc atggtgaagc aagggcgagg ag 32
<210> 34
   <211> 34
   <212> DNA
   <213> Artificial Sequence
<220>
   <223> Synthetic Primer 3'eGFP_xba
<400> 34
   gacttctaga ttacttgtac agctcgtcca tgcc 34
<210> 35
   <211> 32
   <212> DNA
   <213> Artificial Sequence
<220>
   <223> Synthetic Primer 5'ORFCproKpn-2
<400> 35
   gatcggtacc ggtgttcttt gttttgattt ct 32
<210> 36
   <211> 31
   <212> DNA
   <213> Artificial Sequence
<220>
   <223> Synthetic Primer 3'ORFCproKpn-2
<400> 36
   gatcggtacc gtctctgccg ctttttcttt a 31
<210> 37
   <211> 20
   <212> PRT
   <213> Schizochytrium
<400> 37
   Met Lys Phe Ala Thr Ser Val Ala Ile Leu Leu Val Ala Asn Ile Ala
   1               5                   10                  15
   Thr Ala Leu Ala
               20
<210> 38
   <211> 60
   <212> DNA
   <213> Schizochytrium
<400> 38
   atgaagttcg cgacctcggt cgcaattttg cttgtggcca acatagccac cgccctcgcg 60
<210> 39
   <211> 28
   <212> DNA
   <213> Artificial Sequence
<220>
   <223> Synthetic Primer 5'ss-X Bgl long
<400> 39
   gactagatct atgaagttcg cgacctcg 28
<210> 40
   <211> 33
   <212> DNA
   <213> Artificial Sequence
<220>
   <223> Synthetic Primer 3'ritx_kap_bh_Bg1
<400> 40
   gactagatct tcagcactca ccgcggttaa agg 33
<210> 41
   <211> 702
   <212> DNA
   <213> Artificial Sequence
<220>
   <223> Optimized Sec1
<400> 41
   atgaagttcg cgacctcggt cgcaattttg cttgtggcca acatagccac cgccctcgcg 60
   cagatcgtcc tcagccagtc ccccgccatc ctttccgctt cccccggtga gaaggtgacc 120
   atgacctgcc gcgctagctc ctccgtctcg tacatccact ggttccagca gaagcccggc 180
   tcgtccccca agccctggat ctacgccacc tccaacctcg cctccggtgt tcccgttcgt 240
   ttttccggtt ccggttccgg cacctcctac tccctcacca tctcccgcgt cgaggccgag 300
   gatgccgcca cctactactg ccagcagtgg accagcaacc cccccacctt cggcggtggt 360
   acgaagctcg agattaagcg caccgtcgcc gccccctccg tcttcatttt tcccccctcc 420
   gatgagcagc tcaagtccgg taccgcctcc gtcgtttgcc tcctcaacaa cttctacccc 480
   cgtgaggcca aggtccagtg gaaggtcgac aacgcgcttc agtccggtaa ctcccaggag 540
   tccgtcaccg agcaggattc gaaggacagc acctactccc tctcctccac cctcaccctc 600
   tccaaggccg actacgagaa gcacaaggtc tacgcctgcg aggtcacgca ccagggtctt 660
   tcctcccccg tcacgaagtc ctttaaccgc ggtgagtgct ga 702
<210> 42
   <211> 853
   <212> DNA
   <213> Schizochytrium
<400> 42
   ctccatcgat cgtgcggtca aaaagaaagg aagaagaaag gaaaaagaaa ggcgtgcgca 60
   cccgagtgcg cgctgagcgc ccgctcgcgg ccccgcggag cctccgcgtt agtccccgcc 120
   ccgcgccgcg cagtcccccg ggaggcatcg cgcacctctc gccgccccct cgcgcctcgc 180
   cgattccccg cctccccttt tccgcttctt cgccgcctcc gctcgcggcc gcgtcgcccg 240
   cgccccgctc cctatctgct ccccaggggg gcactccgca ccttttgcgc ccgctgccgc 300
   cgccgcggcc gccccgccgc cctggtttcc cccgcgagcg cggccgcgtc gccgcgcaaa 360
   gactcgccgc gtgccgcccc gagcaacggg tggcggcggc gcggcggcgg gcggggcgcg 420
   gcggcgcgta ggcggggcta ggcgccggct aggcgaaacg ccgcccccgg gcgccgccgc 480
   cgcccgctcc agagcagtcg ccgcgccaga ccgccaacgc agagaccgag accgaggtac 540
   gtcgcgcccg agcacgccgc gacgcgcggc agggacgagg agcacgacgc cgcgccgcgc 600
   cgcgcggggg gggggaggga gaggcaggac gcgggagcga gcgtgcatgt ttccgcgcga 660
   gacgacgccg cgcgcgctgg agaggagata aggcgcttgg atcgcgagag ggccagccag 720
   gctggaggcg aaaatgggtg gagaggatag tatcttgcgt gcttggacga ggagactgac 780
   gaggaggacg gatacgtcga tgatgatgtg cacagagaag aagcagttcg aaagcgacta 840
   ctagcaagca agg 853
<210> 43
   <211> 1064
   <212> DNA
   <213> Schizochytrium
<400> 43
   ctcttatctg cctcgcgccg ttgaccgccg cttgactctt ggcgcttgcc gctcgcatcc 60
   tgcctcgctc gcgcaggcgg gcgggcgagt gggtgggtcc gcagccttcc gcgctcgccc 120
   gctagctcgc tcgcgccgtg ctgcagccag cagggcagca ccgcacggca ggcaggtccc 180
   ggcgcggatc gatcgatcca tcgatccatc gatccatcga tcgtgcggtc aaaaagaaag 240
   gaagaagaaa ggaaaaagaa aggcgtgcgc acccgagtgc gcgctgagcg cccgctcgcg 300
   gtcccgcgga gcctccgcgt tagtccccgc cccgcgccgc gcagtccccc gggaggcatc 360
   gcgcacctct cgccgccccc tcgcgcctcg ccgattcccc gcctcccctt ttccgcttct 420
   tcgccgcctc cgctcgcggc cgcgtcgccc gcgccccgct ccctatctgc tccccagggg 480
   ggcactccgc accttttgcg cccgctgccg ccgccgcggc cgccccgccg ccctggtttc 540
   ccccgcgagc gcggccgcgt cgccgcgcaa agactcgccg cgtgccgccc cgagcaacgg 600
   gtggcggcgg cgcggcggcg ggcggggcgc ggcggcgcgt aggcggggct aggcgccggc 660
   taggcgaaac gccgcccccg ggcgccgccg ccgcccgctc cagagcagtc gccgcgccag 720
   accgccaacg cagagaccga gaccgaggta cgtcgcgccc gagcacgccg cgacgcgcgg 780
   cagggacgag gagcacgacg ccgcgccgcg ccgcgcgggg gggggagggg agaggcagga 840
   cgcgggagcg agcgtgcatg tttccgcgcg agacgacgcc gcgcgcgctg gagaggagat 900
   aaggcgcttg gatcgcgaga gggccagcca ggctggaggc gaaaatgggt ggagaggata 960
   gtatcttgcg tgcttggacg aggagactga cgaggaggac ggatacgtcg atgatgatgt 1020
   gcacagagaa gaagcagttc gaaagcgact actagcaagc aagg 1064
<210> 44
   <211> 837
   <212> DNA
   <213> Schizochytrium
<400> 44
   cttcgctttc tcaacctatc tggacagcaa tccgccactt gccttgatcc ccttccgcgc      60
   ctcaatcact cgctccacgt ccctcttccc cctcctcatc tccgtgcttt ctctgccccc     120
   cccccccccg ccgcggcgtg cgcgcgcgtg gcgccgcggc cgcgacacct tccatactat     180
   cctcgctccc aaaatgggtt gcgctatagg gcccggctag gcgaaagtct agcaggcact     240
   tgcttggcgc agagccgccg cggccgctcg ttgccgcgga tggagaggga gagagagccc     300
   gcctcgataa gcagagacag acagtgcgac tgacagacag acagagagac tggcagaccg     360
   gaatacctcg aggtgagtgc ggcgcgggcg agcgggcggg agcgggagcg caagagggac     420
   ggcgcggcgc ggcggccctg cgcgacgccg cggcgtattc tcgtgcgcag cgccgagcag     480
   cgggacgggc ggctggctga tggttgaagc ggggcggggt gaaatgttag atgagatgat     540
   catcgacgac ggtccgtgcg tcttggctgg cttggctggc ttggctggcg ggcctgccgt     600
   gtttgcgaga aagaggatga ggagagcgac gaggaaggac gagaagactg acgtgtaggg     660
   cgcgcgatgg atgatcgatt gattgattga ttgattggtt gattggctgt gtggtcgatg     720
   aacgtgtaga ctcagggagc gtggttaaat tgttcttgcg ccagacgcga ggactccacc     780
   cccttctttc gcctttacac agcctttttg tgaagcaaca agaaagaaaa agccaag       837
<210> 45
   <211> 1020
   <212> DNA
   <213> Schizochytrium
<400> 45
   ctttttccgc tctgcataat cctaaaagaa agactatacc ctagtcactg tacaaatggg     60
   acatttctct cccgagcgat agctaaggat ttttgcttcg tgtgcactgt gtgctctggc    120
   cgcgcatcga aagtccagga tcttactgtt tctctttcct ttcctttatt tcctgttctc    180
   ttcttcgctt tctcaaccta tctggacagc aatccgccac ttgccttgat ccccttccgc    240
   gcctcaatca ctcgctccac gtccctcttc cccctcctca tctccgtgct ttctctcgcc    300
   cccccccccc ccgccgcggc gtgcgcgcgc gtggcgccgc ggccgcgaca ccttccatac    360
   tatcctcgct cccaaaatgg gttgcgctat agggcccggc taggcgaaag tctagcaggc    420
   acttgcttgg cgcagagccg ccgcggccgc tcgttgccgc ggatggagag ggagagagag    480
   cccgcctcga taagcagaga cagacagtgc gactgacaga cagacagaga gactggcaga    540
   ccggaatacc tcgaggtgag tgcggcgcgg gcgagcgggc gggagcggga gcgcaagagg    600
   gacggcgcgg cgcggcggcc ctgcgcgacg ccgcggcgta ttctcgtgcg cagcgccgag    660
   cagcgggacg ggcggctggc tgatggttga agcggggcgg ggtgaaatgt tagatgagat    720
   gatcatcgac gacggtccgt gcgtcttggc tggcttggct ggcttggctg gcgggcctgc    780
   cgtgtttgcg agaaagagga tgaggagagc gacgaggaag gacgagaaga ctgacgtgta    840
   gggcgcgcga tggatgatcg attgattgat tgattgattg gttgattggc tgtgtggtcg    900
   atgaacgtgt agactcaggg agcgtggtta aattgttctt gcgccagacg cgaggactcc    960
   acccccttct ttcgccttta cacagccttt ttgtgaagca acaagaaaga aaaagccaag   1020
<210> 46
   <211> 1416
   <212> DNA
   <213> Schizochytrium
<400> 46
   cccgtccttg acgccttcgc ttccggcgcg gccatcgatt caattcaccc atccgatacg     60
   ttccgccccc tcacgtccgt ctgcgcacga cccctgcacg accacgccaa ggccaacgcg    120
   ccgctcagct cagcttgtcg acgagtcgca cgtcacatat ctcagatgca ttgcctgcct    180
   gcctgcctgc ctgcctgcct gcctgcctgc ctgcctgcct cagcctctct ttgctctctc    240
   tgcggcggcc gctgcgacgc gctgtacagg agaatgactc caggaagtgc ggctgggata    300
   cgcgctggcg tcggccgtga tgcgcgtgac gggcggcggg cacggccggc acgggttgag    360
   cagaggacga agcgaggcga gacgagacag gccaggcgcg gggagcgctc gctgccgtga    420
   gcagcagacc agggcgcagg aatgtacttt tcttgcggga gcggagacga ggctgccggc     480
   tgctggctgc cggttgctct gcacgcgccg cccgacttgg cgtagcgtgg acgcgcggcg     540
   gcggccgccg tctcgtcgcg gtcggctttg ccgtgtatcg acgctgcggg cttgacacgg     600
   gatggcggaa gttcagcatc gctgcgatcc ctcgcgccgc agaacgagga gagcgcaggc     660
   cggcttcaag tttgaaagga gaggaaggca ggcaaggagc tggaagcttg ccgcggaagg     720
   cgcaggcatg cgtcacgtga aaaaaaggga tttcaagagt agtaagtagg tatggtctac     780
   aagtccccta ttcttacttc gcggaacgtg ggctgctcgt gcgggcgtcc atcttgtttt     840
   tgtttttttt tccgctaggc gcgtgcattg cttgatgagt ctcagcgttc gtctgcagcg     900
   agggcaggaa aataagcggc ccgtgccgtc gagcgcacag gacgtgcaag cgccttgcga     960
   gcgcagcatc cttgcacggc gagcatagag accgcggccg atggactcca gcgaggaatt    1020
   ttcgaccctc tctatcaagc tgcgcttgac agccgggaat ggcagcctga ggagagaggg    1080
   gcgaaggaag ggacttggag aaaagaggta aggcaccctc aatcacggcg cgtgaaagcc    1140
   agtcatccct cgcaaagaaa agacaaaagc gggttttttg tttcgatggg aaagaatttc    1200
   ttagaggaag aagcggcaca cagactcgcg ccatgcagat ttctgcgcag ctcgcgatca    1260
   aaccaggaac gtggtcgctg cgcgccacta tcaggggtag cgcacgaata ccaaacgcat    1320
   tactagctac gcgcctgtga cccgaggatc gggccacaga cgttgtctct tgccatccca    1380
   cgacctggca gcgagaagat cgtccattac tcatcg                              1416
<210> 47
   <211> 24
   <212> DNA
   <213> Artificial Sequence
<220>
   <223> Synthetic Primer 5'60S-807
<400> 47
   tcgatttgcg gatacttgct caca 24
<210> 48
   <211> 21
   <212> DNA
   <213> Artificial Sequence
<220>
   <223> Synthetic Primer 3'60S-2821
<400> 48
   gacgacctcg cccttggaca c 21
<210> 49
   <211> 34
   <212> DNA
   <213> Artificial Sequence
<220>
   <223> Synthetic Primer 5'60Sp-1302-Kpn
<400> 49
   gactggtacc tttttccgct ctgcataatc ctaa 34
<210> 50
   <211> 32
   <212> DNA
   <213> Artificial Sequence
<220>
   <223> Synthetic Primer 3'60Sp-Bam
<400> 50
   gactggatcc ttggcttttt ctttcttgtt gc 32
<210> 51
   <211> 24
   <212> DNA
   <213> Artificial Sequence
<220>
   <223> Synthetic Primer 5'EF1-68
<400> 51
   cgccgttgac cgccgcttga ctct 24
<210> 52
   <211> 24
   <212> DNA
   <213> Artificial Sequence
<220>
   <223> Synthetic Primer 3'EF1-2312
<400> 52
   cgggggtagc ctcggggatg gact 24
<210> 53
   <211> 34
   <212> DNA
   <213> Artificial Sequence
<220>
   <223> Synthetic Primer 5'EF1-54-Kpn
<400> 53
   gactggtacc tcttatctgc ctcgcgccgt tgac 34
<210> 54
   <211> 37
   <212> DNA
   <213> Artificial Sequence
<220>
   <223> Synthetic Primer 5'EF1-1114-Bam
<400> 54
   gactggatcc cttgcttgct agtagtcgct ttcgaac 37
<210> 55
   <211> 29
   <212> DNA
   <213> Artificial Sequence
<220>
   <223> Synthetic Primer 5'Sec1P-kpn
<400> 55
   gactggtacc ccgtccttga cgccttcgc 29
<210> 56
   <211> 31
   <212> DNA
   <213> Artificial Sequence
<220>
   <223> Synthetic Primer 3'Sec1P-ba
<400> 56
   gactggatcc gatgagtaat ggacgatctt c 31
<210> 57
   <211> 1614
   <212> DNA
   <213> Artificial sequence
<220>
   <223> Secretion signal
<400> 57
   ggatccatga agttcgcgac ctcggtcgca attttgcttg tggccaacat agccaccgcc 60
   ctcgcgtcga tgaccaacga gacctcggac cgccctctcg tgcactttac ccccaacaag 120
   ggttggatga acgatcccaa cggcctctgg tacgacgaga aggatgctaa gtggcacctt 180
   tactttcagt acaaccctaa cgacaccgtc tggggcaccc cgctcttctg gggccacgcc 240
   acctccgacg acctcaccaa ctgggaggac cagcccattg ctatcgcccc caagcgcaac 300
   gactcgggag ctttttccgg ttccatggtt gtggactaca acaacacctc cggttttttt 360
   aacgacacca ttgacccccg ccagcgctgc gtcgccatct ggacctacaa cacgcccgag 420
   agcgaggagc agtacatcag ctacagcctt gatggaggct acacctttac cgagtaccag 480
   aagaaccctg tcctcgccgc caactccacc cagttccgcg accctaaggt tttttggtac 540
   gagccttccc agaagtggat tatgaccgcc gctaagtcgc aggattacaa gatcgagatc 600
   tacagcagcg acgacctcaa gtcctggaag cttgagtccg cctttgccaa cgagggtttt 660
   ctcggatacc agtacgagtg ccccggtctc atcgaggtcc ccaccgagca ggacccgtcc 720
   aagtcctact gggtcatgtt tatttccatc aaccctggcg cccctgccgg cggcagcttc 780
   aaccagtact tcgtcggctc ctttaacggc acgcattttg aggccttcga caaccagtcc 840
   cgcgtcgtcg acttcggcaa ggactactac gccctccaga ccttctttaa caccgacccc 900
   acctacggca gcgccctcgg tattgcttgg gcctccaact gggagtactc cgctttcgtc 960
   cccactaacc cctggcgcag ctcgatgtcc ctcgtccgca agttttcgct taacaccgag 1020
   taccaggcca accccgagac cgagcttatt aacctgaagg ccgagcctat tctcaacatc 1080
   tccaacgctg gcccctggtc ccgctttgct actaacacta ccctcaccaa ggccaactcc 1140
   tacaacgtcg atctctccaa ctccaccggt actcttgagt ttgagctcgt ctacgccgtc 1200
   aacaccaccc agaccatctc caagtccgtc ttcgccgacc tctccctctg gttcaagggc 1260
   cttgaggacc ccgaggagta cctgcgcatg ggttttgagg tctccgcctc ctccttcttc 1320
   ctcgatcgcg gtaactccaa ggttaagttt gtcaaggaga acccctactt tactaaccgt 1380
   atgagcgtca acaaccagcc ctttaagtcc gagaacgatc ttagctacta caaggtttac 1440
   ggcctcctcg accagaacat tctcgagctc tactttaacg acggagatgt cgtcagcacc 1500
   aacacctact ttatgaccac tggaaacgcc ctcggcagcg tgaacatgac caccggagtc 1560
   gacaacctct tttacattga caagtttcag gttcgcgagg ttaagtaaca tatg 1614
<210> 58
   <211> 11495
   <212> DNA
   <213> Artificial sequence
<220>
   <223> pCL0076
<400> 58
   ctcttatctg cctcgcgccg ttgaccgccg cttgactctt ggcgcttgcc gctcgcatcc     60
   tgcctcgctc gcgcaggcgg gcgggcgagt gggtgggtcc gcagccttcc gcgctcgccc    120
   gctagctcgc tcgcgccgtg ctgcagccag cagggcagca ccgcacggca ggcaggtccc    180
   ggcgcggatc gatcgatcca tcgatccatc gatccatcga tcgtgcggtc aaaaagaaag    240
   gaagaagaaa ggaaaaagaa aggcgtgcgc acccgagtgc gcgctgagcg cccgctcgcg    300
   gtcccgcgga gcctccgcgt tagtccccgc cccgcgccgc gcagtccccc gggaggcatc    360
   gcgcacctct cgccgccccc tcgcgcctcg ccgattcccc gcctcccctt ttccgcttct    420
   tcgccgcctc cgctcgcggc cgcgtcgccc gcgccccgct ccctatctgc tccccagggg    480
   ggcactccgc accttttgcg cccgctgccg ccgccgcggc cgccccgccg ccctggtttc    540
   ccccgcgagc gcggccgcgt cgccgcgcaa agactcgccg cgtgccgccc cgagcaacgg    600
   gtggcggcgg cgcggcggcg ggcggggcgc ggcggcgcgt aggcggggct aggcgccggc    660
   taggcgaaac gccgcccccg ggcgccgccg ccgcccgctc cagagcagtc gccgcgccag    720
   accgccaacg cagagaccga gaccgaggta cgtcgcgccc gagcacgccg cgacgcgcgg    780
   cagggacgag gagcacgacg ccgcgccgcg ccgcgcgggg ggggggaggg agaggcagga 840
   cgcgggagcg agcgtgcatg tttccgcgcg agacgacgcc gcgcgcgctg gagaggagat 900
   aaggcgcttg gatcgcgaga gggccagcca ggctggaggc gaaaatgggt ggagaggata 960
   gtatcttgcg tgcttggacg aggagactga cgaggaggac ggatacgtcg atgatgatgt 1020
   gcacagagaa gaagcagttc gaaagcgact actagcaagc aagggatcca tgaagttcgc 1080
   gacctcggtc gcaattttgc ttgtggccaa catagccacc gccctcgcgt cgatgaccaa 1140
   cgagacctcg gaccgccctc tcgtgcactt tacccccaac aagggttgga tgaacgatcc 1200
   caacggcctc tggtacgacg agaaggatgc taagtggcac ctttactttc agtacaaccc 1260
   taacgacacc gtctggggca ccccgctctt ctggggccac gccacctccg acgacctcac 1320
   caactgggag gaccagccca ttgctatcgc ccccaagcgc aacgactcgg gagctttttc 1380
   cggttccatg gttgtggact acaacaacac ctccggtttt tttaacgaca ccattgaccc 1440
   ccgccagcgc tgcgtcgcca tctggaccta caacacgccc gagagcgagg agcagtacat 1500
   cagctacagc cttgatggag gctacacctt taccgagtac cagaagaacc ctgtcctcgc 1560
   cgccaactcc acccagttcc gcgaccctaa ggttttttgg tacgagcctt cccagaagtg 1620
   gattatgacc gccgctaagt cgcaggatta caagatcgag atctacagca gcgacgacct 1680
   caagtcctgg aagcttgagt ccgcctttgc caacgagggt tttctcggat accagtacga 1740
   gtgccccggt ctcatcgagg tccccaccga gcaggacccg tccaagtcct actgggtcat 1800
   gtttatttcc atcaaccctg gcgcccctgc cggcggcagc ttcaaccagt acttcgtcgg 1860
   ctcctttaac ggcacgcatt ttgaggcctt cgacaaccag tcccgcgtcg tcgacttcgg 1920
   caaggactac tacgccctcc agaccttctt taacaccgac cccacctacg gcagcgccct 1980
   cggtattgct tgggcctcca actgggagta ctccgctttc gtccccacta acccctggcg 2040
   cagctcgatg tccctcgtcc gcaagttttc gcttaacacc gagtaccagg ccaaccccga 2100
   gaccgagctt attaacctga aggccgagcc tattctcaac atctccaacg ctggcccctg 2160
   gtcccgcttt gctactaaca ctaccctcac caaggccaac tcctacaacg tcgatctctc 2220
   caactccacc ggtactcttg agtttgagct cgtctacgcc gtcaacacca cccagaccat 2280
   ctccaagtcc gtcttcgccg acctctccct ctggttcaag ggccttgagg accccgagga 2340
   gtacctgcgc atgggttttg aggtctccgc ctcctccttc ttcctcgatc gcggtaactc 2400
   caaggttaag tttgtcaagg agaaccccta ctttactaac cgtatgagcg tcaacaacca 2460
   gccctttaag tccgagaacg atcttagcta ctacaaggtt tacggcctcc tcgaccagaa 2520
   cattctcgag ctctacttta acgacggaga tgtcgtcagc accaacacct actttatgac 2580
   cactggaaac gccctcggca gcgtgaacat gaccaccgga gtcgacaacc tcttttacat 2640
   tgacaagttt caggttcgcg aggttaagta acatatgtta tgagagatcc gaaagtgaac 2700
   cttgtcctaa cccgacagcg aatggcggga gggggcgggc taaaagatcg tattacatag 2760
   tatttttccc ctactctttg tgtttgtctt tttttttttt ttgaacgcat tcaagccact 2820
   tgtctgggtt tacttgtttg tttgcttgct tgcttgcttg cttgcctgct tcttggtcag 2880
   acggcccaaa aaagggaaaa aattcattca tggcacagat aagaaaaaga aaaagtttgt 2940
   cgaccaccgt catcagaaag caagagaaga gaaacactcg cgctcacatt ctcgctcgcg 3000
   taagaatctt agccacgcat acgaagtaat ttgtccatct ggcgaatctt tacatgagcg 3060
   ttttcaagct ggagcgtgag atcatacctt tcttgatcgt aatgttccaa ccttgcatag 3120
   gcctcgttgc gatccgctag caatgcgtcg tactcccgtt gcaactgcgc catcgcctca 3180
   ttgtgacgtg agttcagatt cttctcgaga ccttcgagcg ctgctaattt cgcctgacgc 3240
   tccttctttt gtgcttccat gacacgccgc ttcaccgtgc gttccacttc ttcctcagac 3300
   atgcccttgg ctgcctcgac ctgctcggta agcttcgtcg taatctcctc gatctcggaa 3360
   ttcttcttgc cctccatcca ctcggcacca tacttggcag cctgttcaac acgctcattg 3420
   aaaaactttt cattctcttc cagctccgca acccgcgctc gaagctcatt cacttccgcc 3480
   accacggctt cggcatcgag cgccgaatca gtcgccgaac tttccgaaag atacaccacg 3540
   gcccctccgc tgctgctgcg cagcgtcatc atcagtcgcg tgttatcttc gcgcagattc 3600
   tccacctgct ccgtaagcag cttcacggtg gcctcttgat tctgagggct cacgtcgtgg 3660
   attagcgctt gcagctcttg cagctccgtc agcttggaag agctcgtaat catggctttg 3720
   cacttgtcca gacgtcgcag agcgttcgag agccgcttcg cgttatctgc catggacgct 3780
   tctgcgctcg cggcctccct gacgacagtc tcttgcagtt tcactagatc atgtccaatc 3840
   agcttgcggt gcagctctcc aatcacgttc tgcatcttgt ttgtgtgtcc gggccgcgcc 3900
   tcgtcttgcg atttgcgaat ttcctcctcg agctcgcgtt cgagctccag ggcgccttta 3960
   agtagctcga agtcagccgc cgttagcccc agctccgtcg ccgcgttcag acagtcggtt 4020
   agcttgattc gattccgctt ttccatggca agtttaagat cctggcccag ctgcacctcc 4080
   tgcgccttgc gcatcatgcg cggttccgcc tggcgcaaaa gcttcgagtc gtatcctgcc 4140
   tgccatgcca gcgcaatggc acgcacgagc gacttgagtt gccaactatt catcgccgag 4200
   atgagcagca ttttgatctg catgaacacc tcgtcagagt cgtcatcctc tgcctcctcc 4260
   agctctgcgg gcgagcgacg ctctccttgc agatgaagcg agggccgcag gcctccgaag 4320
   agcacctctt gcgcgagatc ctcctccgtc gtcgccctcc gcaggattgc ggtcgtgtcc 4380
   gccatcttgc cgccacagca gcttttgctc gctctgcacc ttcaatttct ggtgccgctg 4440
   gtgccgctgg tgccgcttgt gctggtgctg gtgctggtgc tggtgctggt gccttgtgct 4500
   ggtgctgcca cagacaccgc cgctcctgct gctgctcttc cggccccctc gccgccgccg 4560
   cgagcccccg ccgcgcgccg tgcctgggct ctccgcgctc tccgcgggct cctcggcctc 4620
   ggcctcgccg tccgcgacga cgtctgcgcg gccgatggtg cggatctgct ctagagggcc 4680
   cttcgaaggt aagcctatcc ctaaccctct cctcggtctc gattctacgc gtaccggtca 4740
   tcatcaccat caccattgag tttaaacggg ccccagcacg tgctacgaga tttcgattcc 4800
   accgccgcct tctatgaaag gttgggcttc ggaatcgttt tccgggacgc cggctggatg 4860
   atcctccagc gcggggatct catgctggag ttcttcgccc accccaactt gtttattgca 4920
   gcttataatg gttacaaata aagcaatagc atcacaaatt tcacaaataa agcatttttt 4980
   tcactgcatt ctagttgtgg tttgtccaaa ctcatcaatg tatcttatca tacatggtcg 5040
   acctgcagga acctgcatta atgaatcggc caacgcgcgg ggagaggcgg tttgcgtatt 5100
   gggcgctctt ccgcttcctc gctcactgac tcgctgcgct cggtcgttcg gctgcggcga 5160
   gcggtatcag ctcactcaaa ggcggtaata cggttatcca cagaatcagg ggataacgca 5220
   ggaaagaaca tgtgagcaaa aggccagcaa aaggccagga accgtaaaaa ggccgcgttg 5280
   ctggcgtttt tccataggct ccgcccccct gacgagcatc acaaaaatcg acgctcaagt 5340
   cagaggtggc gaaacccgac aggactataa agataccagg cgtttccccc tggaagctcc 5400
   ctcgtgcgct ctcctgttcc gaccctgccg cttaccggat acctgtccgc ctttctccct 5460
   tcgggaagcg tggcgctttc tcatagctca cgctgtaggt atctcagttc ggtgtaggtc 5520
   gttcgctcca agctgggctg tgtgcacgaa ccccccgttc agcccgaccg ctgcgcctta 5580
   tccggtaact atcgtcttga gtccaacccg gtaagacacg acttatcgcc actggcagca 5640
   gccactggta acaggattag cagagcgagg tatgtaggcg gtgctacaga gttcttgaag 5700
   tggtggccta actacggcta cactagaaga acagtatttg gtatctgcgc tctgctgaag 5760
   ccagttacct tcggaaaaag agttggtagc tcttgatccg gcaaacaaac caccgctggt 5820
   agcggtggtt tttttgtttg caagcagcag attacgcgca gaaaaaaagg atctcaagaa 5880
   gatcctttga tcttttctac ggggtctgac gctcagtgga acgaaaactc acgttaaggg 5940
   attttggtca tgagattatc aaaaaggatc ttcacctaga tccttttaaa ttaaaaatga 6000
   agttttaaat caatctaaag tatatatgag taaacttggt ctgacagtta ccaatgctta 6060
   atcagtgagg cacctatctc agcgatctgt ctatttcgtt catccatagt tgcctgactc 6120
   cccgtcgtgt agataactac gatacgggag ggcttaccat ctggccccag tgctgcaatg 6180
   ataccgcgag acccacgctc accggctcca gatttatcag caataaacca gccagccgga 6240
   agggccgagc gcagaagtgg tcctgcaact ttatccgcct ccatccagtc tattaattgt 6300
   tgccgggaag ctagagtaag tagttcgcca gttaatagtt tgcgcaacgt tgttgccatt 6360
   gctacaggca tcgtggtgtc acgctcgtcg tttggtatgg cttcattcag ctccggttcc 6420
   caacgatcaa ggcgagttac atgatccccc atgttgtgca aaaaagcggt tagctccttc 6480
   ggtcctccga tcgttgtcag aagtaagttg gccgcagtgt tatcactcat ggttatggca 6540
   gcactgcata attctcttac tgtcatgcca tccgtaagat gcttttctgt gactggtgag 6600
   tactcaacca agtcattctg agaatagtgt atgcggcgac cgagttgctc ttgcccggcg 6660
   tcaatacggg ataataccgc gccacatagc agaactttaa aagtgctcat cattggaaaa 6720
   cgttcttcgg ggcgaaaact ctcaaggatc ttaccgctgt tgagatccag ttcgatgtaa 6780
   cccactcgtg cacccaactg atcttcagca tcttttactt tcaccagcgt ttctgggtga 6840
   gcaaaaacag gaaggcaaaa tgccgcaaaa aagggaataa gggcgacacg gaaatgttga 6900
   atactcatac tcttcctttt tcaatattat tgaagcattt atcagggtta ttgtctcatg 6960
   agcggataca tatttgaatg tatttagaaa aataaacaaa taggggttcc gcgcacattt 7020
   ccccgaaaag tgccacctga cgtctaagaa accattatta tcatgacatt aacctataaa 7080
   aataggcgta tcacgaggcc ctttcgtctc gcgcgtttcg gtgatgacgg tgaaaacctc 7140
   tgacacatgc agctcccgga gacggtcaca gcttgtctgt aagcggatgc cgggagcaga 7200
   caagcccgtc agggcgcgtc agcgggtgtt ggcgggtgtc ggggctggct taactatgcg 7260
   gcatcagagc agattgtact gagagtgcac caagctttgc ctcaacgcaa ctaggcccag 7320
   gcctactttc actgtgtctt gtcttgcctt tcacaccgac cgagtgtgca caaccgtgtt 7380
   ttgcacaaag cgcaagatgc tcactcgact gtgaagcaaa ggttgcgcgc aagcgactgc 7440
   gactgcgagg atgaggatga ctggcagcct gttcaaaaac tgaaaatccg cgatgggtca 7500
   gctgccattc gcgcatgacg cctgcgagag acaagttaac tcgtgtcact ggcatgtcct 7560
   agcatcttta cgcgagcaaa attcaatcgc tttatttttt cagtttcgta accttctcgc 7620
   aaccgcgaat cgccgtttca gcctgactaa tctgcagctg cgtggcactg tcagtcagtc 7680
   agtcagtcgt gcgcgctgtt ccagcaccga ggtcgcgcgt cgccgcgcct ggaccgctgc 7740
   tgctactgct agtggcacgg caggtaggag cttgttgccg gaacaccagc agccgccagt 7800
   cgacgccagc caggggaaag tccggcgtcg aagggagagg aaggcggcgt gtgcaaacta 7860
   acgttgacca ctggcgcccg ccgacacgag caggaagcag gcagctgcag agcgcagcgc 7920
   gcaagtgcag aatgcgcgaa agatccactt gcgcgcggcg ggcgcgcact tgcgggcgcg 7980
   gcgcggaaca gtgcggaaag gagcggtgca gacggcgcgc agtgacagtg ggcgcaaagc 8040
   cgcgcagtaa gcagcggcgg ggaacggtat acgcagtgcc gcgggccgcc gcacacagaa 8100
   gtatacgcgg gccgaagtgg ggcgtcgcgc gcgggaagtg cggaatggcg ggcaaggaaa 8160
   ggaggagacg gaaagagggc gggaaagaga gagagagaga gtgaaaaaag aaagaaagaa 8220
   agaaagaaag aaagaaagct cggagccacg ccgcggggag agagagaaat gaaagcacgg 8280
   cacggcaaag caaagcaaag cagacccagc cagacccagc cgagggagga gcgcgcgcag 8340
   gacccgcgcg gcgagcgagc gagcacggcg cgcgagcgag cgagcgagcg agcgcgcgag 8400
   cgagcaaggc ttgctgcgag cgatcgagcg agcgagcggg aaggatgagc gcgacccgcg 8460
   cggcgacgag gacagcggcg gcgctgtcct cggcgctgac gacgcctgta aagcagcagc 8520
   agcagcagca gctgcgcgta ggcgcggcgt cggcacggct ggcggccgcg gcgttctcgt 8580
   ccggcacggg cggagacgcg gccaagaagg cggccgcggc gagggcgttc tccacgggac 8640
   gcggccccaa cgcgacacgc gagaagagct cgctggccac ggtccaggcg gcgacggacg 8700
   atgcgcgctt cgtcggcctg accggcgccc aaatctttca tgagctcatg cgcgagcacc 8760
   aggtggacac catctttggc taccctggcg gcgccattct gcccgttttt gatgccattt 8820
   ttgagagtga cgcgcttcaa gttcattctc gctcgccacg agcagggcgc cggccacatg 8880
   gccgagggct acgcgcgcgc cacgggcaag cccggcgttg tcctcgtcac ctcgggccct 8940
   ggagccacca acaccatcac cccgatcatg gatgcttaca tggacggtac gccgctgctc 9000
   gtgttcaccg gccaggtgca gacctctgct gtcggcacgg acgctttcca ggagtgtgac 9060
   attgttggca tcagccgcgc gtgcaccaag tggaacgtca tggtcaagga cgtgaaggag 9120
   ctcccgcgcc gcatcaatga ggcctttgag attgccatga gcggccgccc gggtcccgtg 9180
   ctcgtcgatc ttcctaagga tgtgaccgcc gttgagctca aggaaatgcc cgacagctcc 9240
   ccccaggttg ctgtgcgcca gaagcaaaag gtcgagcttt tccacaagga gcgcattggc 9300
   gctcctggca cggccgactt caagctcatt gccgagatga tcaaccgtgc ggagcgaccc 9360
   gtcatctatg ctggccaggg tgtcatgcag agcccgttga atggcccggc tgtgctcaag 9420
   gagttcgcgg agaaggccaa cattcccgtg accaccacca tgcagggtct cggcggcttt 9480
   gacgagcgta gtcccctctc cctcaagatg ctcggcatgc acggctctgc ctacgccaac 9540
   tactcgatgc agaacgccga tcttatcctg gcgctcggtg cccgctttga tgatcgtgtg 9600
   acgggccgcg ttgacgcctt tgctccggag gctcgccgtg ccgagcgcga gggccgcggt 9660
   ggcatcgttc actttgagat ttcccccaag aacctccaca aggtcgtcca gcccaccgtc 9720
   gcggtcctcg gcgacgtggt cgagaacctc gccaacgtca cgccccacgt gcagcgccag 9780
   gagcgcgagc cgtggtttgc gcagatcgcc gattggaagg agaagcaccc ttttctgctc 9840
   gagtctgttg attcggacga caaggttctc aagccgcagc aggtcctcac ggagcttaac 9900
   aagcagattc tcgagattca ggagaaggac gccgaccagg aggtctacat caccacgggc 9960
   gtcggaagcc accagatgca ggcagcgcag ttccttacct ggaccaagcc gcgccagtgg 10020
   atctcctcgg gtggcgccgg cactatgggc tacggccttc cctcggccat tggcgccaag 10080
   attgccaagc ccgatgctat tgttattgac atcgatggtg atgcttctta ttcgatgacc 10140
   ggtatggaat tgatcacagc agccgaattc aaggttggcg tgaagattct tcttttgcag 10200
   aacaactttc agggcatggt caagaacgtt caggatctct tttacgacaa gcgctactcg 10260
   ggccaccgcc atgttcaacc cgcgcttcga caaggtcgcc gatgcgatgc gtgccaaggg 10320
   tctctactgc gcgaaacagt cggagctcaa ggacaagatc aaggagtttc tcgagtacga 10380
   tgagggtccc gtcctcctcg aggttttcgt ggacaaggac acgctcgtct tgcccatggt 10440
   ccccgctggc tttccgctcc acgagatggt cctcgagcct cctaagccca aggacgccta 10500
   agttcttttt tccatggcgg gcgagcgagc gagcgcgcga gcgcgcaagt gcgcaagcgc 10560
   cttgccttgc tttgcttcgc ttcgctttgc tttgcttcac acaacctaag tatgaattca 10620
   agttttcttg cttgtcggcg atgcctgcct gccaaccagc cagccatccg gccggccgtc 10680
   cttgacgcct tcgcttccgg cgcggccatc gattcaattc acccatccga tacgttccgc 10740
   cccctcacgt ccgtctgcgc acgacccctg cacgaccacg ccaaggccaa cgcgccgctc 10800
   agctcagctt gtcgacgagt cgcacgtcac atatctcaga tgcatttgga ctgtgagtgt 10860
   tattatgcca ctagcacgca acgatcttcg gggtcctcgc tcattgcatc cgttcgggcc 10920
   ctgcaggcgt ggacgcgagt cgccgccgag acgctgcagc aggccgctcc gacgcgaggg 10980
   ctcgagctcg ccgcgcccgc gcgatgtctg cctggcgccg actgatctct ggagcgcaag 11040
   gaagacacgg cgacgcgagg aggaccgaag agagacgctg gggtatgcag gatatacccg 11100
   gggcgggaca ttcgttccgc atacactccc ccattcgagc ttgctcgtcc ttggcagagc 11160
   cgagcgcgaa cggttccgaa cgcggcaagg attttggctc tggtgggtgg actccgatcg 11220
   aggcgcaggt tctccgcagg ttctcgcagg ccggcagtgg tcgttagaaa tagggagtgc 11280
   cggagtcttg acgcgcctta gctcactctc cgcccacgcg cgcatcgccg ccatgccgcc 11340
   gtcccgtctg tcgctgcgct ggccgcgacc ggctgcgcca gagtacgaca gtgggacaga 11400
   gctcgaggcg acgcgaatcg ctcgggttgt aagggtttca agggtcgggc gtcgtcgcgt 11460
   gccaaagtga aaatagtagg gggggggggg ggtac 11495
<210> 59
   <211> 32
   <212> PRT
   <213> Schizochytrium
<400> 59
   Met Arg Thr Val Arg Gly Pro Gln Thr Ala Ala Leu Ala Ala Leu Leu
   1 5 10 15
   Ala Leu Ala Ala Thr His Val Ala Val Ser Pro Phe Thr Lys Val Glu
   20 25 30
<210> 60
   <211> 96
   <212> DNA
   <213> Schizochytrium
<400> 60
   atgcgcacgg tgagggggcc gcaaacggcg gcactcgccg cccttctggc acttgccgcg 60
   acgcacgtgg ctgtgagccc gttcaccaag gtggag 96
<210> 61
   <211> 30
   <212> PRT
   <213> Schizochytrium
<400> 61
   Met Gly Arg Leu Ala Lys Ser Leu Val Leu Leu Thr Ala Val Leu Ala
   1 5 10 15
   Val Ile Gly Gly Val Arg Ala Glu Glu Asp Lys Ser Glu Ala
   20 25 30
<210> 62
   <211> 90
   <212> DNA
   <213> Schizochytrium
<400> 62
   atgggccgcc tcgcgaagtc gcttgtgctg ctgacggccg tgctggccgt gatcggaggc 60
   gtccgcgccg aagaggacaa gtccgaggcc 90
<210> 63
   <211> 38
   <212> PRT
   <213> Schizochytrium
<400> 63
   Met Thr Ser Thr Ala Arg Ala Leu Ala Leu Val Arg Ala Leu Val Leu
   1 5 10 15
   Ala Leu Ala Val Leu Ala Leu Leu Ala Ser Gln Ser Val Ala Val Asp
   20 25 30
   Arg Lys Lys Phe Arg Thr
   35
<210> 64
   <211> 114
   <212> DNA
   <213> Schizochytrium
<400> 64
   atgacgtcaa cggcgcgcgc gctcgcgctc gtgcgtgctt tggtgctcgc tctggctgtc     60
   ttggcgctgc tagcgagcca aagcgtggcc gtggaccgca aaaagttcag gacc          114
<210> 65
   <211> 34
   <212> PRT
   <213> Schizochytrium
<400> 65
   Met Leu Arg Leu Lys Pro Leu Leu Leu Leu Phe Leu Cys Ser Leu Ile
   1               5                   10                  15
   Ala Ser Pro Val Val Ala Trp Ala Arg Gly Gly Glu Gly Pro Ser Thr
               20                  25                  30
   Ser Glu
<210> 66
   <211> 102
   <212> DNA
   <213> Schizochytrium
<400> 66
   atgttgcggc tcaagccact tttactcctc ttcctctgct cgttgattgc ttcgcctgtg     60
   gttgcctggg caagaggagg agaagggccg tccacgagcg aa                       102
<210> 67
   <211> 29
   <212> PRT
   <213> Schizochytrium
<400> 67
   Met Ala Lys Ile Leu Arg Ser Leu Leu Leu Ala Ala Val Leu Val Val
   1               5                   10                  15
   Thr Pro Gln Ser Leu Arg Ala His Ser Thr Arg Asp Ala
               20                  25
<210> 68
   <211> 87
   <212> DNA
   <213> Schizochytrium
<400> 68
   atggccaaga tcttgcgcag tttgctcctg gcggccgtgc tcgtggtgac tcctcaatca 60
   ctgcgtgctc attcgacgcg ggacgca 87
<210> 69
   <211> 36
   <212> PRT
   <213> Schizochytrium
<400> 69
   Met Val Phe Arg Arg Val Pro Trp His Gly Ala Ala Thr Leu Ala Ala
   1 5 10 15
   Leu Val Val Ala Cys Ala Thr Cys Leu Gly Leu Gly Leu Asp Ser Glu
   20 25 30
   Glu Ala Thr Tyr
   35
<210> 70
   <211> 108
   <212> DNA
   <213> Schizochytrium
<400> 70
   atggtgtttc ggcgcgtgcc atggcacggc gcggcgacgc tggcggcctt ggtcgtggcc 60
   tgcgcgacgt gtttaggcct gggactggac tcggaggagg ccacgtac 108
<210> 71
   <211> 30
   <212> PRT
   <213> Schizochytrium
<400> 71
   Met Thr Ala Asn Ser Val Lys Ile Ser Ile Val Ala Val Leu Val Ala
   1 5 10 15
   Ala Leu Ala Trp Glu Thr Cys Ala Lys Ala Asn Tyr Gln Trp
   20 25 30
<210> 72
   <211> 90
   <212> DNA
   <213> Schizochytrium
<400> 72
   atgacagcta actcggtgaa aataagcatc gtggctgtgc tggtcgcggc actggcttgg 60
   gaaacatgcg caaaagctaa ctatcagtgg 90
<210> 73
   <211> 35
   <212> PRT
   <213> Schizochytrium
<400> 73
   Met Ala Arg Arg Ala Ser Arg Leu Gly Ala Ala Val Val Val Val Leu
   1 5 10 15
   Val Val Val Ala Ser Ala Cys Cys Trp Gln Ala Ala Ala Asp Val Val
   20 25 30
   Asp Ala Gln
   35
<210> 74
   <211> 105
   <212> DNA
   <213> Schizochytrium
<400> 74
   atggcgcgca gggcgtcgcg cctcggcgcc gccgtcgtcg tcgtcctcgt cgtcgtcgcc 60
   tccgcctgct gctggcaagc cgctgcggac gtcgtggacg cgcag 105
<210> 75
   <211> 1785
   <212> DNA
   <213> Artificial Sequence
<220>
   <223> Codon optimized nucleic acid sequence
<400> 75
   atgaagttcg cgacctcggt cgcaattttg cttgtggcca acatagccac cgccctcgcg      60
   gcctccccct cgatgcagac ccgtgcctcc gtcgtcattg attacaacgt cgctcctcct     120
   aacctctcca ccctcccgaa cggcagcctc tttgagacct ggcgtcctcg cgcccacgtt     180
   cttcccccta acggtcagat tggcgatccc tgcctccact acaccgatcc ctcgactggc     240
   ctctttcacg tcggctttct ccacgatggc tccggcattt cctccgccac tactgacgac     300
   ctcgctacct acaaggatct caaccagggc aaccaggtca tcgtccccgg cggtatcaac     360
   gaccctgtcg ctgttttcga cggctccgtc attccttccg gcattaacgg cctccctacc     420
   ctcctctaca cctccgtcag ctacctcccc attcactggt ccatccccta cacccgcggt     480
   tccgagacgc agagcctggc tgtctccagc gatggtggct ccaactttac taagctcgac     540
   cagggccccg ttattcctgg cccccccttt gcctacaacg tcaccgcctt ccgcgacccc     600
   tacgtctttc agaaccccac cctcgactcc ctcctccact ccaagaacaa cacctggtac     660
   accgtcattt cgggtggcct ccacggcaag ggccccgccc agtttcttta ccgtcagtac     720
   gaccccgact ttcagtactg ggagttcctc ggccagtggt ggcacgagcc taccaactcc     780
   acctggggca acggcacctg ggccggccgc tgggccttca acttcgagac cggcaacgtc     840
   ttttcgcttg acgagtacgg ctacaacccc cacggccaga tcttctccac cattggcacc     900
   gagggctccg accagcccgt tgtcccccag ctcacctcca tccacgatat gctttgggtc     960
   tccggtaacg tttcgcgcaa cggatcggtt tccttcactc ccaacatggc cggcttcctc    1020
   gactggggtt tctcgtccta cgccgccgcg ggtaaggttc ttccttccac gtcgctcccc    1080
   tccaccaagt ccggtgcccc cgatcgcttc atttcgtacg tttggctctc cggcgacctc    1140
   tttgagcagg ctgagggctt tcctaccaac cagcagaact ggaccggcac cctcctcctc    1200
   ccccgtgagc tccgcgtcct ttacatcccc aacgtggttg ataacgccct tgcgcgcgag    1260
   tccggcgctt cctggcaggt cgtctcctcc gatagctcgg ccggtactgt ggagctccag    1320
   accctcggca tttccatcgc ccgcgagacc aaggccgccc tcctgtccgg cacctcgttc    1380
   actgagtccg accgcactct taactcctcc ggcgtcgttc cctttaagcg ttccccctcc   1440
   gagaagtttt tcgtcctctc cgcccagctc tccttccccg cctccgcccg cggctcgggc    1500
   ctcaagtccg gcttccagat tctttcctcc gagctcgagt ccaccacggt ctactaccag    1560
   tttagcaacg agtccatcat cgtcgaccgc agcaacacca gcgccgccgc ccgtactacc    1620
   gacggtatcg actcctccgc cgaggccggc aagctccgcc tctttgacgt cctcaacggc    1680
   ggcgagcagg ctattgagac cctcgacctt accctcgtcg ttgataactc cgtgctcgag    1740
   atttacgcca acggtcgttt cgcgctttcc acctgggttc gctaa                     1785
<210> 76
   <211> 1682
   <212> DNA
   <213> Artificial Sequence
<220>
   <223> Codon Optimized HA
<400> 76
   atgaaggcta acctcctcgt tcttctttcc gctctcgctg ctgcggatgc cgacaccatc      60
   tgcattggct accacgctaa caacagcacg gacaccgtcg atactgtcct ggagaagaac     120
   gttaccgcac ccattcggtc aacctcctgg aggacagcca caacggcaag ctctgccgtc     180
   ttaagggcat cgcccccctc cagctcggca agtgcaacat cgccggctgg ctcctcggca     240
   acccggagtg cgatccctcc tccccgttcg ctcctggtcg tacattgtgg agactccgaa     300
   cagcgagaac ggtatctgct accccggcga ttttatcgac tacgaggagc tccgcgagca     360
   gctctcctcc gtgtccagct tgagcgtttc gagatttttc cgaaggagtc ctcgtggccc     420
   aaccacaaca ccaacggcgt caccgccgcc tgctcccacg agggcaagtc gagcttttac     480
   cgcaacctgc tttggctcac cgagaagggg gttcgtaccc taagctcaag aactcgtacg     540
   tcaacaagaa gggcaaggag gtcctcgtcc tctggggcat ccaccatccc ccgaacagca     600
   aggagcagca gaacatctac cagaacgaga acgccacgtt tcggtggtca cgtcgaacta     660
   caaccgccgc ttcactcctg agatcgccga gcgccccaag gtgcgcgacc aggctggccg     720
   catgaactac tactggaccc tccttaagcc cggtgacacg atatctttga ggccaacggc     780
   aaccttatcg cgcccatgta cgcgttcgcc ctctcccgcg gctttggtag cggcatcatt     840
   accagcaacg ccagcatgca cgagtgcaac acgaagtgcc agaccccgcc ggtgccatca     900
   acagcagcct gccttaccag aacatccacc ccgtcaccat cggtgagtgc ccgaagtacg 960
   tgcgctcggc caagctccgc atggtcacgg gcctccgcaa cactccttcg atccagcccg 1020
   cggcctcttc ggcgccattg ccggtttcat cgagggcggc tggacgggca tgatcgacgg 1080
   ctggtacggc taccaccacc agaacgagca gggctccggt tacgccgcgg accagaagtc 1140
   caccagaacg ccatcaacgg cattactaac aaggtcaaca cggtcatcga gaagatgaac 1200
   attcagttta ccgctgtcgg caaggagttc aacaagctgg agaagcgcat ggagaacctc 1260
   aacaagaagg ggacgatggt ttcctggaca tttggaccta caacgccgag ctcctcgtgc 1320
   tccttgagaa cgagcgtacc ctcgacttcc acgactccaa cgtcaagaac ctctacgaga 1380
   aggtcaagtc gcagctcaga acaacgccaa ggagattggc aacggttgct tcgagtttta 1440
   ccacaagtgc gacaacgagt gcatggagtc cgtccgcaac ggcacctacg actacccgaa 1500
   gtactccgag gagtcgaagc tgaacgcgag aaggtggacg gcgtgaagct ggagtccatg 1560
   ggcatctacc agatcctcgc catttactcg acggttgcct cgtcgctcgt cctccttgtc 1620
   tccctcggtg cgatttcgtt ctggatgtgc tgaacggcag ccttcagtgc cgcatctgca 1680
   tc 1682
<210> 77
   <211> 565
   <212> **PRT**
   <213> **Influenza A** virus
<400> 77
   Met Lys Ala Asn Leu Leu Val Leu Leu Ser Ala Leu Ala Ala Ala Asp
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Asp Ser His Asn Gly Lys Leu Cys Arg Leu Lys Gly Ile
       50                  55                  60
   Ala Pro Leu Gln Leu Gly Lys Cys Asn Ile Ala Gly Trp Leu Leu Gly
   65                  70                  75                  80
   Asn Pro Glu Cys Asp Pro Leu Leu Pro Val Arg Ser Trp Ser Tyr Ile
                   85                  90                  95
   Val Glu Thr Pro Asn Ser Glu Asn Gly Ile Cys Tyr Pro Gly Asp Phe
               100                 105                 110
   Ile Asp Tyr Glu Glu Leu Arg Glu Gln Leu Ser Ser Val Ser Ser Phe
           115                 120                 125
   Glu Arg Phe Glu Ile Phe Pro Lys Glu Ser Ser Trp Pro Asn His Asn
       130                 135                 140
   Thr Asn Gly Val Thr Ala Ala Cys Ser His Glu Gly Lys Ser Ser Phe
   145                 150                 155                 160
   Tyr Arg Asn Leu Leu Trp Leu Thr Glu Lys Glu Gly Ser Tyr Pro Lys
                   165                 170                 175
   Leu Lys Asn Ser Tyr Val Asn Lys Lys Gly Lys Glu Val Leu Val Leu
               180                 185                 190
   Trp Gly Ile His His Pro Pro Asn Ser Lys Glu Gln Gln Asn Ile Tyr
           195                 200                 205
   Gln Asn Glu Asn Ala Tyr Val Ser Val Val Thr Ser Asn Tyr Asn Arg
       210                 215                 220
   Arg Phe Thr Pro Glu Ile Ala Glu Arg Pro Lys Val Arg Asp Gln Ala
   225                 230                 235                 240
   Gly Arg Met Asn Tyr Tyr Trp Thr Leu Leu Lys Pro Gly Asp Thr Ile
                   245                 250                 255
   Ile Phe Glu Ala Asn Gly Asn Leu Ile Ala Pro Met Tyr Ala Phe Ala
               260                 265                 270
   Leu Ser Arg Gly Phe Gly Ser Gly Ile Ile Thr Ser Asn Ala Ser Met
           275                 280                 285
   His Glu Cys Asn Thr Lys Cys Gln Thr Pro Leu Gly Ala Ile Asn Ser
       290                 295                 300
   Ser Leu Pro Tyr Gln Asn Ile His Pro Val Thr Ile Gly Glu Cys Pro
   305                 310                 315                 320
   Lys Tyr Val Arg Ser Ala Lys Leu Arg Met Val Thr Gly Leu Arg Asn
                   325                 330                 335
   Thr Pro Ser Ile Gln Ser Arg Gly Leu Phe Gly Ala Ile Ala Gly Phe
               340                 345                 350
   Ile Glu Gly Gly Trp Thr Gly Met Ile Asp Gly Trp Tyr Gly Tyr His
               355                 360                 365
   His Gln Asn Glu Gln Gly Ser Gly Tyr Ala Ala Asp Gln Lys Ser Thr
       370                 375                 380
   Gln Asn Ala Ile Asn Gly Ile Thr Asn Lys Val Asn Thr Val Ile Glu
   385                 390                 395                 400
   Lys Met Asn Ile Gln Phe Thr Ala Val Gly Lys Glu Phe Asn Lys Leu
                   405                 410                 415
   Glu Lys Arg Met Glu Asn Leu Asn Lys Lys Val Asp Asp Gly Phe Leu
               420                 425                 430
   Asp Ile Trp Thr Tyr Asn Ala Glu Leu Leu Val Leu Leu Glu Asn Glu
           435                 440                 445
   Arg Thr Leu Asp Phe His Asp Ser Asn Val Lys Asn Leu Tyr Glu Lys
       450                 455                 460
   Val Lys Ser Gln Leu Lys Asn Asn Ala Lys Glu Ile Gly Asn Gly Cys
   465                 470                 475                 480
   Phe Glu Phe Tyr His Lys Cys Asp Asn Glu Cys Met Glu Ser Val Arg
                   485                 490                 495
   Asn Gly Thr Tyr Asp Tyr Pro Lys Tyr Ser Glu Glu Ser Lys Leu Asn
               500                 505                 510
   Arg Glu Lys Val Asp Gly Val Lys Leu Glu Ser Met Gly Ile Tyr Gln
           515                 520                 525
   Ile Leu Ala Ile Tyr Ser Thr Val Ala Ser Ser Leu Val Leu Leu Val
       530                 535                 540
   Ser Leu Gly Ala Ile Ser Phe Trp Met Cys Ser Asn Gly Ser Leu Gln
   545                 550                 555                 560
   Cys Arg Ile Cys Ile
                   565
<210> 78
   <211> 51
   <212> PRT
   <213> Schizochytrium
<220>
   <223> GlcNac-transferase-I-like protein
<400> 78
   Met Arg Gly Pro Gly Met Val Gly Leu Ser Arg Val Asp Arg Glu His
   1               5                   10                  15
   Leu Arg Arg Arg Gln Gln Gln Ala Ala Ser Glu Trp Arg Arg Trp Gly
               20                  25                  30
   Phe Phe Val Ala Thr Ala Val Val Leu Leu Val Phe Leu Thr Val Tyr
           35                  40                  45
   Pro Asn Val
       50
<210> 79
   <211> 153
   <212> **DNA**
   <213> Schizochytrium
<220>
   <223> signal anchor sequence
<400> 79
   atgcgcggcc cgggcatggt cggcctcagc cgcgtggacc gcgagcacct gcggcggcgg      60
   cagcagcagg cggcgagcga atggcggcgc tgggggttct tcgtcgcgac ggccgtcgtc     120
   ctgctcgtct ttctcaccgt atacccgaac gta                                  153
<210> 80
   <211> 66
   <212> PRT
   <213> Schizochytrium
<220>
   <223> beta-1,2- xylosyltransferase-like protein
<400> 80
   Met Arg Thr Arg Gly Ala Ala Tyr Val Arg Pro Gly Gln His Glu Ala
   1               5                   10                  15
   Lys Ala Leu Ser Ser Arg Ser Ser Asp Glu Gly Tyr Thr Thr Val Asn
               20                  25                  30
   Val Val Arg Thr Lys Arg Lys Arg Thr Thr Val Ala Ala Leu Val Ala
           35                  40                  45
   Ala Ala Leu Leu Val Thr Gly Phe Ile Val Val Val Val Phe Val Val
       50                  55                  60
   Val Val
   65
<210> 81
   <211> 198
   <212> **DNA**
   <213> Schizochytrium
<220>
   <223> signal anchor sequence
<400> 81
   atgcgcacgc ggggcgcggc gtacgtgcgg ccgggacagc acgaggcgaa ggcgctctcg     60
   tcaaggagca gcgacgaggg atatacgacg gtcaacgttg tcaggaccaa gcgaaagagg    120
   accactgtag ccgcgcttgt agccgcggcg ctgctggtga cgggctttat cgtcgtcgtc    180
   gtcttcgtcg tcgttgtt                                                  198
<210> 82
   <211> 64
   <212> PRT
   <213> Schizochytrium
<220>
   <223> beta-1,4-xylosidase-like protein
<400> 82
   Met Glu Ala Leu Arg Glu Pro Leu Ala Ala Pro Pro Thr Ser Ala Arg
   1               5                   10                  15
   Ser Ser Val Pro Ala Pro Leu Ala Lys Glu Glu Gly Glu Glu Glu Asp
               20                  25                  30
   Gly Glu Lys Gly Thr Phe Gly Ala Gly Val Leu Gly Val Val Ala Val
           35                  40                  45
   Leu Val Ile Val Val Phe Ala Ile Val Ala Gly Gly Gly Gly Asp Ile
       50                  55                  60
<210> 83
   <211> 192
   <212> **DNA**
   <213> Schizochytrium
<220>
   <223> signal anchor sequence
<400> 83
   atggaggccc tgcgcgagcc cttggctgcg ccgccaacgt cggcgcgatc gtcggtgcca     60
   gcgccgctcg cgaaggagga gggggaggag gaggacgggg aaaaagggac gtttggggcg    120
   ggggtcctcg gtgtcgtggc ggtgctcgtc atcgtggtgt ttgcgatcgt ggcgggaggc    180
   ggaggcgata tt                                                        192
<210> 84
   <211> 73
   <212> PRT
   <213> Schizochytrium
<220>
   <223> galactosyltransferase-like protein
<400> 84
   Met Leu Ser Val Ala Gln Val Ala Gly Ser Ala His Ser Arg Pro Arg
   1               5                   10                  15
   Arg Gly Gly Glu Arg Met Gln Asp Val Leu Ala Leu Glu Glu Ser Ser
               20                  25                  30
   Arg Asp Arg Lys Arg Ala Thr Ala Arg Pro Gly Leu Tyr Arg Ala Leu
           35                  40                  45
   Ala Ile Leu Gly Leu Pro Leu Ile Val Phe Ile Val Trp Gln Met Thr
       50                  55                  60
   Ser Ser Leu Thr Thr Ala Pro Ser Ala
   65                  70
<210> 85
   <211> 219
   <212> DNA
   <213> Schizochytrium
<220>
   <223> signal anchor sequence
<400> 85
   atgttgagcg tagcacaagt cgcggggtcg gcccactcgc ggccgagacg aggtggtgag 60
   cggatgcaag acgtgctggc cctggaggaa agcagcagag atcgaaaacg agcaacagca 120
   aggcccgggc tatatcgcgc acttgcgatt ctggggctgc cgctcatcgt attcatcgta 180
   tggcaaatga ctagctccct cacgactgcc ccgagcgcc 219
<210> 86
   <211> 997
   <212> PRT
   <213> Schizochytrium
<220>
   <223> EMC1
<400> 86
   Met Gly Thr Thr Thr Ala Arg Met Ala Val Ala Val Leu Ala Ala Ala
   1               5                   10                  15
   Val Ser Val Ala His Gly Leu His Glu Asp Gln Ala Gly Val Asn Asp
               20                  25                  30
   Trp Thr Val Arg Asn Leu Gly Ala Tyr Ala His Gly Val Phe Leu Asp
           35                  40                  45
   Asp Asp Leu Ala Leu Val Ala Thr Thr Gln Ala Thr Val Gly Ala Val
       50                  55                  60
   Arg Met Thr Asp Gly Glu Val Val Trp Arg Glu Thr Leu Pro Thr Ala
   65                  70                  75                  80
   Arg Ser Ala Pro Leu Ala Ser Gln Val Lys His Glu Leu Phe Ala Thr
                   85                  90                  95
   Ala Ser Ala Asp Ala Cys Val Ile Glu Leu Trp Ala Thr Pro Ser Gly
               100                 105                 110
   Asp Val Met Thr Ser Asp Ser Arg Gln Ala Gly Leu Glu Trp Asp Ala
           115                 120                 125
   Lys Ile Cys Asp Asn Thr Asp Ala Asp Ala Thr Gly Val Leu Glu Leu
       130                 135                 140
   Leu Asp Asn Asp Phe Asn Asn Asp Gly Thr Pro Asp Val Ala Ala Leu
   145                 150                 155                 160
   Thr Pro Phe Gln Phe Val Ile Leu Asp Gly Val Ser Gly Arg Val Leu
                   165                 170                 175
   His Glu Val Asp Leu Asp Lys Thr Ile Ala Trp Gln Gly Leu Val Glu
               180                 185                 190
   Ala Ala Gly Ser Ala Thr Gly Gly Lys Arg Lys Arg Pro Ser Ile Met
           195                 200                 205
   Ala Tyr Gly Val Asp Ile Lys Thr Gly Lys Leu Glu Val Arg Lys Leu
       210                 215                 220
   Ala Asn Ser Gly Ala Thr Leu Asp Pro Val Ser Gly Leu Glu Gly Val
   225                 230                 235                 240
   Ser Ala Asp Glu Ile Thr Val Leu Lys Ser Gly Val Ala Lys Val Gly
   245 250 255
   Ser Ala Leu Leu Phe Val Arg Lys Glu Ser Gly Ala Leu Val Ala Phe
   260 265 270
   Asp Cys Val Ala Asn Gln Leu Gln Glu Leu Thr Asn Ala Pro Ser Ile
   275 280 285
   Lys Gly Ser Val Gln Ser Leu Gly Ser Ala Arg Phe Phe Ala Thr Asp
   290 295 300
   Ala Gly Val Ile Tyr Ala Val Asp Gly Glu Leu Lys Ile Ala Glu Thr
   305 310 315 320
   Leu Lys Gly Val Glu Ala Ala Ala Ile Gly Val Ser Gly Ala Ser Val
   325 330 335
   Ile Ala Ala Val Gln Ser Ser Thr Ala Ser Gly Thr Gly Asp Glu Ala
   340 345 350
   Gln Cys Gly Pro Ile Ser Arg Val Leu Val Gln Ser Ala Ser Gly Val
   355 360 365
   Thr Glu Ile Ala Phe Pro Glu Gln Gln Gly Gln Ser Gly Ala Arg Gly
   370 375 380
   Leu Val Glu Lys Ile Ile Val Gly Asp Ser Ser Thr Gly Thr Arg Ala
   385 390 395 400
   Ile Phe Val Phe Glu Asp Ala Ser Ala Val Gly Ile Glu Ile Glu Ser
   405 410 415
   Gly Ala Ser Glu Ala Ser Thr Leu Phe Val Arg Glu Glu Ala Leu Ala
   420 425 430
   Asn Val Val Glu Ala Val Ala Val Asp Leu Pro Pro Thr Asp Glu Val
   435 440 445
   Gly Ser Leu Gly Asp Glu Ala Ala His Val Phe Ala His Gly Ser His
   450 455 460
   Ala Ser Ile Phe Met Phe Arg Leu Lys Asp Gln Val Arg Thr Val Gln
   465 470 475 480
   Arg Phe Val Gln Ser Leu Phe Gly Ala Ala Thr Gln His Leu Ser Glu
   485 490 495
   Phe Val Ala Ser Gln Gly Lys Thr Leu Val Gln Ala Ile Arg Gly Glu
               500                 505                 510
   Leu Pro Arg Ala Glu Ser Leu Ser Gln Ser Glu Met Phe Ser Phe Gly
           515                 520                 525
   Phe Arg Arg Val Leu Val Leu Arg Ser Ala Ser Gly Lys Val Phe Gly
       530                 535                 540
   Leu Asn Ser Ala Asp Gly Ser Leu Leu Trp Ala Ala Gln Ser Pro Gly
   545                 550                 555                 560
   Ser Arg Leu Phe Val Thr Arg Ala Arg Glu Ala Gly Leu Asp His Pro
                   565                 570                 575
   Ala Glu Val Ala Ile Val Asp Glu Ala His Gly Arg Val Thr Trp Arg
               580                 585                 590
   Asn Ala Ile Thr Gly Ala Val Thr Arg Val Glu Asp Ile Asp Thr Pro
           595                 600                 605
   Leu Ala Gln Ile Ala Val Leu Pro Gly Asp Ile Phe Pro Ser Thr Ala
       610                 615                 620
   Ser Ser Glu Glu Asp Val Ser Pro Ala Ala Val Leu Ile Ala Leu Asp
   625                 630                 635                 640
   His Ala Gln Arg Val His Ile Leu Pro Ser Ser Arg Thr Glu Ser Val
                   645                 650                 655
   Leu Gln Leu Glu Asp Leu Leu Arg Ala Leu His Phe Val Val Tyr Ser
               660                 665                 670
   Asn Glu Thr Gly Ala Leu Thr Gly Tyr Ala Val Asp Pro Ser Gln Arg
           675                 680                 685
   Ala Gly Val Glu Leu Trp Ser Met Ile Val Pro Ala Ser Gln Thr Leu
       690                 695                 700
   Leu Ala Val Glu Gly Gln Ser Gly Gly Ala Leu Asn Asn Pro Gly Ile
   705                 710                 715                 720
   Lys Arg Gly Asp Gly Ala Val Leu Val Lys Phe Val Asp Pro His Leu
                   725                 730                 735
   Leu Met Val Ala Thr Gln Ser Gly Pro His Leu Gln Val Ser Ile Leu
               740                 745                 750
   Asn Gly Ile Ser Gly Arg Val Ile Ser Arg Phe Thr His Lys Lys Ser
           755                 760                 765
   Thr Gly Pro Val His Ala Val Leu Ala Asp Asn Thr Val Thr Tyr Ser
       770                 775                 780
   Phe Trp Asn Gln Val Lys Ser Arg Gln Glu Val Ser Val Val Gly Leu
   785                 790                 795                 800
   Phe Glu Gly Glu Ile Gly Pro Arg Glu Leu Asn Met Trp Ser Ser Arg
                   805                 810                 815
   Pro Asn Met Gly Ser Gly Lys Ala Met Ser Ala Phe Asp Asp Ser Met
               820                 825                 830
   Met Pro Asn Val Gln Gln Lys Thr Phe Tyr Thr Glu Arg Ala Ile Ala
           835                 840                 845
   Ala Leu Gly Val Thr Lys Thr Arg Phe Gly Ile Ala Asp Arg Arg Val
       850                 855                 860
   Leu Ile Gly Thr Ala Asn Gly Ala Val Asn Met Gln Val Pro Gln Ile
   865                 870                 875                 880
   Leu Ser Pro Arg Arg Pro Val Gly Lys Leu Ser Asp Met Glu Lys Glu
                   885                 890                 895
   Glu Gly Leu Met Leu Tyr Ala Pro Glu Leu Pro Leu Ile Pro Thr Gln
               900                 905                 910
   Thr Ile Thr Tyr Tyr Glu Ser Ile Pro Gln Leu Arg Leu Ile Arg Ser
           915                 920                 925
   Phe Ala Thr Arg Leu Glu Ser Thr Ser Leu Val Leu Ala Ala Gly Leu
       930                 935                 940
   Asp Ile Phe Tyr Thr Arg Val Met Pro Ser Arg Gly Phe Asp Val Leu
   945                 950                 955                 960
   Asp Glu Asp Phe Ala Ser Gly Leu Leu Leu Ala Leu Ile Ala Ala Leu
                   965                 970                 975
   Leu Ala Leu Thr Ile Tyr Leu Ser Lys Ala Val Gly Lys Ser Thr Leu
               980                 985                 990
   Asp Glu Thr Trp Lys
   995
<210> 87
   <211> 731
   <212> PRT
   <213> Schizochytrium
<220>
   <223> Nicastrin-like
<400> 87
   Met Gly Ala Ala Arg Arg Ser Met Gly Ala Ala Arg Lys Ala Leu Ala
   1               5                   10                  15
   Ala Ser Ala Thr Leu Ala Ala Leu Ala Leu Ala Gly Leu Gln Pro Ala
               20                  25                  30
   Arg Ala Glu Val Asn Gly Val Asn Ala Met Thr Glu Ala Met Leu Thr
           35                  40                  45
   Glu Tyr Ala Ser Leu Pro Cys Val Arg Ser Ile Ala Arg Asp Gly Ala
       50                  55                  60
   Val Gly Cys Gly Ser Pro Ser Asp Arg Ser Val Ala Glu Gly Gly Ala
   65                  70                  75                  80
   Leu Phe Leu Val Glu Ser Val Glu Asp Val Thr Gly Leu Ile Glu Asn
                   85                  90                  95
   Ala Gln Gly Leu Asp Ala Val Ala Leu Val Val Asp Asp Ala Leu Leu
               100                 105                 110
   His Gly Asp Ser Leu Arg Ala Met Gln Asp Leu Ala Lys Lys Ile Arg
           115                 120                 125
   Val Thr Ala Val Ile Val Thr Val Glu Glu Asp Gly Ser Pro Gln Glu
       130                 135                 140
   Pro Pro Arg Ser Ser Ala Ala Pro Thr Thr Trp Ile Pro Ser Gly Asp
   145                 150                 155                 160
   Gly Leu Leu Asn Glu Thr Val Ser Phe Val Val Thr Arg Leu Arg Asn
                   165                 170                 175
   Ala Thr Gln Ser Glu Glu Ile Arg Ala Leu Ala Ala Ser Asn Arg Asp
               180                 185                 190
   Arg Gly Tyr Val Asp Ala Val Phe Gln His Ser Ala Arg Tyr Gln Phe
   195 200 205
   Tyr Leu Gly Lys Glu Thr Ala Thr Ser Leu Ser Cys Leu Ala Ser Gly
   210 215 220
   Arg Cys Asp Pro Leu Gly Gly Leu Ser Val Trp Ala Ser Ala Gly Pro
   225 230 235 240
   Val Pro Val Asn Ser Ala Lys Glu Thr Val Leu Leu Thr Ala Asn Leu
   245 250 255
   Asp Ala Ala Ser Phe Phe His Asp Val Val Pro Ala Arg Asp Thr Thr
   260 265 270
   Ala Ser Gly Val Ala Ala Val Leu Leu Ala Ala Lys Ala Leu Ala Ser
   275 280 285
   Val Asp Glu Ser Val Leu Glu Ala Leu Ser Lys Gln Ile Ala Val Ala
   290 295 300
   Leu Phe Asn Gly Glu Val Trp Ser Arg Ala Gly Ser Arg Arg Phe Val
   305 310 315 320
   His Asp Val Ala Leu Gly Glu Cys Leu Ser Pro Gln Thr Ala Ser Pro
   325 330 335
   Tyr Asn Glu Ser Thr Cys Ala Asn Pro Pro Val Tyr Ala Leu Ala Trp
   340 345 350
   Thr Ser Leu Gly Leu Asp Asn Ile Thr Asp Val Val Ser Val Asn Asn
   355 360 365
   Val Ala Gly Ser Glu Ser Gly Ala Phe Tyr Val His Thr Ala Ala Gly
   370 375 380
   Thr Ala Ser Ala Asn Ala Ala Ala Ala Leu Gln Ser Val Ala Ser Ser
   385 390 395 400
   Ser Thr Asp Val Asp Val Ser Ile Thr Gly Ala Thr Thr Ser Gly Val
   405 410 415
   Val Pro Pro Ser Pro Leu Asp Ser Phe Leu Ala Ala Glu Met Glu Thr
   420 425 430
   Asp Val Ser Phe Ser Gly Ala Gly Leu Val Val Ser Gly Phe Asp Ala
   435 440 445
   Ala Ile Thr Asp Ala Asn Pro Arg Tyr Ser Ser Arg Tyr Asp Arg Arg
       450             455             460
   Asp Lys Gly Pro Glu Ala Asp Asp Ala Glu Ala Leu Thr Ala Ala Arg
   465             470             475             480
   Ile Ala Asp Val Ala Thr Leu Leu Ala Arg His Ala Phe Val Gln Ala
                   485             490             495
   Gly Gly Ser Ile Ser Asp Ala Val Asn Phe Val Leu Val Asp Gly Thr
               500             505             510
   His Ala Ala Glu Leu Trp Asp Cys Leu Thr Lys Asp Phe Ala Cys Thr
           515             520             525
   Leu Val Ala Asp Val Ile Gly Ala Glu Asp Thr Thr Ala Val Ala Asp
       530             535             540
   Phe Met Gly Ser Thr Leu Leu Ala Ala Ser Glu Gly Val Ala Gly Gly
   545             550             555             560
   Ala Pro Asn Phe Phe Ser Gly Ile Tyr Ser Pro Phe Pro Val Glu Asn
                   565             570             575
   Asn Val Met Arg Pro Val Pro Leu Phe Val Arg Asp Tyr Leu Ala Gln
               580             585             590
   Tyr Gly Arg Asn Ala Ser Leu Ile Glu Lys Val Thr Glu Ser Ala Lys
           595             600             605
   Tyr Ala Cys Ala Gln Asp Leu Asp Cys Met Val Met Thr Glu Pro Pro
       610             615             620
   Ala Cys Glu Leu Gly Arg Ser Ala Leu Ala Cys Leu Arg Gly Gly Cys
   625             630             635             640
   Val Cys Ser Asn Ala Tyr Phe His Asp Ala Val Ser Pro Ala Leu Val
                   645             650             655
   Tyr Glu Asp Gly Ala Phe Ser Val Asp Ala Gln Lys Leu Thr Asp Asp
               660             665             670
   Asp Gly Leu Trp Thr Glu Pro Arg Trp Ser Asp Gly Thr Leu Thr Leu
           675             680             685
   Tyr Thr Ser Ala Asn Ser Ala Ser Thr Thr Ile Ala Leu Leu Val Cys
   690 695 700
   Gly Ile Leu Leu Thr Ile Gly Cys Val Phe Ala Leu Arg Lys Ala Gln
   705 710 715 720
   Gly Met Leu Asp Asn Thr Lys Tyr Lys Leu Asn
   725 730
<210> 88
   <211> 232
   <212> **PRT**
   <213> Schizochytrium
<220>
   <223> Emp24
<400> 88
   Met Ala Thr Thr Glu Asn Glu Ala Arg Leu Pro Pro Gly Lys Gln Arg
   1 5 10 15
   Leu Gly Arg Arg Arg Arg Gly Arg Val Ser Lys Ala Ser Gly Trp Gly
   20 25 30
   Thr Thr Leu Ala Leu Ala Ala Ala Val Leu Val Phe Ser Val Asp Arg
   35 40 45
   Ala Ser Gly Val Arg Phe Glu Val Ala Ser Thr Glu Glu Arg Cys Ile
   50 55 60
   Phe Asp Val Leu Arg Lys Asp Gln Leu Val Thr Gly Glu Phe Glu Val
   65 70 75 80
   His Ala Asp Gly Asp Asp Val Asn Met Asp Ile His Val Thr Gly Pro
   85 90 95
   Leu Gly Glu Glu Val Phe Ser Lys Gln Asn Ser Lys Met Ala Lys Phe
   100 105 110
   Gly Phe Thr Ala Glu Ala Ala Gly Glu His Val Leu Cys Leu Arg Asn
   115 120 125
   Asn Asp Met Ile Met Arg Glu Val Gln Val Lys Leu Arg Ser Gly Val
   130 135 140
   Glu Ala Lys Asp Leu Thr Glu Val Val Gln Arg His His Leu Lys Pro
   145 150 155 160
   Leu Ser Ala Glu Val Ile Arg Ile Gln Glu Thr Ile Arg Asp Val Arg
                   165                 170                 175
   His Glu Leu Thr Ala Leu Lys Gln Arg Glu Ala Glu Met Arg Asp Met
               180                 185                 190
   Asn Glu Ser Ile Asn Thr Arg Val Ser Leu Phe Ser Phe Phe Ser Ile
           195                 200                 205
   Ala Val Val Gly Ser Leu Gly Ala Trp Gln Ile Met Tyr Leu Lys Ser
       210                 215                 220
   Tyr Phe Gln Arg Lys Lys Leu Ile
   225                 230
<210> 89
   <211> 550
   <212> PRT
   <213> Schizochytrium
<220>
   <223> Calnexin-like
<400> 89
   Met Arg Thr Thr Phe Val Ala Ala Tyr Ala Ala Val Ala Ala Leu Ala
   1               5                   10                  15
   Leu Gly Gln Cys Glu Ala Ile Asn Phe Arg Glu Ser Phe Glu Gly Ala
               20                  25                  30
   Asn Val Glu Lys Glu Trp Val Lys Ser Ala Ser Asp Arg Tyr Ala Gly
           35                  40                  45
   Ser Glu Trp Ala Phe Asp Thr Ser Lys Asp Thr Gly Asp Val Gly Leu
       50                  55                  60
   Gln Thr Val Lys Pro His Lys Phe Tyr Gly Ile Ser Arg Lys Phe Glu
   65                  70                  75                  80
   Asn Pro Ile Pro Val Gly Asp Gly Glu Lys Pro Phe Val Ala Gln Tyr
                   85                  90                  95
   Glu Val Lys Phe Thr Glu Gly Val Ser Cys Ser Gly Ala Tyr Leu Lys
               100                 105                 110
   Leu Leu Glu Gln Asp Asp Ala Phe Thr Pro Lys Asp Leu Val Glu Ser
           115                 120                 125
   Ser Pro Tyr Ser Ile Met Phe Gly Pro Asp Asn Cys Gly Ala Asn Asn
       130                 135                 140
   Lys Val His Leu Ile Phe Arg Gln Glu Asn Pro Val Thr Lys Glu Tyr
   145                 150                 155                 160
   Glu Glu Lys His Met Thr Lys Lys Val Thr Ser Val Arg Asp Arg Thr
                   165                 170                 175
   Ser His Val Tyr Thr Leu Glu Val His Pro Asp Asn Thr Phe Lys Val
               180                 185                 190
   Lys Val Asp Gly Lys Val Glu Ala Glu Gly Ser Leu Thr Asp Asp Glu
           195                 200                 205
   Ala Phe Ser Pro Pro Phe Gln Gln Pro Lys Glu Ile Asp Asp Pro Asn
       210                 215                 220
   Asp Glu Lys Pro Asp Asp Trp Val Asp Gln Ala Lys Ile Pro Asp Pro
   225                 230                 235                 240
   Glu Ala Ser Lys Pro Asp Asp Trp Asp Glu Asp Ala Pro Lys Arg Ile
                   245                 250                 255
   Ala Asp Pro Asp Ala Val Lys Pro Glu Gly Trp Leu Asp Asp Glu Pro
               260                 265                 270
   Asp Gln Val Pro Asp Pro Ala Ala Ser Glu Pro Glu Asp Trp Asp Glu
           275                 280                 285
   Glu Asp Asp Gly Ile Trp Glu Ala Pro Leu Val Ala Asn Pro Lys Cys
       290                 295                 300
   Thr Ala Gly Pro Gly Cys Gly Glu Trp Asn Ala Pro Met Ile Glu Asn
   305                 310                 315                 320
   Pro Asn Tyr Lys Gly Lys Trp Ser Ala Pro Met Ile Asp Asn Pro Glu
                   325                 330                 335
   Tyr Lys Gly Val Trp Lys Pro Arg Arg Ile Glu Asn Pro Ala Tyr Phe
               340                 345                 350
   Glu Glu Ser Ser Pro Val Thr Thr Ile Lys Pro Ile Gly Ala Val Ala
           355                 360                 365
   Ile Glu Ile Leu Ala Asn Asp Lys Gly Ile Arg Phe Asp Asn Ile Ile
       370                 375                 380
   Ile Gly Asn Asp Val Lys Glu Ala Ala Glu Phe Ile Asp Lys Glu Phe
   385                 390                 395                 400
   Leu Ala Lys Gln Ala Asp Glu Lys Ala Lys Val Lys Glu Glu Ala Ala
                   405                 410                 415
   Gln Ala Ala Gln Asn Ser Arg Trp Glu Glu Tyr Lys Lys Gly Ser Ile
               420                 425                 430
   Gln Gly Tyr Val Met Trp Tyr Ala Gly Asp Tyr Ile Asp Tyr Val Met
           435                 440                 445
   Glu Leu Tyr Glu Ala Ser Pro Ile Ala Val Gly Val Gly Ala Ala Ala
       450                 455                 460
   Ala Gly Leu Ala Val Leu Val Ala Leu Met Val Met Cys Met Ser Gly
   465                 470                 475                 480
   Ala Pro Glu Glu Tyr Asp Asp Asp Val Ala Leu His Lys Lys Asp Asp
                   485                 490                 495
   Asp Ala Ala Ala Gly Asp Asp Asp Glu Ala Glu Ala Glu Ala Glu Asn
               500                 505                 510
   Asp Ala Ala Asp Glu Asp Glu Asp Glu Glu Asp Asp Asp Asp Glu Glu
           515                 520                 525
   Asp Glu Asp Glu Glu Glu Asp Glu Asp Glu Ala Thr Gly Pro Arg Arg
       530                 535                 540
   Arg Val Asn Arg Ala Asn
   545                 550
<210> 90
   <211> 6175
   <212> DNA
   <213> Artificial
<220>
   <223> pCL0121
<400> 90
   ctcttatctg cctcgcgccg ttgaccgccg cttgactctt ggcgcttgcc gctcgcatcc      60
   tgcctcgctc gcgcaggcgg gcgggcgagt gggtgggtcc gcagccttcc gcgctcgccc     120
   gctagctcgc tcgcgccgtg ctgcagccag cagggcagca ccgcacggca ggcaggtccc 180
   ggcgcggatc gatcgatcca tcgatccatc gatccatcga tcgtgcggtc aaaaagaaag 240
   gaagaagaaa ggaaaaagaa aggcgtgcgc acccgagtgc gcgctgagcg cccgctcgcg 300
   gtcccgcgga gcctccgcgt tagtccccgc cccgcgccgc gcagtccccc gggaggcatc 360
   gcgcacctct cgccgccccc tcgcgcctcg ccgattcccc gcctcccctt ttccgcttct 420
   tcgccgcctc cgctcgcggc cgcgtcgccc gcgccccgct ccctatctgc tccccagggg 480
   ggcactccgc accttttgcg cccgctgccg ccgccgcggc cgccccgccg ccctggtttc 540
   ccccgcgagc gcggccgcgt cgccgcgcaa agactcgccg cgtgccgccc cgagcaacgg 600
   gtggcggcgg cgcggcggcg ggcggggcgc ggcggcgcgt aggcggggct aggcgccggc 660
   taggcgaaac gccgcccccg ggcgccgccg ccgcccgctc cagagcagtc gccgcgccag 720
   accgccaacg cagagaccga gaccgaggta cgtcgcgccc gagcacgccg cgacgcgcgg 780
   cagggacgag gagcacgacg ccgcgccgcg ccgcgcgggg gggggagggg agaggcagga 840
   cgcgggagcg agcgtgcatg tttccgcgcg agacgacgcc gcgcgcgctg gagaggagat 900
   aaggcgcttg gatcgcgaga gggccagcca ggctggaggc gaaaatgggt ggagaggata 960
   gtatcttgcg tgcttggacg aggagactga cgaggaggac ggatacgtcg atgatgatgt 1020
   gcacagagaa gaagcagttc gaaagcgact actagcaagc aagggatcca tgaagttcgc 1080
   gacctcggtc gcaattttgc ttgtggccaa catagccacc gccctcgcgc agagcgatgg 1140
   ctgcaccccc accgaccaga cgatggtgag caagggcgag gagctgttca ccggggtggt 1200
   gcccatcctg gtcgagctgg acggcgacgt aaacggccac aagttcagcg tgtccggcga 1260
   gggcgagggc gatgccacct acggcaagct gaccctgaag ttcatctgca ccaccggcaa 1320
   gctgcccgtg ccctggccca ccctcgtgac caccctgacc tacggcgtgc agtgcttcag 1380
   ccgctacccc gaccacatga agcagcacga cttcttcaag tccgccatgc ccgaaggcta 1440
   cgtccaggag cgcaccatct tcttcaagga cgacggcaac tacaagaccc gcgccgaggt 1500
   gaagttcgag ggcgacaccc tggtgaaccg catcgagctg aagggcatcg acttcaagga 1560
   ggacggcaac atcctgggac acaagctgga gtacaactac aacagccaca acgtctatat 1620
   catggccgac aagcagaaga acggcatcaa ggtgaacttc aagatccgcc acaacatcga 1680
   ggacggcagc gtgcagctcg ccgaccacta ccagcagaac acccccatcg gcgacggccc 1740
   cgtgctgctg cccgacaacc actacctgag cacccagtcc gccctgagca aagaccccaa 1800
   cgagaagcgc gatcacatgg tcctgctgga gttcgtgacc gccgccggga tcactctcgg 1860
   catggacgag ctgtacaagc accaccatca ccaccactaa catatgagtt atgagatccg 1920
   aaagtgaacc ttgtcctaac ccgacagcga atggcgggag ggggcgggct aaaagatcgt 1980
   attacatagt atttttcccc tactctttgt gtttgtcttt tttttttttt tgaacgcatt 2040
   caagccactt gtctgggttt acttgtttgt ttgcttgctt gcttgcttgc ttgcctgctt 2100
   cttggtcaga cggcccaaaa aagggaaaaa attcattcat ggcacagata agaaaaagaa 2160
   aaagtttgtc gaccaccgtc atcagaaagc aagagaagag aaacactcgc gctcacattc 2220
   tcgctcgcgt aagaatctta gccacgcata cgaagtaatt tgtccatctg gcgaatcttt 2280
   acatgagcgt tttcaagctg gagcgtgaga tcataccttt cttgatcgta atgttccaac 2340
   cttgcatagg cctcgttgcg atccgctagc aatgcgtcgt actcccgttg caactgcgcc 2400
   atcgcctcat tgtgacgtga gttcagattc ttctcgagac cttcgagcgc tgctaatttc 2460
   gcctgacgct ccttcttttg tgcttccatg acacgccgct tcaccgtgcg ttccacttct 2520
   tcctcagaca tgcccttggc tgcctcgacc tgctcggtaa aacgggcccc agcacgtgct 2580
   acgagatttc gattccaccg ccgccttcta tgaaaggttg ggcttcggaa tcgttttccg 2640
   ggacgccggc tggatgatcc tccagcgcgg ggatctcatg ctggagttct tcgcccaccc 2700
   caacttgttt attgcagctt ataatggtta caaataaagc aatagcatca caaatttcac 2760
   aaataaagca tttttttcac tgcattctag ttgtggtttg tccaaactca tcaatgtatc 2820
   ttatcataca tggtcgacct gcaggaacct gcattaatga atcggccaac gcgcggggag 2880
   aggcggtttg cgtattgggc gctcttccgc ttcctcgctc actgactcgc tgcgctcggt 2940
   cgttcggctg cggcgagcgg tatcagctca ctcaaaggcg gtaatacggt tatccacaga 3000
   atcaggggat aacgcaggaa agaacatgtg agcaaaaggc cagcaaaagg ccaggaaccg 3060
   taaaaaggcc gcgttgctgg cgtttttcca taggctccgc ccccctgacg agcatcacaa 3120
   aaatcgacgc tcaagtcaga ggtggcgaaa cccgacagga ctataaagat accaggcgtt 3180
   tccccctgga agctccctcg tgcgctctcc tgttccgacc ctgccgctta ccggatacct 3240
   gtccgccttt ctcccttcgg gaagcgtggc gctttctcat agctcacgct gtaggtatct 3300
   cagttcggtg taggtcgttc gctccaagct gggctgtgtg cacgaacccc ccgttcagcc 3360
   cgaccgctgc gccttatccg gtaactatcg tcttgagtcc aacccggtaa gacacgactt 3420
   atcgccactg gcagcagcca ctggtaacag gattagcaga gcgaggtatg taggcggtgc 3480
   tacagagttc ttgaagtggt ggcctaacta cggctacact agaagaacag tatttggtat 3540
   ctgcgctctg ctgaagccag ttaccttcgg aaaaagagtt ggtagctctt gatccggcaa 3600
   acaaaccacc gctggtagcg gtggtttttt tgtttgcaag cagcagatta cgcgcagaaa 3660
   aaaaggatct caagaagatc ctttgatctt ttctacgggg tctgacgctc agtggaacga 3720
   aaactcacgt taagggattt tggtcatgag attatcaaaa aggatcttca cctagatcct 3780
   tttaaattaa aaatgaagtt ttaaatcaat ctaaagtata tatgagtaaa cttggtctga 3840
   cagttaccaa tgcttaatca gtgaggcacc tatctcagcg atctgtctat ttcgttcatc 3900
   catagttgcc tgactccccg tcgtgtagat aactacgata cgggagggct taccatctgg 3960
   ccccagtgct gcaatgatac cgcgagaccc acgctcaccg gctccagatt tatcagcaat 4020
   aaaccagcca gccggaaggg ccgagcgcag aagtggtcct gcaactttat ccgcctccat 4080
   ccagtctatt aattgttgcc gggaagctag agtaagtagt tcgccagtta atagtttgcg 4140
   caacgttgtt gccattgcta caggcatcgt ggtgtcacgc tcgtcgtttg gtatggcttc 4200
   attcagctcc ggttcccaac gatcaaggcg agttacatga tcccccatgt tgtgcaaaaa 4260
   agcggttagc tccttcggtc ctccgatcgt tgtcagaagt aagttggccg cagtgttatc 4320
   actcatggtt atggcagcac tgcataattc tcttactgtc atgccatccg taagatgctt 4380
   ttctgtgact ggtgagtact caaccaagtc attctgagaa tagtgtatgc ggcgaccgag 4440
   ttgctcttgc ccggcgtcaa tacgggataa taccgcgcca catagcagaa ctttaaaagt 4500
   gctcatcatt ggaaaacgtt cttcggggcg aaaactctca aggatcttac cgctgttgag 4560
   atccagttcg atgtaaccca ctcgtgcacc caactgatct tcagcatctt ttactttcac 4620
   cagcgtttct gggtgagcaa aaacaggaag gcaaaatgcc gcaaaaaagg gaataagggc 4680
   gacacggaaa tgttgaatac tcatactctt cctttttcaa tattattgaa gcatttatca 4740
   gggttattgt ctcatgagcg gatacatatt tgaatgtatt tagaaaaata aacaaatagg 4800
   ggttccgcgc acatttcccc gaaaagtgcc acctgacgtc taagaaacca ttattatcat 4860
   gacattaacc tataaaaata ggcgtatcac gaggcccttt cgtctcgcgc gtttcggtga 4920
   tgacggtgaa aacctctgac acatgcagct cccggagacg gtcacagctt gtctgtaagc 4980
   ggatgccggg agcagacaag cccgtcaggg cgcgtcagcg ggtgttggcg ggtgtcgggg 5040
   ctggcttaac tatgcggcat cagagcagat tgtactgaga gtgcaccaag cttccaattt 5100
   taggcccccc actgaccgag gtctgtcgat aatccacttt tccattgatt ttccaggttt 5160
   cgttaactca tgccactgag caaaacttcg gtctttccta acaaaagctc tcctcacaaa 5220
   gcatggcgcg gcaacggacg tgtcctcata ctccactgcc acacaaggtc gataaactaa 5280
   gctcctcaca aatagaggag aattccactg acaactgaaa acaatgtatg agagacgatc 5340
   accactggag cggcgcggcg gttgggcgcg gaggtcggca gcaaaaacaa gcgactcgcc 5400
   gagcaaaccc gaatcagcct tcagacggtc gtgcctaaca acacgccgtt ctaccccgcc 5460
   ttcttcgcgc cccttcgcgt ccaagcatcc ttcaagttta tctctctagt tcaacttcaa 5520
   gaagaacaac accaccaaca ccatggccaa gttgaccagt gccgttccgg tgctcaccgc 5580
   gcgcgacgtc gccggagcgg tcgagttctg gaccgaccgg ctcgggttct cccgggactt 5640
   cgtggaggac gacttcgccg gtgtggtccg ggacgacgtg accctgttca tcagcgcggt 5700
   ccaggaccag gtggtgccgg acaacaccct ggcctgggtg tgggtgcgcg gcctggacga 5760
   gctgtacgcc gagtggtcgg aggtcgtgtc cacgaacttc cgggacgcct ccgggccggc 5820
   catgaccgag atcggcgagc agccgtgggg gcgggagttc gccctgcgcg acccggccgg 5880
   caactgcgtg cacttcgtgg ccgaggagca ggactgacac gtgctacgag atttcgattc 5940
   caccgccgcc ttctatgaaa ggttgggctt cggaatcgtt ttccgggacg ccggctggat 6000
   gatcctccag cgcggggatc tcatgctgga gttcttcgcc caccccaact tgtttattgc 6060
   agcttataat ggttacaaat aaagcaatag catcacaaat ttcacaaata aagcattttt 6120
   ttcactgcat tctagttgtg gtttgtccaa actcatcaat gtatcttatc ggtac 6175
<210> 91
   <211> 6611
   <212> DNA
   <213> Artificial
<220>
   <223> pCL0122
<400> 91
   ctcttatctg cctcgcgccg ttgaccgccg cttgactctt ggcgcttgcc gctcgcatcc      60
   tgcctcgctc gcgcaggcgg gcgggcgagt gggtgggtcc gcagccttcc gcgctcgccc     120
   gctagctcgc tcgcgccgtg ctgcagccag cagggcagca ccgcacggca ggcaggtccc     180
   ggcgcggatc gatcgatcca tcgatccatc gatccatcga tcgtgcggtc aaaaagaaag     240
   gaagaagaaa ggaaaaagaa aggcgtgcgc acccgagtgc gcgctgagcg cccgctcgcg     300
   gtcccgcgga gcctccgcgt tagtccccgc cccgcgccgc gcagtccccc gggaggcatc     360
   gcgcacctct cgccgccccc tcgcgcctcg ccgattcccc gcctcccctt ttccgcttct     420
   tcgccgcctc cgctcgcggc cgcgtcgccc gcgccccgct ccctatctgc tccccagggg     480
   ggcactccgc accttttgcg cccgctgccg ccgccgcggc cgccccgccg ccctggtttc     540
   ccccgcgagc gcggccgcgt cgccgcgcaa agactcgccg cgtgccgccc cgagcaacgg     600
   gtggcggcgg cgcggcggcg ggcggggcgc ggcggcgcgt aggcggggct aggcgccggc     660
   taggcgaaac gccgcccccg ggcgccgccg ccgcccgctc cagagcagtc gccgcgccag     720
   accgccaacg cagagaccga gaccgaggta cgtcgcgccc gagcacgccg cgacgcgcgg     780
   cagggacgag gagcacgacg ccgcgccgcg ccgcgcgggg ggggggaggg agaggcagga       840
   cgcgggagcg agcgtgcatg tttccgcgcg agacgacgcc gcgcgcgctg gagaggagat     900
   aaggcgcttg gatcgcgaga gggccagcca ggctggaggc gaaaatgggt ggagaggata     960
   gtatcttgcg tgcttggacg aggagactga cgaggaggac ggatacgtcg atgatgatgt    1020
   gcacagagaa gaagcagttc gaaagcgact actagcaagc aagggatcca tgaagttcgc    1080
   gacctcggtc gcaattttgc ttgtggccaa catagccacc gccctcgcgc agagcgatgg    1140
   ctgcaccccc accgaccaga cgatggtgag caagggcgag gagctgttca ccggggtggt    1200
   gcccatcctg gtcgagctgg acggcgacgt aaacggccac aagttcagcg tgtccggcga 1260
   gggcgagggc gatgccacct acggcaagct gaccctgaag ttcatctgca ccaccggcaa 1320
   gctgcccgtg ccctggccca ccctcgtgac caccctgacc tacggcgtgc agtgcttcag 1380
   ccgctacccc gaccacatga agcagcacga cttcttcaag tccgccatgc ccgaaggcta 1440
   cgtccaggag cgcaccatct tcttcaagga cgacggcaac tacaagaccc gcgccgaggt 1500
   gaagttcgag ggcgacaccc tggtgaaccg catcgagctg aagggcatcg acttcaagga 1560
   ggacggcaac atcctgggac acaagctgga gtacaactac aacagccaca acgtctatat 1620
   catggccgac aagcagaaga acggcatcaa ggtgaacttc aagatccgcc acaacatcga 1680
   ggacggcagc gtgcagctcg ccgaccacta ccagcagaac acccccatcg gcgacggccc 1740
   cgtgctgctg cccgacaacc actacctgag cacccagtcc gccctgagca aagaccccaa 1800
   cgagaagcgc gatcacatgg tcctgctgga gttcgtgacc gccgccggga tcactctcgg 1860
   catggacgag ctgtacaagc accaccatca ccaccactaa catatgagtt atgagatccg 1920
   aaagtgaacc ttgtcctaac ccgacagcga atggcgggag ggggcgggct aaaagatcgt 1980
   attacatagt atttttcccc tactctttgt gtttgtcttt tttttttttt tgaacgcatt 2040
   caagccactt gtctgggttt acttgtttgt ttgcttgctt gcttgcttgc ttgcctgctt 2100
   cttggtcaga cggcccaaaa aagggaaaaa attcattcat ggcacagata agaaaaagaa 2160
   aaagtttgtc gaccaccgtc atcagaaagc aagagaagag aaacactcgc gctcacattc 2220
   tcgctcgcgt aagaatctta gccacgcata cgaagtaatt tgtccatctg gcgaatcttt    2280
   acatgagcgt tttcaagctg gagcgtgaga tcataccttt cttgatcgta atgttccaac    2340
   cttgcatagg cctcgttgcg atccgctagc aatgcgtcgt actcccgttg caactgcgcc 2400
   atcgcctcat tgtgacgtga gttcagattc ttctcgagac cttcgagcgc tgctaatttc 2460
   gcctgacgct ccttcttttg tgcttccatg acacgccgct tcaccgtgcg ttccacttct 2520
   tcctcagaca tgcccttggc tgcctcgacc tgctcggtaa aacgggcccc agcacgtgct 2580
   acgagatttc gattccaccg ccgccttcta tgaaaggttg ggcttcggaa tcgttttccg 2640
   ggacgccggc tggatgatcc tccagcgcgg ggatctcatg ctggagttct tcgcccaccc 2700
   caacttgttt attgcagctt ataatggtta caaataaagc aatagcatca caaatttcac 2760
   aaataaagca tttttttcac tgcattctag ttgtggtttg tccaaactca tcaatgtatc 2820
   ttatcataca tggtcgacct gcaggaacct gcattaatga atcggccaac gcgcggggag 2880
   aggcggtttg cgtattgggc gctcttccgc ttcctcgctc actgactcgc tgcgctcggt 2940
   cgttcggctg cggcgagcgg tatcagctca ctcaaaggcg gtaatacggt tatccacaga 3000
   atcaggggat aacgcaggaa agaacatgtg agcaaaaggc cagcaaaagg ccaggaaccg 3060
   taaaaaggcc gcgttgctgg cgtttttcca taggctccgc ccccctgacg agcatcacaa 3120
   aaatcgacgc tcaagtcaga ggtggcgaaa cccgacagga ctataaagat accaggcgtt 3180
   tccccctgga agctccctcg tgcgctctcc tgttccgacc ctgccgctta ccggatacct 3240
   gtccgccttt ctcccttcgg gaagcgtggc gctttctcat agctcacgct gtaggtatct 3300
   cagttcggtg taggtcgttc gctccaagct gggctgtgtg cacgaacccc ccgttcagcc 3360
   cgaccgctgc gccttatccg gtaactatcg tcttgagtcc aacccggtaa gacacgactt 3420
   atcgccactg gcagcagcca ctggtaacag gattagcaga gcgaggtatg taggcggtgc 3480
   tacagagttc ttgaagtggt ggcctaacta cggctacact agaagaacag tatttggtat 3540
   ctgcgctctg ctgaagccag ttaccttcgg aaaaagagtt ggtagctctt gatccggcaa 3600
   acaaaccacc gctggtagcg gtggtttttt tgtttgcaag cagcagatta cgcgcagaaa 3660
   aaaaggatct caagaagatc ctttgatctt ttctacgggg tctgacgctc agtggaacga 3720
   aaactcacgt taagggattt tggtcatgag attatcaaaa aggatcttca cctagatcct 3780
   tttaaattaa aaatgaagtt ttaaatcaat ctaaagtata tatgagtaaa cttggtctga 3840
   cagttaccaa tgcttaatca gtgaggcacc tatctcagcg atctgtctat ttcgttcatc 3900
   catagttgcc tgactccccg tcgtgtagat aactacgata cgggagggct taccatctgg 3960
   ccccagtgct gcaatgatac cgcgagaccc acgctcaccg gctccagatt tatcagcaat 4020
   aaaccagcca gccggaaggg ccgagcgcag aagtggtcct gcaactttat ccgcctccat 4080
   ccagtctatt aattgttgcc gggaagctag agtaagtagt tcgccagtta atagtttgcg 4140
   caacgttgtt gccattgcta caggcatcgt ggtgtcacgc tcgtcgtttg gtatggcttc 4200
   attcagctcc ggttcccaac gatcaaggcg agttacatga tcccccatgt tgtgcaaaaa 4260
   agcggttagc tccttcggtc ctccgatcgt tgtcagaagt aagttggccg cagtgttatc 4320
   actcatggtt atggcagcac tgcataattc tcttactgtc atgccatccg taagatgctt 4380
   ttctgtgact ggtgagtact caaccaagtc attctgagaa tagtgtatgc ggcgaccgag 4440
   ttgctcttgc ccggcgtcaa tacgggataa taccgcgcca catagcagaa ctttaaaagt 4500
   gctcatcatt ggaaaacgtt cttcggggcg aaaactctca aggatcttac cgctgttgag 4560
   atccagttcg atgtaaccca ctcgtgcacc caactgatct tcagcatctt ttactttcac 4620
   cagcgtttct gggtgagcaa aaacaggaag gcaaaatgcc gcaaaaaagg gaataagggc 4680
   gacacggaaa tgttgaatac tcatactctt cctttttcaa tattattgaa gcatttatca 4740
   gggttattgt ctcatgagcg gatacatatt tgaatgtatt tagaaaaata aacaaatagg 4800
   ggttccgcgc acatttcccc gaaaagtgcc acctgacgtc taagaaacca ttattatcat 4860
   gacattaacc tataaaaata ggcgtatcac gaggcccttt cgtctcgcgc gtttcggtga 4920
   tgacggtgaa aacctctgac acatgcagct cccggagacg gtcacagctt gtctgtaagc 4980
   ggatgccggg agcagacaag cccgtcaggg cgcgtcagcg ggtgttggcg ggtgtcgggg 5040
   ctggcttaac tatgcggcat cagagcagat tgtactgaga gtgcaccaag cttccaattt 5100
   taggcccccc actgaccgag gtctgtcgat aatccacttt tccattgatt ttccaggttt 5160
   cgttaactca tgccactgag caaaacttcg gtctttccta acaaaagctc tcctcacaaa 5220
   gcatggcgcg gcaacggacg tgtcctcata ctccactgcc acacaaggtc gataaactaa 5280
   gctcctcaca aatagaggag aattccactg acaactgaaa acaatgtatg agagacgatc 5340
   accactggag cggcgcggcg gttgggcgcg gaggtcggca gcaaaaacaa gcgactcgcc 5400
   gagcaaaccc gaatcagcct tcagacggtc gtgcctaaca acacgccgtt ctaccccgcc 5460
   ttcttcgcgc cccttcgcgt ccaagcatcc ttcaagttta tctctctagt tcaacttcaa 5520
   gaagaacaac accaccaaca ccatgattga acaagatgga ttgcacgcag gttctccggc 5580
   cgcttgggtg gagaggctat tcggctatga ctgggcacaa cagacaatcg gctgctctga 5640
   tgccgccgtg ttccggctgt cagcgcaggg gcgcccggtt ctttttgtca agaccgacct 5700
   gtccggtgcc ctgaatgaac tgcaggacga ggcagcgcgg ctatcgtggc tggccacgac 5760
   gggcgttcct tgcgcagctg tgctcgacgt tgtcactgaa gcgggaaggg actggctgct 5820
   attgggcgaa gtgccggggc aggatctcct gtcatctcac cttgctcctg ccgagaaagt 5880
   atccatcatg gctgatgcaa tgcggcggct gcatacgctt gatccggcta cctgcccatt 5940
   cgaccaccaa gcgaaacatc gcatcgagcg agcacgtact cggatggaag ccggtcttgt 6000
   cgatcaggat gatctggacg aagagcatca ggggctcgcg ccagccgaac tgttcgccag 6060
   gctcaaggcg cgcatgcccg acggcgatga tctcgtcgtg acccatggcg atgcctgctt 6120
   gccgaatatc atggtggaaa atggccgctt ttctggattc atcgactgtg gccggctggg 6180
   tgtggcggac cgctatcagg acatagcgtt ggctacccgt gatattgctg aagagcttgg 6240
   cggcgaatgg gctgaccgct tcctcgtgct ttacggtatc gccgctcccg attcgcagcg 6300
   catcgccttc tatcgccttc ttgacgagtt cttctgacac gtgctacgag atttcgattc 6360
   caccgccgcc ttctatgaaa ggttgggctt cggaatcgtt ttccgggacg ccggctggat 6420
   gatcctccag cgcggggatc tcatgctgga gttcttcgcc caccccaact tgtttattgc 6480
   agcttataat ggttacaaat aaagcaatag catcacaaat ttcacaaata aagcattttt 6540
   ttcactgcat tctagttgtg gtttgtccaa actcatcaat gtatcttatc atgtctgaat 6600
   tcccggggta c 6611
<210> 92
   <211> 1314
   <212> **DNA**
   <213> Artificial
<220>
   <223> Codon optimized Isomerase
<400> 92
   atggctaagg agtacttccc ccagatccag aagattaagt tcgagggtaa ggacagcaag      60
   aacccgctcg cctttcatta ctacgacgcc gagaaggagg tgatgggcaa gaagatgaag     120
   gactggcttc gctttgctat ggcttggtgg cacactctct gcgctgaggg cgcggaccag     180
   tttggcggcg gtacgaagag ctttccgtgg aacgagggca ctgacgctat tgagattgct     240
   aagcagaagg ttgacgctgg tttcgagatt atgcagaagc tcggtattcc gtactactgc     300
   tttcacgatg tcgacctcgt ttccgagggc aactcgatcg aggagtacga gtcgaacctc     360
   aaggctgtgg ttgcctacct caaggagaag cagaaggaga ccggaatcaa gctcctctgg     420
   agcaccgcca acgttttcgg ccacaagcgc tacatgaacg gcgcctccac caaccctgac     480
   ttcgatgttg ttgcccgcgc tattgtccag attaagaacg ccatcgacgc tggtatcgag     540
   ctcggagccg agaactacgt tttttggggc ggacgcgagg gttacatgtc cctcctcaac     600
   accgaccaga agcgtgagaa ggagcacatg gccactatgc ttaccatggc ccgcgactac     660
   gcccgcagca agggttttaa gggtactttt ctcattgagc cgaagcccat ggagccgacc     720
   aagcaccagt acgacgtcga caccgagacc gccattggct tccttaaggc ccacaacctt     780
   gacaaggatt ttaaggtgaa catcgaggtt aaccacgcta cgcttgccgg ccacaccttt     840
   gagcatgagc tcgcctgcgc tgttgacgcc ggaatgcttg gttccattga cgccaaccgc     900
   ggcgactacc agaacggctg ggacaccgac cagtttccga ttgaccagta cgagctcgtc     960
   caggcctgga tggagatcat ccgtggtgga ggctttgtta ccggtggtac gaacttcgac    1020
   gccaagacgc gccgtaacag cacggacctc gaggacatca tcattgctca tgtgtcgggc    1080
   atggacgcca tggctcgcgc ccttgagaac gctgctaagc tcctccagga gagcccctac    1140
   acgaagatga agaaggagcg ctacgcgtcg tttgacagcg gaatcggtaa ggacttcgag    1200
   gatggcaagc tcaccctgga gcaggtgtac gagtacggta agaagaacgg cgagccgaag    1260
   cagaccagcg gcaagcagga gctctacgag gccattgtcg ccatgtacca gtag          1314
<210> 93
   <211> 1485
   <212> **DNA**
   <213> Artificial
<220>
   <223> Codon optimized Kinase
<400> 93
   atgaagaccg tcgccggcat cgatcttgga acccagtcca tgaaggttgt catttacgac      60
   tacgagaaga aggagatcat cgagtccgcc tcgtgcccta tggagctcat tagcgagtcg     120
   gacggaaccc gcgagcagac gactgagtgg tttgacaagg gtctcgaggt gtgctttgga     180
   aagctctccg ctgataacaa gaagaccatt gaggcgattg gcatctccgg ccagctccac     240
   ggcttcgtcc ctctcgatgc gaacggaaag gcgctctaca acatcaagct ctggtgcgac     300
   accgccactg tggaggagtg caagatcatt actgacgccg ccggcggcga caaggctgtc     360
   atcgacgcgc tcggcaacct catgctcacc ggattcaccg ccccgaagat tctctggctc     420
   aagcgcaaca agcccgaggc ctttgctaac ctcaagtaca ttatgctgcc ccacgattac     480
   ctcaactgga agctgactgg agactacgtc atggagtacg gcgacgcctc cggcaccgcc     540
   ctttttgatt cgaagaaccg ctgctggtcg aagaagattt gcgacattat tgatcctaag     600
   ctgctcgacc ttctccctaa gctcattgag ccctcggccc ccgccggtaa ggtcaacgac     660
   gaggccgcca aggcgtacgg cattcccgcc ggaatccccg tttccgctgg cggcggtgat     720
   aacatgatgg gtgcggtcgg tactggcacc gtcgctgacg gattcctcac gatgagcatg     780
   ggcacctccg gaactcttta cggctactcg gacaagccta tttccgaccc ggctaacggc     840
   ctcagcggct tctgcagctc cacgggcggc tggcttcccc tcctttgcac catgaactgc     900
   accgtcgcca ccgagttcgt ccgcaacctt tttcagatgg atatcaagga gctgaacgtc     960
   gaggctgcta agtccccctg cggcagcgag ggcgttcttg tcattccttt cttcaacggc    1020
   gagcgcaccc cgaacctccc caacggccgc gcctcgatta ccggcctcac ctccgcgaac    1080
   acgtcccgcg ccaacatcgc tcgcgcctcc tttgagtcgg ccgtctttgc catgcgcggt    1140
   ggcctcgatg cgtttcgtaa gctcggattc cagcccaagg agattcgcct catcggcggt    1200
   ggttcgaagt ccgacctctg gcgccagatc gctgctgaca ttatgaacct tcccatccgt    1260
   gtcccccttc tcgaggaggc cgccgccctc ggcggagctg tccaggccct ttggtgcctt    1320
   aagaaccagt ccggtaagtg cgacatcgtc gagctttgca aggagcatat caagattgac    1380
   gagtccaaga acgccaaccc gattgccgag aacgtcgccg tgtacgataa ggcctacgat    1440
   gagtactgca aggtcgttaa cacgctcagc cctctgtacg cctaa                    1485
<210> 94
   <211> 1569
   <212> DNA
   <213> Artificial
<220>
   <223> Codon optimized transporter
<400> 94
<210> 95
   <211> 1512
   <212> DNA
   <213> Artificial
<220>
   <223> Codon optimized transporter
<400> 95
   atggccctcg accctgagca gcagcagccc atttcctccg tgtcgcgcga gtttggtaag      60
   tcgtccggtg agatctcccc cgagcgtgag cctctcatta aggagaacca cgtccccgag     120
   aactactccg ttgttgccgc catcctcccc ttcctcttcc cggccctggg tggcctcctt     180
   tacggttacg agattggcgc tacgtcgtgc gctacgattt cccttcagtc cccctccctc     240
   tccggcatct cctggtacaa cctctcctcc gtcgatgttg gcctcgtcac ttccggttcc     300
   ctctacggtg ctctgtttgg ctccattgtt gccttcacca ttgccgacgt tattggccgt     360
   cgcaaggagc ttatcctcgc tgctctcctc tacctcgtcg gtgccctcgt taccgctctc     420
   gcccctacgt actccgttct catcatcggc cgtgtcattt acggtgtttc cgtcggtctt     480
   gccatgcatg ctgcccctat gtacatcgcg gagaccgccc cgtcccccat ccgcggccag     540
   ctcgtttccc tcaaggagtt tttcatcgtt ctcggtatgg tcggcggata cggcattggt     600
   tccctcaccg tcaacgtcca ctccggttgg cgctacatgt acgctacctc cgttcccctc     660
   gctgtgatca tgggcattgg catgtggtgg cttcctgcct ccccccgttg gctcctcctc     720
   cgcgtcattc agggtaaggg taacgttgag aaccagcgcg aggctgccat taagtccctc     780
   tgctgcctcc gtggtcctgc cttcgtcgac tcggccgccg agcaggtcaa cgagattctc     840
   gccgagctta ccttcgttgg cgaggataag gaggtcacct tcggcgagct cttccaggga     900
   aagtgcctca aggccctcat tatcggcggc ggccttgttc tctttcagca gatcaccggt     960
   cagccttcgg tcctctacta cgccccctcg atcctccaga ctgcgggctt ctccgccgcc    1020
   ggcgatgcta cccgcgtttc cattcttctc ggcctcctca agctcattat gaccggtgtc    1080
   gccgtcgtcg ttatcgatcg tctcggccgt cgccctctcc tcctcggcgg agtcggtggt    1140
   atggttgttt cgctctttct ccttggctcg tactaccttt tcttcagcgc ttcccccgtc    1200
   gtcgccgttg tcgccctcct tctctacgtg ggttgctacc agctctcctt tggccccatt    1260
   ggctggctta tgatttccga gatttttccc ctcaagctcc gtggtcgcgg actctccctt    1320
   gccgtgcttg tcaactttgg tgccaacgcc ctcgtcacct ttgccttttc ccctctcaag    1380
   gagctcctcg gcgccggcat cctgttttgc ggctttggcg ttatctgcgt tctctccctt    1440
   gtttttatct tttttatcgt cccggagact aagggcctca cgctcgagga gatcgaggcg    1500
   aagtgcctct aa                                                        1512
<210> 96
   <211> 19
   <212> DNA
   <213> Artificial
<220>
   <223> Primer 5' CL0130
<400> 96
   cctcgggcgg cgtcctctt 19
<210> 97
   <211> 20
   <212> DNA
   <213> Artificial
<220>
   <223> Primer 3' CL0130
<400> 97
   ggcggccttc tcctggttgc 20
<210> 98
   <211> 24
   <212> DNA
   <213> Artificial
<220>
   <223> Primer 5' CL0131
<400> 98
   ctactccgtt gttgccgcca tcct 24
<210> 99
   <211> 22
   <212> DNA
   <213> Artificial
<220>
   <223> Primer 3' CL0131
<400> 99
   ccgccgacca taccgagaac ga 22
<210> 100
   <211> 1362
   <212> DNA
   <213> Artificial
<220>
   <223> Codon Optimized NA
<400> 100
   atgaacccca accagaagat tactactatc ggtagcattt gcctcgtcgt tggacttatc        60
   tcccttattc ttcagattgg taacattatc tccatttgga tctcgcatag cattcagacc       120
   ggctcccaga accacaccgg catttgcaac cagaacatta ttacttacaa gaactccact       180
   tgggtcaagg acactactag cgttattctt accggtaact cgtcgctttg ccctattcgc       240
   ggctgggcta tttacagcaa ggacaactcg atccgcatcg gtagcaaggg cgacgttttt       300
   gtcatccgtg agccttttat ttcctgcagc cacctcgagt gccgtacttt ttttctgact       360
   cagggcgctc tcctcaacga taagcattcc aacggcactg tcaaggatcg cagcccctac       420
   cgcgccctta tgtcctgccc tgtcggcgag gctcccagcc cctacaactc ccgttttgag       480
   tccgttgcct ggtccgccag cgcctgccac gacggaatgg gatggctcac tattggtatt       540
   tccggccctg ataacggcgc tgtcgccgtc cttaagtaca acggcattat caccgagacc       600
   atcaagtcct ggcgtaagaa gatcctccgc acccaggagt ccgagtgcgc ctgcgtcaac       660
   ggcagctgct tcacgattat gaccgacggc cctccgacgg gcctcgcttc ctacaagatt       720
   tttaagattg agaagggtaa ggtcacgaag tccatcgagc ttaacgcccc gaactcccac       780
   tacgaggagt gctcctgcta ccctgacact ggcaaggtga tgtgcgtctg ccgcgataac       840
   tggcatggct ccaaccgccc ctgggttagc ttcgatcaga accttgacta ccagattgga       900
   tacatttgct ccggtgtttt tggcgacaac ccgcgccccg aggatggaac tggttcgtgc       960
   ggtcctgttt acgttgacgg cgccaacggc gttaagggtt tttcctaccg ttacggtaac      1020
   ggagtctgga tcggccgcac caagtcgcac agctcgcgcc acggatttga gatgatctgg      1080
   gaccccaacg gatggactga gaccgattcc aagtttagcg ttcgccagga tgtcgttgct      1140
   atgaccgatt ggtcgggata ctccggttcc tttgtgcagc accctgagct caccggcctt      1200
   gactgcatgc gcccttgctt ttgggtcgag ctcattcgcg gtcgccctaa ggagaagact      1260
   atttggacct ccgccagcag catttccttt tgcggcgtta actccgacac cgtcgactgg      1320
   tcgtggcccg atggcgccga gcttcccttt tccattgata ag                         1362
<210> 101
   <211> 1431
   <212> DNA
   <213> Artificial
<220>
   <223> Codon Optimized NA with V5 tag and a polyhistidine tag
<400> 101
   atgaacccca accagaagat tactactatc ggtagcattt gcctcgtcgt tggacttatc        60
   tcccttattc ttcagattgg taacattatc tccatttgga tctcgcatag cattcagacc       120
   ggctcccaga accacaccgg catttgcaac cagaacatta ttacttacaa gaactccact       180
   tgggtcaagg acactactag cgttattctt accggtaact cgtcgctttg ccctattcgc       240
   ggctgggcta tttacagcaa ggacaactcg atccgcatcg gtagcaaggg cgacgttttt       300
   gtcatccgtg agccttttat ttcctgcagc cacctcgagt gccgtacttt ttttctgact       360
   cagggcgctc tcctcaacga taagcattcc aacggcactg tcaaggatcg cagcccctac       420
   cgcgccctta tgtcctgccc tgtcggcgag gctcccagcc cctacaactc ccgttttgag       480
   tccgttgcct ggtccgccag cgcctgccac gacggaatgg gatggctcac tattggtatt       540
   tccggccctg ataacggcgc tgtcgccgtc cttaagtaca acggcattat caccgagacc       600
   atcaagtcct ggcgtaagaa gatcctccgc acccaggagt ccgagtgcgc ctgcgtcaac       660
   ggcagctgct tcacgattat gaccgacggc ccctccgacg gcctcgcttc ctacaagatt       720
   tttaagattg agaagggtaa ggtcacgaag tccatcgagc ttaacgcccc gaactcccac       780
   tacgaggagt gctcctgcta ccctgacact ggcaaggtga tgtgcgtctg ccgcgataac       840
   tggcatggct ccaaccgccc ctgggttagc ttcgatcaga accttgacta ccagattgga       900
   tacatttgct ccggtgtttt tggcgacaac ccgcgccccg aggatggaac tggttcgtgc       960
   ggtcctgttt acgttgacgg cgccaacggc gttaagggtt tttcctaccg ttacggtaac      1020
   ggagtctgga tcggccgcac caagtcgcac agctcgcgcc acggatttga gatgatctgg      1080
   gaccccaacg gatggactga gaccgattcc aagtttagcg ttcgccagga tgtcgttgct      1140
   atgaccgatt ggtcgggata ctccggttcc tttgtgcagc accctgagct caccggcctt      1200
   gactgcatgc gcccttgctt ttgggtcgag ctcattcgcg gtcgccctaa ggagaagact      1260
   atttggacct ccgccagcag catttccttt tgcggcgtta actccgacac cgtcgactgg      1320
   tcgtggcccg atggcgccga gcttcccttt tccattgata agggtaagcc tatccctaac      1380
   cctctcctcg gtctcgattc tacgcgtacc ggtcatcatc accatcacca t               1431
<210> 102
   <211> 539
   <212> PRT
   <213> Human parainfluenza 3 virus
<400> 102
   Met Pro Thr Ser Ile Leu Leu Ile Ile Thr Thr Met Ile Met Ala Ser
   1               5                   10                  15
   Phe Cys Gln Ile Asp Ile Thr Lys Leu Gln His Val Gly Val Leu Val
               20                  25                  30
   Asn Ser Pro Lys Gly Met Lys Ile Ser Gln Asn Phe Glu Thr Arg Tyr
           35                  40                  45
   Leu Ile Leu Ser Leu Ile Pro Lys Ile Glu Asp Ser Asn Ser Cys Gly
       50                  55                  60
   Asp Gln Gln Ile Lys Gln Tyr Lys Arg Leu Leu Asp Arg Leu Ile Ile
   65                  70                  75                  80
   Pro Leu Tyr Asp Gly Leu Arg Leu Gln Lys Asp Val Ile Val Ser Asn
                   85                  90                  95
   Gln Glu Ser Asn Glu Asn Thr Asp Pro Arg Thr Lys Arg Phe Phe Gly
               100                 105                 110
   Gly Val Ile Gly Thr Ile Ala Leu Gly Val Ala Thr Ser Ala Gln Ile
   115 120 125
   Thr Ala Ala Val Ala Leu Val Glu Ala Lys Gln Ala Arg Ser Asp Ile
   130 135 140
   Glu Lys Leu Lys Glu Ala Ile Arg Asp Thr Asn Lys Ala Val Gln Ser
   145 150 155 160
   Val Gln Ser Ser Ile Gly Asn Leu Ile Val Ala Ile Lys Ser Val Gln
   165 170 175
   Asp Tyr Val Asn Lys Glu Ile Val Pro Ser Ile Ala Arg Leu Gly Cys
   180 185 190
   Glu Ala Ala Gly Leu Gln Leu Gly Ile Ala Leu Thr Gln His Tyr Ser
   195 200 205
   Glu Leu Thr Asn Ile Phe Gly Asp Asn Ile Gly Ser Leu Gln Glu Lys
   210 215 220
   Gly Ile Lys Leu Gln Gly Ile Ala Ser Leu Tyr Arg Thr Asn Ile Thr
   225 230 235 240
   Glu Ile Phe Thr Thr Ser Thr Val Asp Lys Tyr Asp Ile Tyr Asp Leu
   245 250 255
   Leu Phe Thr Glu Ser Ile Lys Val Arg Val Ile Asp Val Asp Leu Asn
   260 265 270
   Asp Tyr Ser Ile Thr Leu Gln Val Arg Leu Pro Leu Leu Thr Arg Leu
   275 280 285
   Leu Asn Thr Gln Ile Tyr Arg Val Asp Ser Ile Ser Tyr Asn Ile Gln
   290 295 300
   Asn Arg Glu Trp Tyr Ile Pro Leu Pro Ser His Ile Met Thr Lys Gly
   305 310 315 320
   Ala Phe Leu Gly Gly Ala Asp Val Lys Glu Cys Ile Glu Ala Phe Ser
   325 330 335
   Ser Tyr Ile Cys Pro Ser Asp Pro Gly Phe Val Leu Asn His Glu Met
   340 345 350
   Glu Ser Cys Leu Ser Gly Asn Ile Ser Gln Cys Pro Arg Thr Val Val
               355                 360                 365
   Lys Ser Asp Ile Val Pro Arg Tyr Ala Phe Val Asn Gly Gly Val Val
       370                 375                 380
   Ala Asn Cys Ile Thr Thr Thr Cys Thr Cys Asn Gly Ile Gly Asn Arg
   385                 390                 395                 400
   Ile Asn Gln Pro Pro Asp Gln Gly Val Lys Ile Ile Thr His Lys Glu
                   405                 410                 415
   Cys Asn Thr Ile Gly Ile Asn Gly Met Leu Phe Asn Thr Asn Lys Glu
               420                 425                 430
   Gly Thr Leu Ala Phe Tyr Thr Pro Asn Asp Ile Thr Leu Asn Asn Ser
           435                 440                 445
   Val Ala Leu Asp Pro Ile Asp Ile Ser Ile Glu Leu Asn Lys Ala Lys
       450                 455                 460
   Ser Asp Leu Glu Glu Ser Lys Glu Trp Ile Arg Arg Ser Asn Gln Lys
   465                 470                 475                 480
   Leu Asp Ser Ile Gly Asn Trp His Gln Ser Ser Thr Thr Ile Ile Ile
                   485                 490                 495
   Val Leu Ile Met Ile Ile Ile Leu Phe Ile Ile Asn Val Thr Ile Ile
               500                 505                 510
   Ile Ile Ala Val Lys Tyr Tyr Arg Ile Gln Lys Arg Asn Arg Val Asp
           515                 520                 525
   Gln Asn Asp Lys Pro Tyr Val Leu Thr Asn Lys
       530                 535
<210> 103
   <211> 511
   <212> PRT
   <213> Vesicular stomatitis Indiana virus
<400> 103
   Met Lys Cys Leu Leu Tyr Leu Ala Phe Leu Phe Ile Gly Val Asn Cys
   1               5                   10                  15
   Lys Phe Thr Ile Val Phe Pro His Asn Gln Lys Gly Asn Trp Lys Asn
               20                  25                  30
   Val Pro Ser Asn Tyr His Tyr Cys Pro Ser Ser Ser Asp Leu Asn Trp
           35                  40                  45
   His Asn Asp Leu Val Gly Thr Ala Leu Gln Val Lys Met Pro Lys Ser
       50                  55                  60
   His Lys Ala Ile Gln Ala Asp Gly Trp Met Cys His Ala Ser Lys Trp
   65                  70                  75                  80
   Val Thr Thr Cys Asp Phe Arg Trp Tyr Gly Pro Lys Tyr Ile Thr His
                   85                  90                  95
   Ser Ile Arg Ser Phe Thr Pro Ser Val Glu Gln Cys Lys Glu Ser Ile
               100                 105                 110
   Glu Gln Thr Lys Gln Gly Thr Trp Leu Asn Pro Gly Phe Pro Pro Gln
           115                 120                 125
   Ser Cys Gly Tyr Ala Thr Val Thr Asp Ala Glu Ala Ala Ile Val Gln
       130                 135                 140
   Val Thr Pro His His Val Leu Val Asp Glu Tyr Thr Gly Glu Trp Val
   145                 150                 155                 160
   Asp Ser Gln Phe Ile Asn Gly Lys Cys Ser Asn Asp Ile Cys Pro Thr
                   165                 170                 175
   Val His Asn Ser Thr Thr Trp His Ser Asp Tyr Lys Val Lys Gly Leu
               180                 185                 190
   Cys Asp Ser Asn Leu Ile Ser Met Asp Ile Thr Phe Phe Ser Glu Asp
           195                 200                 205
   Gly Glu Leu Ser Ser Leu Gly Lys Lys Gly Thr Gly Phe Arg Ser Asn
       210                 215                 220
   Tyr Phe Ala Tyr Glu Thr Gly Asp Lys Ala Cys Lys Met Gln Tyr Cys
   225                 230                 235                 240
   Lys His Trp Gly Val Arg Leu Pro Ser Gly Val Trp Phe Glu Met Ala
                   245                 250                 255
   Asp Lys Asp Leu Phe Ala Ala Ala Arg Phe Pro Glu Cys Pro Glu Gly
               260                 265                 270
   Ser Ser Ile Ser Ala Pro Ser Gln Thr Ser Val Asp Val Ser Leu Ile
           275                 280                 285
   Gln Asp Val Glu Arg Ile Leu Asp Tyr Ser Leu Cys Gln Glu Thr Trp
       290                 295                 300
   Ser Lys Ile Arg Ala Gly Leu Pro Ile Ser Pro Val Asp Leu Ser Tyr
   305                 310                 315                 320
   Leu Ala Pro Lys Asn Pro Gly Thr Gly Pro Val Phe Thr Ile Ile Asn
                   325                 330                 335
   Gly Thr Leu Lys Tyr Phe Glu Thr Arg Tyr Ile Arg Val Asp Ile Ala
               340                 345                 350
   Ala Pro Ile Leu Ser Arg Met Val Gly Met Ile Ser Gly Thr Thr Thr
           355                 360                 365
   Glu Arg Val Leu Trp Asp Asp Trp Ala Pro Tyr Glu Asp Val Gly Ile
       370                 375                 380
   Gly Pro Asn Gly Val Leu Arg Thr Ser Ser Gly Tyr Lys Phe Pro Leu
   385                 390                 395                 400
   Tyr Met Ile Gly His Gly Met Leu Asp Ser Asp Leu His Leu Ser Ser
                   405                 410                 415
   Lys Ala Gln Val Phe Glu His Pro His Ile Gln Asp Ala Ala Ser Gln
               420                 425                 430
   Leu Pro Asp Gly Glu Thr Leu Phe Phe Gly Asp Thr Gly Leu Ser Lys
           435                 440                 445
   Asn Pro Ile Glu Phe Val Glu Gly Trp Phe Ser Ser Trp Lys Ser Ser
       450                 455                 460
   Ile Ala Ser Phe Phe Phe Thr Ile Gly Leu Ile Ile Gly Leu Phe Leu
   465                 470                 475                 480
   Val Leu Arg Val Gly Ile Tyr Leu Cys Ile Lys Leu Lys His Thr Lys
                   485                 490                 495
   Lys Arg Gln Ile Tyr Thr Asp Ile Glu Met Asn Arg Leu Gly Thr
               500                 505                 510

## Description

### BACKGROUND OF THE INVENTION

### Field of the Present Disclosure

The present disclosure relates to recombinant microalgal cells and their use in heterologous polypeptide production, methods of production of heterologous polypeptides in microalgal extracellular bodies, microalgal extracellular bodies comprising heterologous polypeptides, and compositions comprising the same.

### Background Art

Advancements in biotechnology and molecular biology have enabled the production of proteins in microbial, plant, and animal cells, many of which were previously available only by extraction from tissues, blood, or urine of humans and other animals. Biologics that are commercially available today are typically manufactured either in mammalian cells, such as Chinese Hamster Ovary (CHO) cells, or in microbial cells, such as yeast or *E. coli* cell lines.

Production of proteins via the fermentation of microorganisms presents several advantages over existing systems such as plant and animal cell culture. For example, microbial fermentation-based processes can offer: (i) rapid production of high concentration of protein; (ii) the ability to use sterile, well-controlled production conditions (such as Good Manufacturing Practice (GMP) conditions); (iii) the ability to use simple, chemically defined growth media allowing for simpler fermentations and fewer impurities; (iv) the absence of contaminating human or animal pathogens; and (v) the ease of recovering the protein (e.g., via isolation from the fermentation media). In addition, fermentation facilities are typically less costly to construct than cell culture facilities.

Microalgae, such as thraustochytrids of the phylum Labyrinthulomycota, can be grown with standard fermentation equipment, with very short culture cycles (e.g., 1-5 days), inexpensive defined media and minimal purification, if any. Furthermore, certain microalgae, e.g., *Schizochytrium,* have a demonstrated history of safety for food applications of both the biomass and lipids derived therefrom. For example, DHA-enriched triglyceride oil from this microorganism has received GRAS (Generally Recognized as Safe) status from the U.S. Food and Drug Administration.

Microalgae have been shown to be capable of expressing recombinant proteins. For example, U.S. Patent No. 7,001,772 disclosed the first recombinant constructs suitable for transforming thraustochytrids, including members of the genus *Schizochytrium.* This publication disclosed, among other things, *Schizochytrium* nucleic acid and amino acid sequences for an acetolactate synthase, an acetolactate synthase promoter and terminator region, an α-tubulin promoter, a promoter from a polyketide synthase (PKS) system, and a fatty acid desaturase promoter. U.S. Publ. Nos. 2006/0275904 and 2006/0286650 subsequently disclosed *Schizochytrium* sequences for actin, elongation factor 1 alpha (ef1α), and glyceraldehyde 3-phosphate dehydrogenase (gapdh) promoters and terminators. EP 2 090 648 A1 relates to the production of glycosylated polypeptides in microalgae.

A continuing need exists for the identification of methods for expressing heterologous polypeptides in microalgae as well as alternative compositions for therapeutic applications.

### BRIEF SUMMARY OF THE INVENTION

The present invention is defined in the attached claims.

In some embodiments, the methods of the invention produce a viral protein selected from the group consisting of a hemagglutinin (HA) protein, a neuraminidase (NA) protein, a fusion (F) protein, a glycoprotein (G) protein, an envelope (E) protein, a glycoprotein of 120 kDa (gp120), a glycoprotein of 41 kDa (gp41), a matrix protein, and combinations thereof, and the methods comprise culturing a recombinant microalgal cell in a medium, wherein the recombinant microalgal cell comprises a nucleic acid molecule comprising a polynucleotide sequence that encodes the viral protein, to produce the viral protein.
In some embodiments, the viral protein is a HA protein. In some embodiments, the HA protein is at least 90% identical to SEQ ID NO: 77. In some embodiments, the microalgal cell is capable of post-translational processing of the HA protein to produce HA1 and HA2 polypeptides in the absence of exogenous protease. In some embodiments, the viral protein is a NA protein. In some embodiments, the NA protein is at least 90% identical to SEQ ID NO: 100. In some embodiments, the viral protein is a F protein. In some embodiments, the F protein is at least 90% identical to SEQ ID NO: 102. In some embodiments, the viral protein is a G protein. In some embodiments, the G protein is at least 90% identical to SEQ ID NO: 103. In some embodiments, the microalgal cell is a member of the order Thraustochytriales. In some embodiments, the microalgal cell is a *Schizochytrium* or a *Thraustochytrium.* In some embodiments, the polynucleotide sequence encoding the viral protein further comprises a HA membrane domain. In some embodiments, the nucleic acid molecule further comprises a polynucleotide sequence selected from the group consisting of: SEQ ID NO: 2, SEQ ID NO: 3, SEQ ID NO: 4, SEQ ID NO: 38, SEQ ID NO: 42, SEQ ID NO: 43, SEQ ID NO: 44, SEQ ID NO: 45, SEQ ID NO: 46, and combinations thereof.

The methods of the present invention can be used to produce an isolated viral membrane protein.

Also disclosed but not forming part of the claimed invention is a recombinant microalgal cell comprising a nucleic acid molecule comprising a polynucleotide sequence that encodes a viral protein selected from the group consisting of a hemagglutinin (HA) protein, a neuraminidase (NA) protein, a fusion (F) protein, a glycoprotein (G) protein, an envelope (E) protein, a glycoprotein of 120 kDa (gp120), a glycoprotein of 41 kDa (gp41), a matrix protein, and combinations thereof. In some instances of the disclosure, the viral protein is a HA protein. In some instances, the HA protein is at least 90% identical to SEQ ID NO: 77. In some instances, the microalgal cell is capable of post-translational processing of the HA protein to produce HA1 and HA2 polypeptides in the absence of exogenous protease. In some instances, the viral protein is a NA protein. In some instances, the NA protein is at least 90% identical to SEQ ID NO: 100. In some instances, the viral protein is a F protein. In some instances, the F protein is at least 90% identical to SEQ ID NO: 102. In some instances, the viral protein is a G protein. In some instances, the G protein is at least 90% identical to SEQ ID NO: 103. In some instances, the microalgal cell is a member of the order Thraustochytriales. In some instances, the microalgal cell is a *Schizochytrium* or a *Thraustochytrium.* In some instances, the polynucleotide sequence encoding the viral protein further comprises a HA membrane domain. In some instances, the nucleic acid molecule further comprises a polynucleotide sequence selected from the group consisting of: SEQ ID NO: 2, SEQ ID NO: 3, SEQ ID NO: 4, SEQ ID NO: 38, SEQ ID NO: 42, SEQ ID NO: 43, SEQ ID NO: 44, SEQ ID NO: 45, SEQ ID NO: 46, and combinations thereof.

The methods of the present invention can be used to produce a composition comprising a microalgal extracellular body and a heterologous membrane protein comprising a transmembrane domain.
In some embodiments, the method further comprises removing the culture supernatant from the composition and resuspending the extracellular body in an aqueous liquid carrier. The present invention is directed to a composition produced by the method.

In some embodiments, the method
comprises a host cell that is a *Labyrinthulomycota* host cell. In some embodiments, the host cell is a *Schizochytrium* or *Thraustochytrium* host cell.

In some embodiments, the present invention is directed to a microalgal extracellular body comprising a heterologous membrane protein comprising a transmembrane domain, wherein the extracellular body is discontinuous with a plasma membrane of a microalgal cell. In some embodiments, the extracellular body is a vesicle, a micelle, a membrane fragment, a membrane aggregate, or a mixture thereof. In some embodiments, the extracellular body is a mixture of a vesicle and a membrane fragment. In some embodiments, the extracellular body is a vesicle. In some embodiments, the heterologous polypeptide comprises a membrane domain. In some embodiments, the heterologous polypeptide is a glycoprotein. In some embodiments, the glycoprotein comprises high-mannose oligosaccharides. In some embodiments, the glycoprotein is substantially free of sialic acid.

In one embodiment, the present invention is directed to a composition comprising microalgal extracellular bodies that comprise a heterologous membrane protein comprising a transmembrane domain and an aqueous liquid carrier. In some embodiments, the aqueous liquid carrier is a culture supernatant.

### BRIEF DESCRIPTION OF THE DRAWINGS

**FIG. 1** shows the polynucleotide sequence (SEQ ID NO: 76) that encodes the hemagglutinin (HA) protein of influenza A virus (A/Puerto Rico/8/34/Mount Sinai(H1N1)), which has been codon-optimized for expression in *Schizochytrium* sp. ATCC 20888.
**FIG. 2** shows a plasmid map of pCL0143.
**FIG. 3** shows the procedure used for the analysis of the CL0143-9 clone.
**FIG. 4** shows secretion of HA protein by transgenic *Schizochytrium* CL0143-9 ("E"). **FIG. 4A** shows the recovered recombinant HA protein (as indicated by arrows) in anti-H1N1 immunoblots from the low-speed supernatant (*i.e.,* cell-free supernatant ("CFS")) of cultures at various temperatures (25°C, 27°C, 29°C) and pH (5.5, 6.0, 6.5, 7.0). **FIG. 4B** shows the recovered recombinant HA protein in Coomassie stained gels ("Coomassie") and anti-H1N1 immunoblots ("IB: anti-H1N1") from the 60% sucrose fraction under non-reducing or reducing conditions.

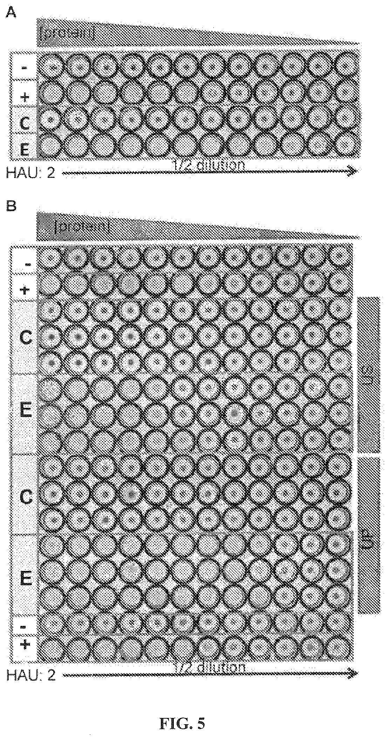

**FIG. 5** shows hemagglutination activity of recombinant HA protein from transgenic *Schizochytrium* CL0143-9 ("E"). **FIG. 5A** shows hemagglutination activity in cell-free supernatant ("CFS"). **FIG. 5B** shows hemagglutination activity in soluble ("US") and insoluble ("UP") fractions. "[protein]" refers to the concentration of protein, decreasing from left to right with increasing dilutions of the samples. "-" refers to negative control lacking HA. "+" refers to Influenza hemagglutinin positive control. "C" refers to the negative control wild-type strain of *Schizochytrium* sp. ATCC 20888. "HAU" refers to Hemagglutinin Activity Unit based on the fold dilution of samples from left to right. "2" refers to a two-fold dilution of the sample in the first well; subsequent wells from left to right represent doubling dilutions over the previous well, such that the fold dilutions from the first to last wells from left to right were 2, 4, 8, 16, 32, 64, 128, 256, 512, 1024, 2048, and 4096.

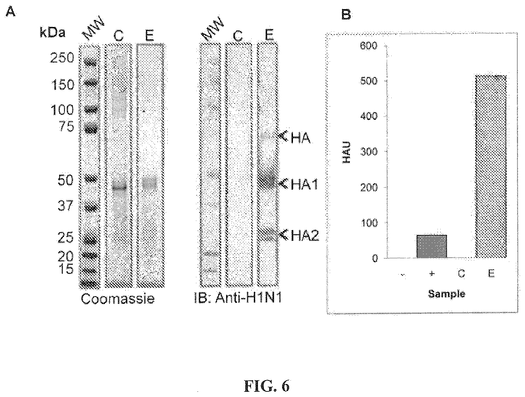

**FIG. 6** shows the expression and hemagglutination activity of HA protein present in the 60% sucrose fraction for transgenic *Schizochytrium* CL0143-9 ("E"). **FIG. 6A** shows the recovered recombinant HA protein (as indicated by arrows) in the Coomassie stained gel ("Coomassie") and anti-H1N1 immunoblot ("IB: anti-H1N1") from the 60% sucrose fraction. **FIG. 6B** shows the corresponding hemagglutination activity. "-" refers to negative control lacking HA. "+" refers to Influenza HA protein positive control. "C" refers to the negative control wild-type strain of *Schizochytrium* sp. ATCC 20888. "HAU" refers to Hemagglutinin Activity Unit based on the fold dilution of samples.
**FIG. 7** shows peptide sequence analysis for the recovered recombinant HA protein, which was identified by a total of 27 peptides (the amino acids associated with the peptides are highlighted in **bold** font), covering over 42% of the entire HA protein sequence (SEQ ID NO: 77). The HA1 polypeptide was identified by a total of 17 peptides, and the HA2 polypeptide was identified by a total of 9 peptides.
**FIG. 8** shows a Coomassie stained gel ("Coomassie") and anti-H1N1 immunoblot ("IB: anti-H1N1") illustrating HA protein glycosylation in *Schizochytrium.* "EndoH" and "PNGase F" refer to enzymatic treatments of the 60% sucrose fraction of transgenic *Schizochytrium* CL0143-9 with the respective enzymes. "NT" refers to transgenic *Schizochytrium* CL0143-9 incubated without enzymes but under the same conditions as the EndoH and PNGase F treatments.
**FIG. 9** shows total *Schizochytrium* sp. ATCC 20888 culture supernatant protein (g/L) over time (hours).
**FIG. 10** shows an SDS-PAGE of total *Schizochytrium* sp. ATCC 20888 culture supernatant protein in lanes 11-15, where the supernatant was collected at five of the six timepoints shown in **FIG. 9** for hours 37-68, excluding hour 52. Bands identified as Actin and Gelsolin (by mass spectral peptide sequencing) are marked with arrows. Lane 11 was loaded with 2.4 µg of total protein; the remaining wells were loaded with 5 µg total protein.
**FIG. 11** shows negatively-stained vesicles from *Schizochytrium* sp. ATCC 20888 ("C: 20888") and transgenic *Schizochytrium* CL0143-9 ("E: CL0143-9").
**FIG. 12** shows anti-H1N1 immunogold labeled vesicles from *Schizochytrium* sp. ATCC 20888 ("C: 20888") and transgenic *Schizochytrium* CL0143-9 ("E: CL0143-9").
**FIG. 13** shows predicted signal anchor sequences native to *Schizochytrium* based on use of the SignalP algorithm. *See,* e.g., Bendsten et al., J, Mol. Biol. 340: 783-795 (2004); Nielsen, H. and Krogh, A. Proc. Int. Conf. Intell. Syst. Mol. Biol. 6: 122-130 (1998); Nielsen, H., et al., Protein Engineering 12: 3-9 (1999); Emanuelsson, O. et al., Nature Protocols 2: 953-971 (2007).
**FIG. 14** shows predicted Type I membrane proteins in *Schizochytrium* based on BLAST searches of genomic and EST DNA *Schizochytrium* databases for genes with homology to known Type I membrane proteins from other organisms and having membrane spanning regions in the extreme C-terminal region of the proteins. Putative membrane spanning regions are shown in bold font.
**FIG. 15** shows a plasmid map of pCL0120.
**FIG. 16** shows a codon usage table for *Schizochytrium.*
**FIG. 17** shows a plasmid map of pCL0130.
**FIG. 18** shows a plasmid map of pCL0131.
**FIG. 19** shows a plasmid map of pCL0121.
**FIG. 20** shows a plasmid map of pCL0122.
**FIG. 21** shows the polynucleotide sequence (SEQ ID NO: 92) that encodes the *Piromyces* sp. E2 xylose isomerase protein "XylA", corresponding to GenBank Accession number CAB76571, optimized for expression in *Schizochytrium* sp. ATCC 20888.
**FIG. 22** shows the polynucleotide sequence (SEQ ID NO: 93) that encodes the *Piromyces* sp. E2 xylulose kinase protein "XylB", corresponding to GenBank Accession number AJ249910, optimized for expression in *Schizochytrium* sp. ATCC 20888.
**FIG. 23** shows a plasmid map of pCL0132.
**FIG. 24** shows a plasmid map of pCL0136.
**FIG. 25A** shows a plasmid map of pCL0140 and **FIG. 25B** shows a plasmid map of pCL0149.
**FIG. 26A** shows the polynucleotide sequence (SEQ ID NO: 100) that encodes neuraminidase (NA) protein of influenza A virus (A/Puerto Rico/8/34/Mount Sinai (H1N1)), optimized for expression in *Schizochytrium* sp. ATCC 20888. **FIG. 26B** shows the polynucleotide sequence (SEQ ID NO: 101) that encodes NA protein of influenza A virus (A/Puerto Rico/8/34/Mount Sinai (H1N1)) followed by a V5 tag and a polyhistidine tag, optimized for expression in *Schizochytrium* sp. ATCC 20888.
**FIG. 27** shows a scheme of the procedure used for the analysis of the CL0140 and CL0149 clones.

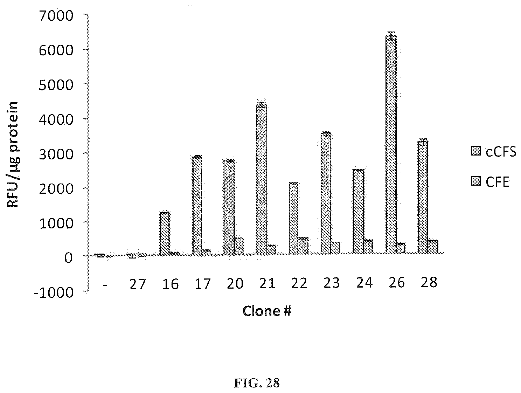

**FIG. 28** shows neuraminidase activity of recombinant NA from transgenic *Schizochytrium* strains CL0140-16, -17, -20, -21, -22, -23, -24, -26, -28. Activity is determined by measuring the fluorescence of 4-methylumbelliferone which arises following the hydrolysis of 4-Methylumbelliferyl -α-D-*N*-Acetylneuraminate (4-MUNANA) by sialidases (Excitation (Exc): 365nm, Emission (Em): 450nm). Activity is expressed as relative fluorescence units (RFU) per µg protein in the concentrated cell-free supernatant (cCFS, leftmost bar for each clone) and the cell-free extract (CFE, rightmost bar for each clone). The wild-type strain of *Schizochytrium* sp. ATCC 20888 ("-") and a PCR-negative strain of *Schizochytrium* transformed with pCL0140 ("27"), grown and prepared in the same manner as the transgenic strains, were used as negative controls.

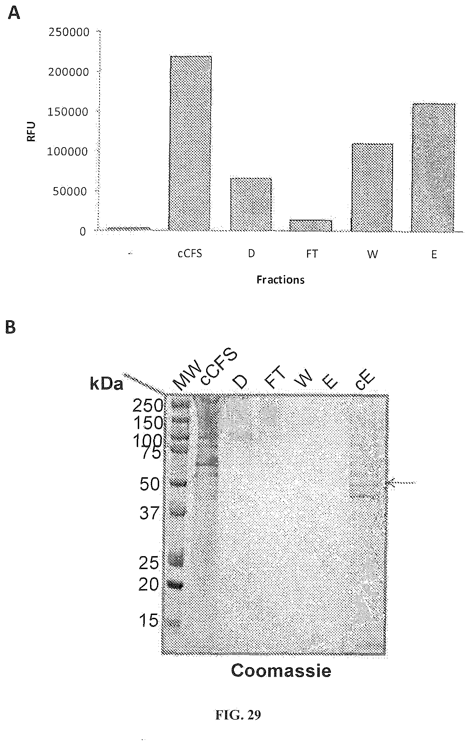

**FIG. 29** shows partial purification of the recombinant NA protein from transgenic *Schizochytrium* strain CL0140-26. The neuraminidase activity of the various fractions is shown in **FIG. 29A.** "cCFS" refers to the concentrated cell-free supernatant. "D" refers to the cCFS diluted with washing buffer, "FT" refers to the flow-through, "W" refers to the wash, "E" refers to the elute and "cE" refers to the concentrated elute fraction. The Coomassie stained gel ("Coomassie") of 12.5 µL of each fraction is shown in **FIG. 29B.** The arrow points to the band identified as the NA protein. SDS-PAGE was used to separate the proteins on NuPAGE^{®} Novex^{®} 12% bis-tris gels with MOPS SDS running buffer.
**FIG. 30** shows peptide sequence analysis for the recovered recombinant NA protein, which was identified by a total of 9 peptides (highlighted in bold red), covering 25% of the protein sequence (SEQ ID NO: 100).

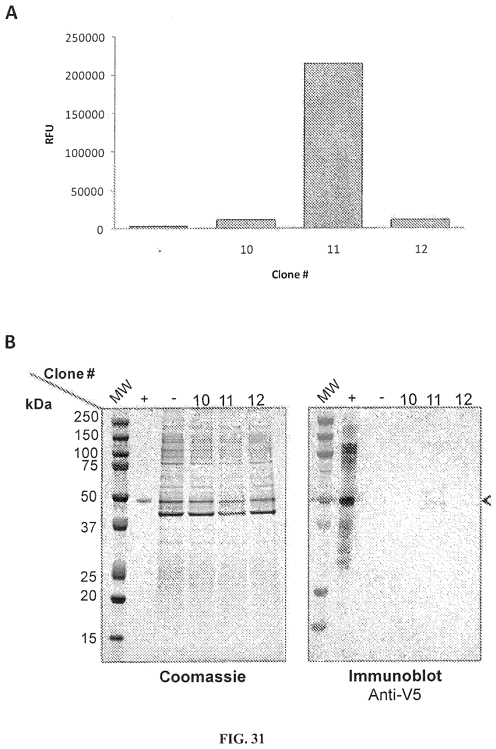

**FIG. 31** shows the neuraminidase activities of transgenic *Schizochytrium* strains CL0149-10, -11, -12 and corresponding Coomassie stained gel ("Coomassie") and anti-V5 immunoblot (("Immunoblot: anti-V5"). **FIG. 31A** shows neuraminidase activity, as determined by measuring the fluorescence of 4-methylumbelliferone which arises following the hydrolysis of 4-MUNANA by sialidases (Exc: 365nm, Em: 450nm). Activity is expressed as relative fluorescence units (RFU) per µg protein in the cell-free supernatant (CFS). The wild-type strain of *Schizochytrium* sp. ATCC 20888 ("-"), grown and prepared in the same manner as the transgenic strain, was used as negative control. **FIG. 31B** shows the Coomassie stained gel and corresponding anti-V5 immunoblot on 12.5 µL CFS for 3 transgenic *Schizochytrium* CL0149 strains ("10", "11", and "12"). The Positope^{™} antibody control protein was used as a positive control ("+"). The wild-type strain of *Schizochytrium* sp. ATCC 20888 ("-"), grown and prepared in the same manner as the transgenic strain, was used as negative control.

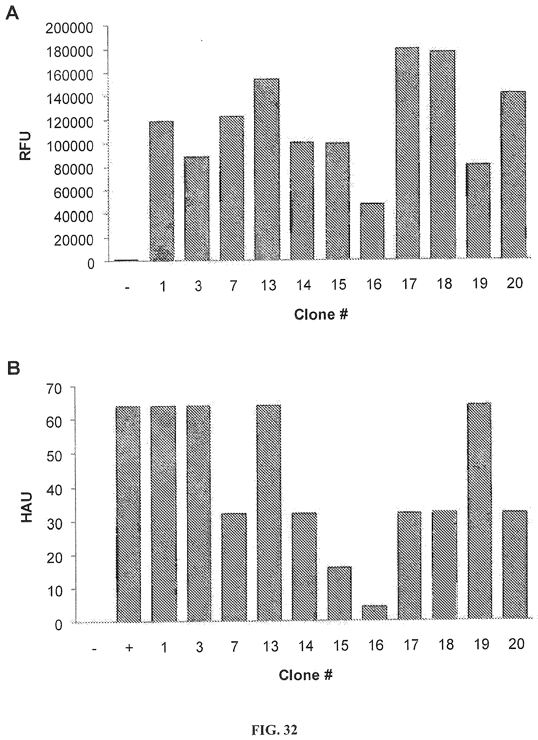

**FIG. 32** shows the enzymatic activities of Influenza HA and NA in the cell-free supernatant of transgenic *Schizochytrium* cotransformed with CL0140 and CL0143. Data are presented for clones CL0140-143-1, -3, -7, -13, -14, -15, -16, -17, -18, -19, -20. **FIG. 32A** shows the neuraminidase activity, as determined by measuring the fluorescence of 4-methylumbelliferone which arises following the hydrolysis of 4-MUNANA by sialidases (Exc: 365nm, Em: 450nm). Activity is expressed as relative fluorescence units (RFU) in 25 µL CFS. The wild-type strain of *Schizochytrium* sp. ATCC 20888 ("-"), grown and prepared in the same manner as the transgenic strain, was used as negative control. **FIG. 32B** shows the hemagglutination activity. "-" refers to negative control lacking HA. "+" refers to Influenza HA positive control. "HAU" refers to Hemagglutinin Activity Unit based on the fold dilution of samples.

### DETAILED DESCRIPTION

The present invention is directed to methods for producing heterologous membrane proteins comprising a transmembrane domain in microalgal host cells.
The present invention is particularly directed to the production of heterologous membrane polypeptides comprising a transmembrane domain in microalgal host cells, wherein the heterologous polypeptides are
associated with microalgal extracellular bodies that are discontinuous with a plasma membrane of the host cells. The present invention is also directed to the production of microalgal extracellular bodies comprising the heterologous membrane polypeptides, as well as the production of compositions comprising the same. The present invention is further directed to the microalgal extracellular bodies comprising the heterologous membrane polypeptides, compositions, and uses thereof.

### Microalgal Host Cells

Microalgae, also known as microscopic algae, are often found in freshwater and marine systems. Microalgae are unicellular but can also grow in chains and groups. Individual cells range in size from a few micrometers to a few hundred micrometers. Because the cells are capable of growing in aqueous suspensions, they have efficient access to nutrients and the aqueous environment.

In some embodiments, the microalgal host cell is a heterokont or stramenopile.

In some embodiments, the microalgal host cell is a member of the phylum Labyrinthulomycota. In some embodiments, the Labyrinthulomycota host cell is a member of the order Thraustochytriales or the order Labyrinthulales. According to the present invention, the term "thraustochytrid" refers to any member of the order Thraustochytriales, which includes the family Thraustochytriaceae, and the term "labyrinthulid" refers to any member of the order Labyrinthulales, which includes the family Labyrinthulaceae. Members of the family Labyrinthulaceae were previously considered to be members of the order Thraustochytriales, but in more recent revisions of the taxonomic classification of such organisms, the family Labyrinthulaceae is now considered to be a member of the order Labyrinthulales. Both Labyrinthulales and Thraustochytriales are considered to be members of the phylum Labyrinthulomycota. Taxonomic theorists now generally place both of these groups of microorganisms with the algae or algae-like protists of the Stramenopile lineage. The current taxonomic placement of the thraustochytrids and labyrinthulids can be summarized as follows:
Realm: Stramenopila (Chromista)
   Phylum: Labyrinthulomycota (Heterokonta)
   Class: Labyrinthulomycetes (Labyrinthulae)
   Order: Labyrinthulales
      Family: Labyrinthulaceae
   Order: Thraustochytriales
      Family: Thraustochytriaceae

For purposes of the present invention, strains described as thraustochytrids include the following organisms: Order: Thraustochytriales; Family:
Thraustochytriaceae; Genera: *Thraustochytrium* (Species: sp., *arudimentale, aureum, benthicola, globosum, kinnei, motivum, multirudimentale, pachydermum, proliferum, roseum, striatum), Ulkenia* (Species: sp., *amoeboidea, kerguelensis, minuta, profunda, radiata, sailens, sarkariana, schizochytrops, visurgensis, yorkensis), Schizochytrium* (Species: sp., *aggregatum, limnaceum, mangrovei, minutum, octosporum), Japonochytrium* (Species: sp., *marinum), Aplanochytrium* (Species: sp., *haliotidis, kerguelensis, profunda, stocchinoi), Althornia* (Species: sp., *crouchii),* or *Elina* (Species: sp., *marisalba, sinorifica).* For the purposes of this invention, species described within *Ulkenia* will be considered to be members of the genus *Thraustochytrium. Aurantiochytrium, Oblongichytrium, Botryochytrium, Parietichytrium,* and *Sicyoidochytrium* are additional genuses encompassed by the phylum Labyrinthulomycota in the present invention.

Strains described in the present invention as Labyrinthulids include the following organisms: Order: Labyrinthulales, Family: Labyrinthulaceae, Genera: *Labyrinthula* (Species: sp., *algeriensis, coenocystis, chattonii, macrocystis, macrocystis atlantica, macrocystis macrocystis, marina, minuta, roscoffensis, valkanovii, vitellina, vitellina pacifica, vitellina vitellina, zopfii), Labyrinthuloides* (Species: sp., *haliotidis, yorkensis), Labyrinthomyxa* (Species: sp., *marina), Diplophrys* (Species: sp., *archeri), Pyrrhosorus* (Species: sp., *marinus), Sorodiplophrys* (Species: sp., *stercorea)* or *Chlamydomyxa* (Species: sp., *labyrinthuloides, montana)* (although there is currently not a consensus on the exact taxonomic placement of *Pyrrhosorus, Sorodiplophrys* or *Chlamydomyxa).*

Microalgal cells of the phylum Labyrinthulomycota include, but are not limited to, deposited strains PTA-10212, PTA-10213, PTA-10214, PTA-10215, PTA-9695, PTA-9696, PTA-9697, PTA-9698, PTA-10208, PTA-10209, PTA-10210, PTA-10211, the microorganism deposited as SAM2179 (named "Ulkenia SAM2179" by the depositor), any *Thraustochytrium* species (including former *Ulkenia* species such as *U. visurgensis, U. amoeboida, U. sarkariana, U. profunda, U radiata, U. minuta and Ulkenia* sp. BP-5601), and including *Thraustochytrium striatum, Thraustochytrium aureum, Thraustochytrium roseum;* and any *Japonochytrium* species. Strains of *Thraustochytriales* include, but are not limited to *Thraustochytrium* sp. (23B) (ATCC 20891); *Thraustochytrium striatum* (Schneider) (ATCC 24473); *Thraustochytrium aureum* (Goldstein) (ATCC 34304); *Thraustochytrium roseum* (Goldstein) (ATCC 28210); and *Japonochytrium* sp. (L1) (ATCC 28207). *Schizochytrium* include, but are not limited to *Schizochytrium aggregatum, Schizochytrium limacinum, Schizochytrium* sp. (S31) (ATCC 20888), *Schizochytrium* sp. (S8) (ATCC 20889), *Schizochytrium* sp. (LC-RM) (ATCC 18915), *Schizochytrium* sp. (SR 21), deposited strain ATCC 28209, and deposited *Schizochytrium limacinum* strain IFO 32693. In some embodiments, the cell is a *Schizochytrium* or a *Thraustochytrium. Schizochytrium* can replicate both by successive bipartition and by forming sporangia, which ultimately release zoospores. *Thraustochytrium,* however, replicate only by forming sporangia, which then release zoospores.

In some embodiments, the microalgal host cell is a Labyrinthulae (also termed Labyrinthulomycetes). Labyrinthulae produce unique structures called "ectoplasmic nets." These structures are branched, tubular extensions of the plasma membrane that contribute significantly to the increased surface area of the plasma membrane. *See,* for example, Perkins, Arch. Mikrobiol. 84:95-118 (1972); Perkins, Can. J. Bot. 51:485-491 (1973). Ectoplasmic nets are formed from a unique cellular structure referred to as a sagenosome or bothrosome. The ectoplasmic net attaches Labyrinthulae cells to surfaces and is capable of penetrating surfaces. *See,* for example, Coleman and Vestal, Can. J. Microbiol. 33:841-843 (1987), and Porter, Mycologia 84:298-299 (1992), respectively. *Schizochytrium* sp. ATCC 20888, for example, has been observed to produce ectoplasmic nets extending into agar when grown on solid media (data not shown). The ectoplasmic net in such instances appears to act as a pseudorhizoid. Additionally, actin filaments have been found to be abundant within certain ectoplasmic net membrane extensions. *See,* for example, Preston, J. Eukaryot. Microbiol. 52:461-475 (2005). Based on the importance of actin filaments within cytoskeletal structures in other organisms, it is expected that cytoskeletal elements such as actin play a role in the formation and/or integrity of ectoplasmic net membrane extensions.

Additional organisms producing pseudorhizoid extensions include organisms termed chytrids, which are taxonomically classified in various groups including the Chytridiomycota, or Phycomyces. Examples of genera include Chytrdium, Chytrimyces, Cladochytium, Lacustromyces, Rhizophydium, Rhisophyctidaceae, Rozella, Olpidium, and Lobulomyces.

In some embodiments, the microalgal host cell comprises a membrane extension. In some embodiments, the microalgal host cell comprises a pseudorhizoid. In some embodiments, the microalgal host cell comprises an ectoplasmic net. In some embodiments, the microalgal host cell comprises a sagenosome or bothrosome.

In some embodiments, the microalgal host cell is a thraustochytrid. In some embodiments, the microalgal host cell is a *Schizochytrium* or *Thraustochytrium* cell.

In some embodiments, the microalgal host cell is a labyrinthulid.

In some embodiments, the microalgal host cell is a eukaryote capable of processing polypeptides through a conventional secretory pathway, such as members of the phylum Labyrinthulomycota, including *Schizochytrium, Thraustochytrium,* and other thraustochytrids. For example, it has been recognized that members of the phylum Labyrinthulomycota produce fewer abundantly-secreted proteins than CHO cells, resulting in an advantage of using *Schizochytrium,* for example, over CHO cells. In addition, unlike *E. coli,* members of the phylum Labyrinthulomycota, such as *Schizochytrium,* perform protein glycosylation, such as N-linked glycosylation, which is required for the biological activity of certain proteins. It has been determined that the N-linked glycosylation exhibited by thraustochytrids such as *Schizochytrium* more closely resembles mammalian glycosylation patterns than does yeast glycosylation.

Effective culture conditions for the host cell include, but are not limited to, effective media, bioreactor, temperature, pH, and oxygen conditions that permit protein production and/or recombination. An effective medium refers to any medium in which a microalgal cell, such as a Thraustochytriales cell, e.g., a *Schizochytrium* host cell, is typically cultured. Such medium typically comprises an aqueous medium having assimilable carbon, nitrogen, and phosphate sources, as well as appropriate salts, minerals, metals, and other nutrients, such as vitamins. Non-limiting examples of suitable media and culture conditions are disclosed in the Examples section. Non-limiting culture conditions suitable for Thraustochytriales microorganisms are also described in U.S. Patent No. 5,340,742.
Cells can be cultured in conventional fermentation bioreactors, shake flasks, test tubes, microtiter dishes, and petri plates. Culturing can be carried out at a temperature, pH, and oxygen content appropriate for a recombinant cell.

In some embodiments, the microalgal host cell contains a recombinant vector comprising a nucleic acid sequence encoding a selection marker. In some embodiments, the selection marker allows for the selection of transformed microorganisms. Examples of dominant selection markers include enzymes that degrade compounds with antibiotic or fungicide activity such as, for example, the Sh ble gene from *Steptoalloteichus hindustanus,* which encodes a "bleomycin-binding protein" represented by SEQ ID NO:5. Another example of a dominant selection marker includes a thraustochytrid acetolactate synthase sequence such as a mutated version of the polynucleotide sequence of SEQ ID NO:6. The acetolactate synthase can be modified, mutated, or otherwise selected to be resistant to inhibition by sulfonylurea compounds, imidazolinone-class inhibitors, and/or pyrimidinyl oxybenzoates. Representative examples of thraustochytrid acetolactate synthase sequences include, but are not limited to, amino acid sequences such as SEQ ID NO:7, SEQ ID NO:8, SEQ ID NO:9, SEQ ID NO:10, or an amino acid sequence that differs from SEQ ID NO:7 by an amino acid deletion, insertion, or substitution at one or more of the following positions: 116G, 117A, 192P, 200A, 251K, 358M, 383D, 592V, 595W, or 599F, and polynucleotide sequences such as SEQ ID NO:11, SEQ ID NO:12, or SEQ ID NO:13, as well as sequences having at least 90%, at least 95%, at least 96%, at least 97%, at least 98%, or at least 99% identity to any of the representative sequences. Further examples of selection markers that can be contained in a recombinant vector for transformation of microalgal cells include ZEOCIN^{™}, paromomycin, hygromycin, blasticidin, or any other appropriate resistance marker.

The term "transformation" is used to refer to any method by which an exogenous nucleic acid molecule (i.e., a recombinant nucleic acid molecule) can be inserted into microbial cells. In microbial systems, the term "transformation" is used to describe an inherited change due to the acquisition of exogenous nucleic acids by the microorganism and is essentially synonymous with the term "transfection." Suitable transformation techniques for introducing exogenous nucleic acid molecules into the microalgal host cells include, but are not limited to, particle bombardment, electroporation, microinjection, lipofection, adsorption, infection, and protoplast fusion. For example, exogenous nucleic acid molecules, including recombinant vectors, can be introduced into a microalgal cell that is in a stationary phase during the exponential growth phase, or when the microalgal cell reaches an optical density of 1.5 to 2 at 600 nm. A microalgal host cell can also be pretreated with an enzyme having protease activity prior to introduction of a nucleic acid molecule into the host cell by electroporation.

In some embodiments, the host cell can be genetically modified to introduce or delete genes involved in biosynthetic pathways associated with the transport and/or synthesis of carbohydrates, including those involved in glycosylation. For example, the host cell can be modified by deleting endogenous glycosylation genes and/or inserting human or animal glycosylation genes to allow for glycosylation patterns that more closely resemble those of humans. Modification of glycosylation in yeast can be found, for example, in U.S. Patent No. 7,029,872 and U.S. Publ. Nos. 2004/0171826, 2004/0230042, 2006/0257399, 2006/0029604, and 2006/0040353. A host cell
also includes a cell in which an RNA viral element is employed to increase or regulate gene expression.

### Expression Systems

The expression system used for expression of a heterologous polypeptide in a microalgal host cell comprises regulatory control elements that are active in microalgal cells. In some embodiments, the expression system comprises regulatory control elements that are active in Labyrinthulomycota cells. In some embodiments, the expression system comprises regulatory control elements that are active in thraustochytrids. In some embodiments, the expression system comprises regulatory control elements that are active in *Schizochytrium* or *Thraustochytrium.* Many regulatory control elements, including various promoters, are active in a number of diverse species. Therefore, regulatory sequences can be utilized in a cell type that is identical to the cell from which they were isolated or can be utilized in a cell type that is different than the cell from which they were isolated. The design and construction of such expression cassettes use standard molecular biology techniques known to persons skilled in the art. *See,* for example, Sambrook et al., 2001, Molecular Cloning: A Laboratory Manual, 3rd edition.

In some embodiments, the expression system used for heterologous polypeptide production in microalgal cells comprises regulatory elements that are derived from Labyrinthulomycota sequences. In some embodiments, the expression system used to produce heterologous polypeptides in microalgal cells comprises regulatory elements that are derived from non-Labyrinthulomycota sequences, including sequences derived from non-Labyrinthulomycota algal sequences. In some embodiments, the expression system comprises a polynucleotide sequence encoding a heterologous polypeptide, wherein the polynucleotide sequence is associated with any promoter sequence, any terminator sequence, and/or any other regulatory sequences that are functional in a microalgal host cell. Inducible or constitutively active sequences can be used. Suitable regulatory control elements also include any of the regulatory control elements associated with the nucleic acid molecules described herein.

The methods of the present invention may utilise an expression cassette for expression of the heterologous membrane polypeptide in a microalgal host cell. The methods of the present invention may also utilise any of the above-described host cells comprising an expression cassette for expression of the
heterologous membrane polypeptide in the host cell. In some embodiments, the expression system comprises an expression cassette containing genetic elements, such as at least a promoter, a coding sequence, and a terminator region operably linked in such a way that they are functional in a host cell. In some embodiments, the expression cassette comprises at least one of the isolated nucleic acid molecules as described herein. In some embodiments, all of the genetic elements of the expression cassette are sequences associated with isolated nucleic acid molecules. In some embodiments, the control sequences are inducible sequences. In some embodiments, the nucleic acid sequence encoding the heterologous membrane polypeptide is integrated into the genome of the host cell. In some embodiments, the nucleic acid sequence encoding the heterologous membrane polypeptide is stably integrated into the genome of the host cell.

In some embodiments, an isolated nucleic acid sequence encoding the heterologous membrane polypeptide to be expressed is operably linked to a promoter sequence and/or a terminator sequence, both of which are functional in the host cell. The promoter and/or terminator sequence to which the isolated nucleic acid sequence encoding a heterologous polypeptide to be expressed is operably linked can include any promoter and/or terminator sequence, including but not limited to the nucleic acid sequences disclosed herein, the regulatory sequences disclosed in U.S. Patent No. 7,001,772, the regulatory sequences disclosed in U.S. Publ. Nos. 2006/0275904 and 2006/0286650, the regulatory sequence disclosed in U.S. Publ. No. 2010/0233760 and WO 2010/107709, or other regulatory sequences functional in the host cell in which they are transformed that are operably linked to the isolated polynucleotide sequence encoding a heterologous polypeptide. In some embodiments, the nucleic acid sequence encoding the heterologous membrane polypeptide is codon-optimized for the specific microalgal host cell to maximize translation efficiency.

The methods of the present invention may utilise recombinant vectors comprising an expression cassette . Recombinant vectors include, but are not limited to, plasmids, phages, and viruses. In some embodiments, the recombinant vector is a linearized vector. In some embodiments, the recombinant vector is an expression vector. As used herein, the phrase "expression vector" refers to a vector that is suitable for production of an encoded product (e.g., a protein of interest). In some embodiments, a nucleic acid sequence encoding the product to be produced is inserted into the recombinant vector to produce a recombinant nucleic acid molecule. The nucleic acid sequence encoding the heterologous polypeptide to be produced is inserted into the vector in a manner that operatively links the nucleic acid sequence to regulatory sequences in the vector (e.g., a Thraustochytriales promoter), which enables the transcription and translation of the nucleic acid sequence within the recombinant microorganism. In some embodiments, a selectable marker, including any of the selectable markers described herein, enables the selection of a recombinant microorganism into which a recombinant nucleic acid molecule has successfully been introduced.

In some embodiments, a heterologous membrane polypeptide produced by the host cell is produced at commercial scale. Commercial scale includes production of heterologous polypeptide from a microorganism grown in an aerated fermentor of a size ≥100 L, ≥1,000 L, ≥10,000 L or ≥100,000 L. In some embodiments, the commercial scale production is done in an aerated fermentor with agitation.

A heterologous polypeptide produced by a host cell
can accumulate within the cell or can be secreted from the cell, e.g., into the culture medium as a soluble heterologous polypeptide.

A heterologous polypeptide may be
recovered from the cell, from the culture medium, or fermentation medium in which the cell is grown. The heterologous polypeptide may be a secreted heterologous polypeptide that is recovered from the culture media as a soluble heterologous polypeptide. The heterologous polypeptide may be a secreted protein comprising a signal peptide.

A heterologous polypeptide may comprise a targeting signal directing its retention in the endoplasmic reticulum, directing its extracellular secretion, or directing it to other organelles or cellular compartments.
The heterologous polypeptide may comprise a signal peptide.
The heterologous polypeptide may comprise a Na/Pi-IIb2 transporter signal peptide or Sec1 transport protein. The signal peptide may comprise the amino acid sequence of SEQ ID NO:1 or SEQ ID NO:37. The heterologous polypeptide comprising a signal peptide having the amino acid sequence of SEQ ID NO:1 or SEQ ID NO:37 may be secreted into the culture medium.

The signal peptide may be cleaved from the protein during the secretory process, resulting in a mature form of the protein.

In some embodiments, a heterologous membrane polypeptide produced by a host cell
is glycosylated. In some embodiments, the glycosylation pattern of the heterologous membrane polypeptide more closely resembles mammalian glycosylation patterns than proteins produced in yeast or *E. coli.* In some embodiments, the heterologous membrane polypeptide produced by a microalgal host cell comprises a N-linked glycosylation pattern. Glycosylated proteins used for therapeutic purposes are less likely to promote anti-glycoform immune responses when their glycosylation patterns are similar to glycosylation patterns found in a subject organism. Conversely, glycosylated proteins having linkages or sugars that are not characteristic of a subject organism are more likely to be antigenic. Effector functions can also be modulated by specific glycoforms. For example, IgG can mediate pro- or antiinflammatory reactions in correlation with the absence or presence, respectively, of terminal sialic acids on Fc region glycoforms (Kaneko et al., Science 313:670-3 (2006)).

A method of producing a recombinant heterologous polypeptide may comprise culturing a recombinant microalgal host cell under conditions sufficient to express a polynucleotide sequence encoding the heterologous polypeptide. The recombinant heterologous polypeptide may be secreted from the host cell and recovered from the culture medium. A heterologous polypeptide that is secreted from the cell may comprise a secretion signal peptide. Depending on the vector and host system used for production, recombinant heterologous polypeptide can remain within the recombinant cell, can be secreted into the fermentation medium, can be secreted into a space between two cellular membranes, or can be retained on the outer surface of a cell membrane. As used herein, the phrase "recovering the protein" refers to collecting fermentation medium containing the protein and need not imply additional steps of separation or purification. Heterologous polypeptides produced by the method
can be purified using a variety of standard protein purification techniques, such as, but not limited to, affinity chromatography, ion exchange chromatography, filtration, electrophoresis, hydrophobic interaction chromatography, gel filtration chromatography, reverse phase chromatography, concanavalin A chromatography, chromatofocusing, and differential solubilization.
Heterologous polypeptides produced by the method
may be isolated in "substantially pure" form. As used herein, "substantially pure" refers to a purity that allows for the effective use of the heterologous polypeptide as a commercial product. The recombinant heterologous polypeptide may accumulate within the cell and be recovered from the cell. The host cell of the method may be a thraustochytrid. The host cell of the method may be a *Schizochytrium* or a *Thraustochytrium.* The recombinant heterologous polypeptide may be a therapeutic protein, a food enzyme, or an industrial enzyme. The recombinant microalgal host cell may be a *Schizochytrium* and the recombinant heterologous polypeptide a therapeutic protein that comprises a secretion signal sequence.

In some embodiments, a recombinant vector used in the methods of the invention is a targeting vector. As used herein, the phrase "targeting vector" refers to a vector that is used to deliver a particular nucleic acid molecule into a recombinant cell, wherein the nucleic acid molecule is used to delete or inactivate an endogenous gene within the host cell (i.e., used for targeted gene disruption or knock-out technology). Such a vector is also known as a "knock-out" vector. In some embodiments, a portion of the targeting vector has a nucleic acid sequence that is homologous to a nucleic acid sequence of a target gene in the host cell (i.e., a gene which is targeted to be deleted or inactivated). In some embodiments, the nucleic acid molecule inserted into the vector (i.e., the insert) is homologous to the target gene. In some embodiments, the nucleic acid sequence of the vector insert is designed to bind to the target gene such that the target gene and the insert undergo homologous recombination, whereby the endogenous target gene is deleted, inactivated, or attenuated (i.e., by at least a portion of the endogenous target gene being mutated or deleted).

### Isolated Nucleic Acid Molecules

An isolated nucleic acid molecule that has utility in the methods of the present invention is a nucleic acid molecule that has been removed from its natural milieu (i.e., that has been subject to human manipulation), its natural milieu being the genome or chromosome in which the nucleic acid molecule is found in nature. As such, "isolated" does not necessarily reflect the extent to which the nucleic acid molecule has been purified, but indicates that the molecule does not include an entire genome or an entire chromosome in which the nucleic acid molecule is found in nature. An isolated nucleic acid molecule can include DNA, RNA (e.g., mRNA), or derivatives of either DNA or RNA (e.g., cDNA). Although the phrase "nucleic acid molecule" primarily refers to the physical nucleic acid molecule and the phrases "nucleic acid sequence" or "polynucleotide sequence" primarily refers to the sequence of nucleotides on the nucleic acid molecule, the phrases are used interchangeably, especially with respect to a nucleic acid molecule, polynucleotide sequence, or a nucleic acid sequence that is capable of encoding a heterologous polypeptide. In some embodiments, an isolated nucleic acid molecule
is produced using recombinant DNA technology (e.g., polymerase chain reaction (PCR) amplification, cloning) or chemical synthesis. Isolated nucleic acid molecules include natural nucleic acid molecules and homologues thereof, including, but not limited to, natural allelic variants and modified nucleic acid molecules in which nucleotides have been inserted, deleted, substituted, and/or inverted in such a manner that such modifications provide the desired effect on sequence, function, and/or the biological activity of the encoded heterologous polypeptide.

A nucleic acid sequence complement of a promoter sequence, terminator sequence, signal peptide sequence, or any other sequence refers to the nucleic acid sequence of the nucleic acid strand that is complementary to the strand with the promoter sequence, terminator sequence, signal peptide sequence, or any other sequence. It will be appreciated that a double-stranded DNA comprises a single-strand DNA and its complementary strand having a sequence that is a complement to the single-strand DNA. As such, nucleic acid molecules can be either double-stranded or single-stranded, and include those nucleic acid molecules that form stable hybrids under "stringent" hybridization conditions with a sequence of the disclosure, and/or with a complement of a sequence of the disclosure. Methods to deduce a complementary sequence are known to those skilled in the art.

The term "polypeptide" includes single-chain polypeptide molecules as well as multiple-polypeptide complexes where individual constituent polypeptides are linked by covalent or non-covalent means. According to the present disclosure, an isolated polypeptide is a polypeptide that has been removed from its natural milieu (i.e., that has been subject to human manipulation) and can include purified proteins, purified peptides, partially purified proteins, partially purified peptides, recombinantly produced proteins or peptides, and synthetically produced proteins or peptides, for example.

As used herein, a recombinant microorganism has a genome which is modified (i.e., mutated or changed) from its normal (i.e., wild-type or naturally occurring) form using recombinant technology. A recombinant microorganism of use in the methods of the present invention can include a microorganism in which nucleic acid molecules have been inserted, deleted, or modified (i.e., mutated, e.g., by insertion, deletion, substitution, and/or inversion of nucleotides), in such a manner that such modification or modifications provide the desired effect within the microorganism. As used herein, genetic modifications which result in a decrease in gene expression, in the function of the gene, or in the function of the gene product (i.e., the protein encoded by the gene) can be referred to as inactivation (complete or partial), deletion, interruption, blockage or down-regulation of a gene. For example, a genetic modification in a gene which results in a decrease in the function of the protein encoded by such gene, can be the result of a complete deletion of the gene (i.e., the gene does not exist in the recombinant microorganism, and therefore the protein does not exist in the recombinant microorganism), a mutation in the gene which results in incomplete or no translation of the protein (e.g., the protein is not expressed), or a mutation in the gene which decreases or abolishes the natural function of the protein (e.g., a protein is expressed which has decreased or no activity (for example, enzymatic activity or action). Genetic modifications which result in an increase in gene expression or function can be referred to as amplification, overproduction, overexpression, activation, enhancement, addition, or up-regulation of a gene.

### Promoters

A promoter is a region of DNA that directs transcription of an associated coding region.

In some embodiments, the promoter is from a microorganism of the phylum Labyrinthulomycota. In some embodiments, the promoter is from a thraustochytrid including, but not limited to: the microorganism deposited as SAM2179 (named "Ulkenia SAM2179" by the depositor), a microorganism of the genus *Ulkenia* or *Thraustochytrium,* or a *Schizochytrium. Schizochytrium* include, but are not limited to, *Schizochytrium aggregatum, Schizochytrium limacinum, Schizochytrium* sp. (S31) (ATCC 20888), *Schizochytrium* sp. (S8) (ATCC 20889), *Schizochytrium* sp. (LC-RM) (ATCC 18915), *Schizochytrium* sp. (SR 21), deposited *Schizochytrium* strain ATCC 28209, and deposited *Schizochytrium* strain IFO 32693.

A promoter can have promoter activity at least in a thraustochytrid, and includes full-length promoter sequences and functional fragments thereof, fusion sequences, and homologues of a naturally occurring promoter. A homologue of a promoter differs from a naturally occurring promoter in that at least one, two, three, or several, nucleotides have been deleted, inserted, inverted, substituted and/or derivatized. A homologue of a promoter can retain activity as a promoter, at least in a thraustochytrid, although the activity can be increased, decreased, or made dependant upon certain stimuli. Promoters can comprise one or more sequence elements that confer developmental and tissue-specific regulatory control or expression.

In some embodiments, an isolated nucleic acid molecule as described herein comprises a PUFA PKS OrfC promoter ("PKS OrfC promoter"; also known as the PFA3 promoter) such as, for example, a polynucleotide sequence represented by SEQ ID NO:3. A PKS OrfC promoter includes a PKS OrfC promoter homologue that is sufficiently similar to a naturally occurring PKS OrfC promoter sequence that the nucleic acid sequence of the homologue is capable of hybridizing under moderate, high, or very high stringency conditions to the complement of the nucleic acid sequence of a naturally occurring PKS OrfC promoter such as, for example, SEQ ID NO:3 or the OrfC promoter of pCL0001 as deposited in ATCC Accession No. PTA-9615.

In some embodiments, an isolated nucleic acid molecule of use in the invention comprises an EF1 short promoter ("EF1 short" or "EF1-S" promoter) or EF1 long promoter ("EF1 long" or "EF1-L" promoter) such as, for example, an EF1 short promoter as represented by SEQ ID NO:42, or an EF1 long promoter as represented by SEQ ID NO:43. An EF1 short or EF1 long promoter includes an EF1 short or long promoter homologue that is sufficiently similar to a naturally occurring EF1 short and/or long promoter sequence, respectively, that the nucleic acid sequence of the homologue is capable of hybridizing under moderate, high, or very high stringency conditions to the complement of the nucleic acid sequence of a naturally occurring EF1 short and/or long promoter such as, for example, SEQ ID NO:42 and/or SEQ ID NO:43, respectively, or the EF1 long promoter of pAB0018 as deposited in ATCC Accession No. PTA-9616.

In some embodiments, an isolated nucleic acid molecule of use in the invention comprises a 60S short promoter ("60S short" or "60S-S" promoter) or 60S long promoter ("60S long" or "60S-L" promoter) such as, for example, a 60S short promoter as represented by SEQ ID NO:44, or a 60S long promoter has a polynucleotide sequence represented by SEQ ID NO:45. In some embodiments, a 60S short or 60S long promoter includes a 60S short or 60S long promoter homologue that is sufficiently similar to a naturally occurring 60S short or 60S long promoter sequence, respectively, that the nucleic acid sequence of the homologue is capable of hybridizing under moderate, high, or very high stringency conditions to the complement of the nucleic acid sequence of a naturally occurring 60S short and/or 60S long such as, for example, SEQ ID NO:44 and/or SEQ ID NO:45, respectively, or the 60S long promoter of pAB0011 as deposited in ATCC Accession No. PTA-9614.

In some embodiments, an isolated nucleic acid molecule comprises a Sec1 promoter ("Sec1 promoter") such as, for example, a polynucleotide sequence represented by SEQ ID NO:46. In some embodiments, a Sec1 promoter includes a Sec1 promoter homologue that is sufficiently similar to a naturally occurring Sec1 promoter sequence that the nucleic acid sequence of the homologue is capable of hybridizing under moderate, high, or very high stringency conditions to the complement of the nucleic acid sequence of a naturally occurring Sec1 promoter such as, for example, SEQ ID NO:46,or the Sec1 promoter of pAB0022 as deposited in ATCC Accession No. PTA-9613.

### Terminators

A terminator region is a section of genetic sequence that marks the end of a gene sequence in genomic DNA for transcription.

In some embodiments, the terminator region is from a microorganism of the phylum Labyrinthulomycota. In some embodiments, the terminator region is from a thraustochytrid. In some embodiments, the terminator region is from a *Schizochytrium* or a *Thraustochytrium. Schizochytrium* include, but are not limited to, *Schizochytrium aggregatum, Schizochytrium limacinum, Schizochytrium* sp. (S31) (ATCC 20888), *Schizochytrium* sp. (S8) (ATCC 20889), *Schizochytrium* sp. (LC-RM) (ATCC 18915), *Schizochytrium* sp. (SR 21), deposited strain ATCC 28209, and deposited strain IFO 32693. In some embodiments, the terminator region is a heterologous terminator region, such as, for example, a heterologous SV40 terminator region.

A terminator region can have terminator activity at least in a thraustochytrid and includes full-length terminator sequences and functional fragments thereof, fusion sequences, and homologues of a naturally occurring terminator region. A homologue of a terminator differs from a naturally occurring terminator in that at least one or a few, but not limited to one or a few, nucleotides have been deleted, inserted, inverted, substituted and/or derivatized. In some embodiments, homologues of a terminator retain activity as a terminator region at least in a thraustochytrid, although the activity can be increased, decreased, or made dependent upon certain stimuli.

In some embodiments, an isolated nucleic acid molecule can comprise a terminator region of a PUFA PKS OrfC gene ("PKS OrfC terminator region", also known as the PFA3 terminator) such as, for example, a polynucleotide sequence represented by SEQ ID NO:4. The terminator region disclosed in SEQ ID NO:4 is a naturally occurring (wild-type) terminator sequence from a thraustochytrid microorganism, and, specifically, is a *Schizochytrium* PKS OrfC terminator region and is termed "OrfC terminator element 1." In some embodiments, a PKS OrfC terminator region includes a PKS OrfC terminator region homologue that is sufficiently similar to a naturally occurring PUFA PKS OrfC terminator region that the nucleic acid sequence of a homologue is capable of hybridizing under moderate, high, or very high stringency conditions to the complement of the nucleic acid sequence of a naturally occurring PKS OrfC terminator region such as, for example, SEQ ID NO:4 or the OrfC terminator region of pAB0011 as deposited in ATCC Accession No. PTA-9614.

### Signal Peptides

In some embodiments, an isolated nucleic acid molecule can comprise a polynucleotide sequence encoding a signal peptide of a secreted protein from a microorganism of the phylum Labyrinthulomycota. In some embodiments, the microorganism is a thraustochytrid. In some embodiments, the microorganism is a *Schizochytrium* or a *Thraustochytrium.*

A signal peptide can have secretion signal activity in a thraustochytrid, and includes full-length peptides and functional fragments thereof, fusion peptides, and homologues of a naturally occurring signal peptide. A homologue of a signal peptide differs from a naturally occurring signal peptide in that at least one or a few, but not limited to one or a few, amino acids have been deleted (e.g., a truncated version of the protein, such as a peptide or fragment), inserted, inverted, substituted and/or derivatized (e.g., by glycosylation, phosphorylation, acetylation, myristoylation, prenylation, palmitation, amidation, and/or addition of glycosylphosphatidyl inositol). In some embodiments, homologues of a signal peptide retain activity as a signal at least in a thraustochytrid, although the activity can be increased, decreased, or made dependant upon certain stimuli.

In some embodiments, the isolated nucleic acid molecule comprises a polynucleotide sequence encoding a Na/Pi-IIb2 transporter protein signal peptide. A Na/Pi-IIb2 transporter protein signal peptide can have signal targeting activity at least for a Na/Pi-IIb2 transporter protein at least in a thraustochytrid, and includes full-length peptides and functional fragments thereof, fusion peptides, and homologues of a naturally occurring Na/Pi-IIb2 transporter protein signal peptide. In some embodiments, the Na/Pi-IIb2 transporter protein signal peptide has an amino acid sequence represented by SEQ ID NO:1. In some embodiments, the Na/Pi-IIb2 transporter protein signal peptide has an amino acid sequence represented by SEQ ID NO:15. In some embodiments, the isolated nucleic acid molecule comprises a polynucleotide sequence encoding an isolated amino acid sequence comprising a functional fragment of SEQ ID NO:1 or SEQ ID NO:15 that functions as a signal peptide, at least for a Na/Pi-IIb2 transporter protein, at least in a thraustochytrid. In some embodiments, the isolated nucleic acid molecule comprises SEQ ID NO:2.

Also disclosed herein is an isolated polypeptide comprising a Na/Pi-IIb2 transporter signal peptide amino acid sequence.

In some embodiments, the isolated nucleic acid molecule comprises a polynucleotide sequence encoding an alpha-1,6-mannosyltransferase (ALG12) signal peptide. An ALG12 signal peptide can have signal targeting activity at least for an ALG12 protein, at least in a thraustochytrid, and includes full-length peptides and functional fragments thereof, fusion peptides, and homologues of a naturally occurring ALG12 signal peptide. In some embodiments, the ALG12 signal peptide has an amino acid sequence represented by SEQ ID NO:59. In some embodiments, the isolated nucleic acid molecule comprises a polynucleotide sequence encoding an isolated amino acid sequence comprising a functional fragment of SEQ ID NO:59 that functions as a signal peptide at least for an ALG12 protein, at least in a thraustochytrid. In some embodiments, the isolated nucleic acid molecule comprises SEQ ID NO:60.

Also disclosed herein is an isolated polypeptide comprising a ALG12 signal peptide amino acid sequence.

In some embodiments, the isolated nucleic acid molecule comprises a polynucleotide sequence encoding a binding immunoglobulin protein (BiP) signal peptide. A BiP signal peptide can have signal targeting activity at least for a BiP protein, at least in a thraustochytrid, and includes full-length peptides and functional fragments thereof, fusion peptides, and homologues of a naturally occurring BiP signal peptide. In some embodiments, the BiP signal peptide has an amino acid sequence represented by SEQ ID NO:61. In some embodiments, the isolated nucleic acid molecule comprises a polynucleotide sequence encoding an isolated amino acid sequence comprising a functional fragment of SEQ ID NO:61 that functions as a signal peptide at least for a BiP protein, at least in a thraustochytrid. In some embodiments, the isolated nucleic acid molecule comprises SEQ ID NO:62.

Also disclosed herein is an isolated polypeptide comprising a BiP signal peptide amino acid sequence.

In some embodiments, the isolated nucleic acid molecule comprises a polynucleotide sequence encoding an alpha-1,3-glucosidase (GLS2) signal peptide. A GLS2 signal peptide can have signal targeting activity at least for a GLS2 protein, at least in a thraustochytrid, and includes full-length peptides and functional fragments thereof, fusion peptides, and homologues of a naturally occurring GLS2 signal peptide. In some embodiments, the GLS2 signal peptide has an amino acid sequence represented by SEQ ID NO:63. In some embodiments, the isolated nucleic acid molecule comprises a polynucleotide sequence encoding an isolated amino acid sequence comprising a functional fragment of SEQ ID NO:63 that functions as a signal peptide at least for a GLS2 protein, at least in a thraustochytrid. In some embodiments, the isolated nucleic acid molecule comprises SEQ ID NO:64.

Also disclosed herein is an isolated polypeptide comprising a GLS2 signal peptide amino acid sequence.

In some embodiments, the isolated nucleic acid molecule comprises a polynucleotide sequence encoding an alpha-1,3-1,6-mannosidase-like signal peptide. A alpha-1,3-1,6-mannosidase-like signal peptide can have signal targeting activity at least for an alpha-1,3-1,6-mannosidase-like protein, at least in a thraustochytrid, and includes full-length peptides and functional fragments thereof, fusion peptides, and homologues of a naturally occurring alpha-1,3-1,6-mannosidase-like signal peptide. In some embodiments, the alpha-1,3-1,6-mannosidase-like signal peptide has an amino acid sequence represented by SEQ ID NO:65. In some embodiments, the isolated nucleic acid molecule comprises a polynucleotide sequence encoding an isolated amino acid sequence comprising a functional fragment of SEQ ID NO:65 that functions as a signal peptide at least for an alpha-1,3-1,6-mannosidase-like protein, at least in a thraustochytrid. In some embodiments, the isolated nucleic acid molecule comprises SEQ ID NO:66.

Also disclosed herein is an isolated polypeptide comprising a alpha-1,3-1,6-mannosidase-like signal peptide amino acid sequence.

In some embodiments, the isolated nucleic acid molecule comprises a polynucleotide sequence encoding an alpha-1,3-1,6-mannosidase-like #1 signal peptide. An alpha-1,3-1,6-mannosidase-like #1 signal peptide can have signal targeting activity at least for an alpha-1,3-1,6-mannosidase-like #1 protein, at least in a thraustochytrid, and includes full-length peptides and functional fragments thereof, fusion peptides, and homologues of a naturally occurring alpha-1,3-1,6-mannosidase-like #1 signal peptide. In some embodiments, the alpha-1,3-1,6-mannosidase-like #1 signal peptide has an amino acid sequence represented by SEQ ID NO:67. In some embodiments, the isolated nucleic acid molecule comprises a polynucleotide sequence encoding an isolated amino acid sequence comprising a functional fragment of SEQ ID NO:67 that functions as a signal peptide at least for an alpha-1,3-1,6-mannosidase-like #1 protein, at least in a thraustochytrid. In some embodiments, the isolated nucleic acid molecule comprises SEQ ID NO:68.

Also disclosed herein is an isolated polypeptide comprising a alpha-1,3-1,6-mannosidase-like #1 signal peptide amino acid sequence.

In some embodiments, the isolated nucleic acid molecule comprises a polynucleotide sequence encoding an alpha-1,2-mannosidase-like signal peptide. An alpha-1,2-mannosidase-like signal peptide can have signal targeting activity at least for an alpha-1,2-mannosidase-like protein, at least in a thraustochytrid, and includes full-length peptides and functional fragments thereof, fusion peptides, and homologues of a naturally occurring alpha-1,2-mannosidase-like signal peptide. In some embodiments, the alpha-1,2-mannosidase-like signal peptide has an amino acid sequence represented by SEQ ID NO:69. In some embodiments, the isolated nucleic acid molecule comprises a polynucleotide sequence encoding an isolated amino acid sequence comprising a functional fragment of SEQ ID NO:69 that functions as a signal peptide at least for an alpha-1,2-mannosidase-like protein, at least in a thraustochytrid. In some embodiments, the isolated nucleic acid molecule comprises SEQ ID NO:70.

Also disclosed herein is an isolated polypeptide comprising a alpha-1,2-mannosidase-like signal peptide amino acid sequence.

In some embodiments, the isolated nucleic acid molecule comprises a polynucleotide sequence encoding a beta-xylosidase-like signal peptide. A beta-xylosidase-like signal peptide can have signal targeting activity at least for a beta-xylosidase-like protein, at least in a thraustochytrid, and includes full-length peptides and functional fragments thereof, fusion peptides, and homologues of a naturally occurring beta-xylosidase-like signal peptide. In some embodiments, the beta-xylosidase-like signal peptide has an amino acid sequence represented by SEQ ID NO:71. In some embodiments, the isolated nucleic acid molecule comprises a polynucleotide sequence encoding an isolated amino acid sequence comprising a functional fragment of SEQ ID NO:71 that functions as a signal peptide at least for a beta xylosidase-like protein, at least in a thraustochytrid. In some embodiments, the isolated nucleic acid molecule comprises SEQ ID NO:72.

Also disclosed herein is an isolated polypeptide comprising a beta-xylosidase-like signal peptide amino acid sequence.

In some embodiments, the isolated nucleic acid molecule comprises a polynucleotide sequence encoding a carotene synthase signal peptide. A carotene synthase signal peptide can have signal targeting activity at least for a carotene synthase protein, at least in a thraustochytrid, and includes full-length peptides and functional fragments thereof, fusion peptides, and homologues of a naturally occurring carotene synthase signal peptide. In some embodiments, the carotene synthase signal peptide has an amino acid sequence represented by SEQ ID NO:73. In some embodiments, the isolated nucleic acid molecule comprises a polynucleotide sequence encoding an isolated amino acid sequence comprising a functional fragment of SEQ ID NO:73 that functions as a signal peptide at least for a carotene synthase protein, at least in a thraustochytrid. In some embodiments, the isolated nucleic acid molecule comprises SEQ ID NO:74.

Also disclosed herein is an isolated polypeptide comprising a carotene synthase signal peptide amino acid sequence.

In some embodiments, the isolated nucleic acid molecule comprises a polynucleotide sequence encoding a Sec1 protein ("Sec1") signal peptide. A Sec1 signal peptide can have secretion signal activity at least for a Sec1 protein at least in a thraustochytrid, and includes full-length peptides and functional fragments thereof, fusion peptides, and homologues of a naturally occurring Sec1 signal peptide. In some embodiments, the Sec1 signal peptide is represented by SEQ ID NO:37. In some embodiments, the isolated nucleic acid molecule comprises a polynucleotide sequence encoding an isolated amino acid sequence comprising a functional fragment of SEQ ID NO:37 that functions as a signal peptide, at least for a Sec1 protein, at least in a thraustochytrid. In some embodiments, the isolated nucleic acid molecule comprises SEQ ID NO:38.

Also disclosed herein is an isolated polypeptide comprising a Sec1 signal peptide amino acid sequence.

In some embodiments, an isolated nucleic acid molecule can comprise a promoter sequence, a terminator sequence, and/or a signal peptide sequence that is at least 90%, 95%, 96%, 97%, 98%, or 99% identical to any of the promoter, terminator, and/or signal peptide sequences described herein.

In some embodiments, an isolated nucleic acid molecule comprises an OrfC promoter, EF1 short promoter, EF1 long promoter, 60S short promoter, 60S long promoter, Sec1 promoter, PKS OrfC terminator region, sequence encoding a Na/Pi-IIb2 transporter protein signal peptide, or sequence encoding a Sec1 transport protein signal peptide that is operably linked to the 5' end of a nucleic acid sequence encoding a heterologous polypeptide. Recombinant vectors (including, but not limited to, expression vectors), expression cassettes, and host cells can also comprise an OrfC promoter, EF1 short promoter, EF1 long promoter, 60S short promoter, 60S long promoter, Sec1 promoter, PKS OrfC terminator region, sequence encoding a Na/Pi-IIb2 transporter protein signal peptide, or sequence encoding a Sec1 transport protein signal peptide that is operably linked to the 5' end of a nucleic acid sequence encoding a heterologous polypeptide.

As used herein, unless otherwise specified, reference to a percent (%) identity (and % identical) refers to an evaluation of homology which is performed using: (1) a BLAST 2.0 Basic BLAST homology search using blastp for amino acid searches and blastn for nucleic acid searches with standard default parameters, wherein the query sequence is filtered for low complexity regions by default *(see,* for example, Altschul, S., et al., Nucleic Acids Res. 25:3389-3402 (1997));
(2) a BLAST 2 alignment using the parameters described below; (3) and/or PSI-BLAST (Position-Specific Iterated BLAST) with the standard default parameters. It is noted that due to some differences in the standard parameters between BLAST 2.0 Basic BLAST and BLAST 2, two specific sequences might be recognized as having significant homology using the BLAST 2 program, whereas a search performed in BLAST 2.0 Basic BLAST using one of the sequences as the query sequence may not identify the second sequence in the top matches. In addition, PSI-BLAST provides an automated, easy-to-use version of a "profile" search, which is a sensitive way to look for sequence homologues. The program first performs a gapped BLAST database search. The PSI-BLAST program uses the information from any significant alignments returned to construct a position-specific score matrix, which replaces the query sequence for the next round of database searching. Therefore, it is to be understood that percent identity can be determined by using any one of these programs.

Two specific sequences can be aligned to one another using BLAST 2 sequence as described, for example, in Tatusova and Madden, FEMS Microbiol. Lett. 174:247-250 (1999). BLAST 2 sequence alignment is performed in blastp or blastn using the BLAST 2.0 algorithm to perform a Gapped BLAST search (BLAST 2.0) between the two sequences allowing for the introduction of gaps (deletions and insertions) in the resulting alignment. BLAST 2 sequence alignment may be performed using the standard default parameters as follows.
For blastn, using 0 BLOSUM62 matrix:
   Reward for match = 1
   Penalty for mismatch = -2
   Open gap (5) and extension gap (2) penalties gap x_dropoff (50) expect (10) word size (11) filter (on).
For blastp, using 0 BLOSUM62 matrix:
   Open gap (11) and extension gap (1) penalties
   gap x_dropoff (50) expect (10) word size (3) filter (on).

As used herein, hybridization conditions refer to standard hybridization conditions under which nucleic acid molecules are used to identify similar nucleic acid molecules. See, for example, Sambrook J. and Russell D. (2001) Molecular cloning: A laboratory manual, 3rd ed. Cold Spring Harbor Laboratory Press, Cold Spring Harbor, New York.
In addition, formulae to calculate the appropriate hybridization and wash conditions to achieve hybridization permitting varying degrees of mismatch of nucleotides are disclosed, for example, in Meinkoth et al., Anal. Biochem. 138:267-284 (1984).
One of skill in the art can use the formulae in Meinkoth *et al.,* for example, to calculate the appropriate hybridization and wash conditions to achieve particular levels of nucleotide mismatch. Such conditions will vary, depending on whether DNA:RNA or DNA:DNA hybrids are being formed. Calculated melting temperatures for DNA:DNA hybrids are 10°C less than for DNA:RNA hybrids. In particular instances, stringent hybridization conditions for DNA:DNA hybrids include hybridization at an ionic strength of 6X SSC (0.9 M Na⁺) at a temperature of between 20°C and 35°C (lower stringency), between 28°C and 40°C (more stringent), and between 35°C and 45°C (even more stringent), with appropriate wash conditions. In particular instances, stringent hybridization conditions for DNA:RNA hybrids include hybridization at an ionic strength of 6X SSC (0.9 M Na⁺) at a temperature of between 30°C and 45°C, between 38°C and 50°C, and between 45°C and 55°C, with similarly stringent wash conditions. These values are based on calculations of a melting temperature for molecules larger than about 100 nucleotides, 0% formamide, and a G+C content of about 40%. Alternatively, Tₘ can be calculated empirically as set forth in Sambrook *et al.* In general, the wash conditions should be as stringent as possible, and should be appropriate for the chosen hybridization conditions. For example, hybridization conditions can include a combination of salt and temperature conditions that are approximately 20-25°C below the calculated Tₘ of a particular hybrid, and wash conditions typically include a combination of salt and temperature conditions that are approximately 12-20°C below the calculated Tₘ of the particular hybrid. One example of hybridization conditions suitable for use with DNA:DNA hybrids includes a 2-24 hour hybridization in 6X SSC (50% formamide) at 42°C, followed by washing steps that include one or more washes at room temperature in 2X SSC, followed by additional washes at higher temperatures and lower ionic strength (e.g., at least one wash as 37°C in 0.1X - 0.5X SSC, followed by at least one wash at 68°C in 0.1X - 0.5X SSC).

### Heterologous Polypeptides

The term "heterologous" as used herein refers to a sequence that is not naturally found in the microalgal host cell. In some embodiments, heterologous membrane polypeptides produced by the methods of the invention include, but are not limited to, therapeutic proteins. A "therapeutic protein" as used herein includes proteins that are useful for the treatment or prevention of diseases, conditions, or disorders in animals and humans.

In certain embodiments, therapeutic proteins include, but are not limited to, biologically active proteins, e.g., enzymes, antibodies, or antigenic proteins. ,

In some embodiments, heterologous polypeptides produced by the methods
of the invention include, but are not limited to industrial enzymes. Industrial enzymes include, but are not limited to, enzymes that are used in the manufacture, preparation, preservation, nutrient mobilization, or processing of products, including food, medical, chemical, mechanical, and other industrial products.

In some embodiments, heterologous polypeptides produced by the methods
of the invention include an auxotrophic marker, a dominant selection marker (such as, for example, an enzyme that degrades antibiotic activity) or another protein involved in transformation selection, a protein that functions as a reporter, an enzyme involved in protein glycosylation, and an enzyme involved in cell metabolism.

In some embodiments, a heterologous polypeptide produced by the methods
of the invention includes a viral protein selected from the group consisting of a H or HA (hemagglutinin) protein, a N or NA (neuraminidase) protein, a F (fusion) protein, a G (glycoprotein) protein, an E or env (envelope) protein, a gp120 (glycoprotein of 120 kDa), and a gp41 (glycoprotein of 41 kDa). In some embodiments, a heterologous polypeptide produced by the methods of the invention is a viral matrix protein. In some embodiments, a heterologous polypeptide produced by the methods
of the invention is a viral matrix protein selected from the group consisting of M1, M2 (a membrane channel protein), Gag, and combinations thereof. In some embodiments, the HA, NA, F, G, E, gp120, gp41, or matrix protein is from a viral source, e.g., an influenza virus or a measles virus.

Influenza is the leading cause of death in humans due to a respiratory virus. Common symptoms include fever, sore throat, shortness of breath, and muscle soreness, among others. Influenza viruses are enveloped viruses that bud from the plasma membrane of infected mammalian and avian cells. They are classified into types A, B, or C, based on the nucleoproteins and matrix protein antigens present. Influenza type A viruses can be further divided into subtypes according to the combination of HA and NA surface glycoproteins presented. HA is an antigenic glycoprotein, and plays a role in binding the virus to cells that are being infected. NA removes terminal sialic acid residues from glycan chains on host cell and viral surface proteins, which prevents viral aggregation and facilitates virus mobility.

The influenza viral HA protein is a homo trimer with a receptor binding pocket on the globular head of each monomer, and the influenza viral NA protein is a tetramer with an enzyme active site on the head of each monomer. Currently, 16 HA (H1-H16) and 9 NA (N1-N9) subtypes are recognized. Each type A influenza virus presents one type of HA and one type of NA glycoprotein. Generally, each subtype exhibits species specificity; for example, all HA and NA subtypes are known to infect birds, while only subtypes H1, H2, H3, H5, H7, H9, H10, N1 , N2, N3 and N7 have been shown to infect humans. Influenza viruses are characterized by the type of HA and NA that they carry, *e.g.,* H1N1, H5N1, H1N2, H1N3, H2N2, H3N2, H4N6, H5N2, H5N3, H5N8, H6N1, H7N7, H8N4, H9N2, H10N3, H11N2, H11N9, H12N5, H13N8, H15N8, H16N3, etc. Subtypes are further divided into strains; each genetically distinct virus isolate is usually considered to be a separate strain, e.g., influenza A/Puerto Rico/8/34/Mount Sinai(H1N1) and influenza A/Vietnam/1203/2004(H5N1). In certain embodiments of the invention, the HA is from an influenza virus, e.g., the HA is from a type A influenza, a type B influenza, or is a subtype of type A influenza, selected from the group consisting of H1, H2, H3, H4, H5, H6, H7, H8, H9, H10, H11, H12, H13, H14, H15, and H16. In another embodiment, the HA is from a type A influenza, selected from the group consisting of H1, H2, H3, H5, H6, H7 and H9. In one embodiment, the HA is from influenza subtype H1N1.

An influenza virus HA protein is translated in cells as a single protein, which after cleavage of the signal peptide is an approximately 62 kDa protein (by conceptual translation) referred to as HA0 (i.e., hemagglutinin precursor protein). For viral activation, hemagglutinin precursor protein (HA0) must be cleaved by a trypsin-like serine endoprotease at a specific site, normally coded for by a single basic amino acid (usually arginine) between the HA1 and HA2 polypeptides of the protein. In the specific example of the A/Puerto Rico/8/34 strain, this cleavage occurs between the arginine at amino acid 343 and the glycine at amino acid 344. After cleavage, the two disulfide-bonded protein polypeptides produce the mature form of the protein subunits as a prerequisite for the conformational change necessary for fusion and hence viral infectivity.

In some embodiments, the HA protein is cleaved, e.g., a HA0 protein is cleaved into HA1 and HA2. In some embodiments, expression of the HA protein in a microalgal host cell such as *Schizochytrium,* results in proper cleavage of the HA0 protein into functional HA1 and HA2 polypeptides without addition of an exogenous protease. Such cleavage of hemagglutinin in a non-vertebrate expression system without addition of exogenous protease has not been previously demonstrated.

A viral F protein can comprise a single-pass transmembrane domain near the C-terminus. The F protein can be split into two peptides at the Furin cleavage site (amino acid 109). The first portion of the protein designated F2 contains the N-terminal portion of the complete F protein. The remainder of the viral F protein containing the C-terminal portion of the F protein is designated F1. The F1 and/or F2 regions can be fused individually to heterologous sequences, such as, for example, a sequence encoding a heterologous signal peptide. Vectors containing the F1 and F2 portions of the viral F protein can be expressed individually or in combination. A vector expressing the complete F protein can be co-expressed with the furin enzyme that will cleave the protein at the furin cleavage site. Alternatively, the sequence encoding the furin cleavage site of the F protein can be replaced with a sequence encoding an alternate protease cleavage site that is recognized and cleaved by a different protease. The F protein containing an alternate protease cleavage site can be co-expressed with a corresponding protease that recognizes and cleaves the alternate protease cleavage site.

In some embodiments, an HA, NA, F, G, E, gp120, gp41, or matrix protein is a full-length protein, a fragment, a variant, a derivative, or an analogue thereof. In some embodiments, a HA, NA, F, G, E, gp120, gp41, or matrix protein is a polypeptide comprising an amino acid sequence or a polynucleotide encoding a polypeptide comprising an amino acid sequence at least 90%, at least 91%, at least 92%, at least 93%, at least 94%, at least 95%, at least 96%, at least 97%, at least 98%, at least 99%, or 100% identical to a known sequence for the respective viral proteins, wherein the polypeptide is recognizable by an antibody that specifically binds to the known sequence. The HA sequence, for example, can be a full-length HA protein which consists essentially of the extracellular (ECD) domain, the transmembrane (TM) domain, and the cytoplasmic (CYT) domain; or a fragment of the entire HA protein which consists essentially of the HA1 polypeptide and the HA2 polypeptide, e.g., produced by cleavage of a full-length HA; or a fragment of the entire HA protein which consists essentially of the HA1 polypeptide, HA2 polypeptide and the TM domain; or a fragment of the entire HA protein which consists essentially of the CYT domain; or a fragment of the entire HA protein which consists essentially of the TM domain; or a fragment of the entire HA protein which consists essentially of the HA1 polypeptide; or a fragment of the entire HA protein which consists essentially of the HA2 polypeptide. The HA sequence can also include an HA1/HA2 cleavage site. The HA1/HA2 cleavage site can be located between the HA1 and HA2 polypeptides, but also can be arranged in any order relative to the other sequences of the polynucleotide or polypeptide construct. The viral proteins can be from a pathogenic virus strain.

In some embodiments, a heterologous polypeptide produced in the invention is a fusion polypeptide comprising a full-length HA, NA, F, G, E, gp120, gp41, or matrix protein, or a fragment, variant, derivative, or analogue thereof.

In some embodiments, a heterologous polypeptide is a fusion polypeptide comprising a HA0 polypeptide, a HA1 polypeptide, a HA2 polypeptide, a TM domain, fragments thereof, and combinations thereof. In some embodiments, the heterologous polypeptide comprises combinations of two or more of a HA1 polypeptide, a HA2 polypeptide, a TM domain, or fragments thereof from different subtypes or different strains of a virus, such as from different subtypes or strains of an influenza virus. In some embodiments, the heterologous polypeptide comprises combinations of two or more of a HA1 polypeptide, a HA2 polypeptide, a TM domain, or fragments thereof from different viruses, such as from an influenza virus and a measles virus.

Hemagglutination activity can be determined by measuring agglutination of red blood cells. Hemagglutination and subsequent precipitation of red blood cells results from hemagglutinins being adsorbed onto the surface of red blood cells. Clusters of red blood cells, distinguishable to the naked eye as heaps, lumps, and/or clumps, are formed during hemagglutination. Hemagglutination is caused by the interaction of the agglutinogens present in red blood cells with plasma that contains agglutinins. Each agglutinogen has a corresponding agglutinin. A hemagglutination reaction is used, *e.g.,* to determine antiserum activity or type of virus. A distinction is made between active hemagglutination, which is caused by the direct action of an agent on the red blood cells, and passive hemagglutination, caused by a specific antiserum to the antigen previously adsorbed by the red blood cells. The amount of hemagglutination activity in a sample can be measured, e.g., in hemagglutination activity units (HAU). Hemagglutination may be caused by, e.g., the polysaccharides of the causative bacteria of tuberculosis, plague, and tularemia, by the polysaccharides of the colon bacillus, and by the viruses of influenza, mumps, pneumonia of white mice, swine and horse influenza, smallpox vaccine, yellow fever, and other hemagglutination-inducing diseases.

### Microalgal Extracellular Bodies

The present invention is also directed to a microalgal extracellular body comprising a heterologous membrane protein comprising a transmembrane domain, wherein the extracellular body is discontinuous with the plasma membrane. By "discontinuous with the plasma membrane" is meant that the microalgal extracellular body is not connected to the plasma membrane of a host cell. In some embodiments, the extracellular body is a membrane. In some embodiments, the extracellular body is a vesicle, micelle, membrane fragment, membrane aggregate, or a mixture thereof. The term "vesicle" as used herein refers to a closed structure comprising a lipid bilayer (unit membrane), e.g., a bubble-like structure formed by a cell membrane. The term "membrane aggregate" as used herein refers to any collection of membrane structures that become associated as a single mass. A membrane aggregate can be a collection of a single type of membrane structure such as, but not limited to, a collection of membrane vesicles, or can be a collection of more than a single type of membrane structure such as, but not limited to, a collection of at least two of a vesicle, micelle, or membrane fragment. The term "membrane fragment" as used herein refers to any portion of a membrane capable of comprising a heterologous polypeptide as described herein. In some embodiments, a membrane fragment is a membrane sheet. In some embodiments, the extracellular body is a mixture of a vesicle and a membrane fragment. In some embodiments, the extracellular body is a vesicle. In some embodiments, the vesicle is a collapsed vesicle. In some embodiments, the vesicle is a virus-like particle. In some embodiments, the extracellular body is an aggregate of biological materials comprising native and heterologous polypeptides produced by the host cell. In some embodiments, the extracellular body is an aggregate of native and heterologous polypeptides. In some embodiments, the extracellular body is an aggregate of heterologous polypeptides.

In some embodiments, the ectoplasmic net of a microalgal host cell becomes fragmented during culturing of a microalgal host cell, resulting in the formation of a microalgal extracellular body. In some embodiments, the microalgal extracellular body is formed by fragmentation of the ectoplasmic net of a microalgal host cell as a result of hydrodynamic forces in the stirred media that physically shear ectoplasmic net membrane extensions.

In some embodiments, the microalgal extracellular body is formed by extrusion of a microalgal membrane, such as, but not limited to, extrusion of a plasma membrane, an ectoplasmic net, a pseudorhizoid, or a combination thereof, wherein the extruding membrane becomes separated from the plasma membrane.

In some embodiments, the microalgal extracellular bodies are vesicles or micelles having different diameters, membrane fragments having different lengths, or a combination thereof.

In some embodiments, the extracellular body is a vesicle having a diameter from 10 nm to 2500 nm, 10 nm to 2000 nm, 10 nm to 1500 nm, 10 nm to 1000 nm, 10 nm to 500 nm, 10 nm to 300 nm, 10 nm to 200 nm, 10 nm to 100 nm, 10 nm to 50 nm, 20 nm to 2500 nm, 20 nm to 2000 nm, 20 nm to 1500 nm, 20 nm to 1000 nm, 20 nm to 500 nm, 20 nm to 300 nm, 20 nm to 200 nm, 20 nm to 100 nm, 50 nm to 2500 nm, 50 nm to 2000 nm, 50 nm to 1500 nm, 50 nm to 1000 nm, 50 nm to 500 nm, 50 nm to 300 nm, 50 nm to 200 nm, 50 nm to 100 nm, 100 nm to 2500 nm, 100 nm to 2000 nm, 100 nm to 1500 nm, 100 nm to 1000 nm, 100 nm to 500 nm, 100 nm to 300 nm, 100 nm to 200 nm, 500 nm to 2500 nm, 500 nm to 2000 nm, 500 nm to 1500 nm, 500 nm to 1000 nm, 2000 nm or less, 1500 nm or less, 1000 nm or less, 500 nm or less, 400 nm or less, 300 nm or less, 200 nm or less, 100 nm or less, or 50 nm or less.

Non-limiting fermentation conditions for producing microalgal extracellular bodies from thraustochytrid host cells are shown below in **Table 1**:

**Table 1: Vessel Media**

| Ingredient | Concentration | | Ranges |
|---|---|---|---|
| Na₂SO₄ | g/L | 13.62 | 0-50, 15-45, or 25-35 |
| K2SO4 | g/L | 0.72 | 0-25, 0.1-10, or 0.5-5 |
| KCl | g/L | 0.56 | 0-5, 0.25-3, or 0.5-2 |
| MgSO₄·7H₂O | g/L | 2.27 | 0-10, 1-8, or 2-6 |
| (NH₄)₂SO₄ | g/L | 17.5 | 0-50, 0.25-30, or 5-20 |
| CaCl₂·2H₂O | g/L | 0.19 | 0.1-5, 0.1-3, or 0.15-1 |
| KH₂PO₄ | g/L | 6.0 | 0-20, 0.1-10, or 1-7 |

| Post autoclave (Metals) | | | |
|---|---|---|---|
| Citric acid | mg/L | 3.50 | 0.1-5000 1-3000 or 3-2500 |
| FeSO₄·7H₂O | mg/L | 51.5 | 0.1-1000, 1-500, or 5-100 |
| MnCl₂·4H₂O | mg/L | 3.10 | 0.1-100, 1-50, or 2-25 |
| ZnSO₄·7H₂O | mg/L | 6.20 | 0.1-100, 1-50, or 2-25 |
| CoCl₂·6H₂O | mg/L | 0.04 | 0-1, 0.001-0.1, or 0.01-0.1 |
| Na₂MoO₄·2H₂O | mg/L | 0.04 | 0.001-1, 0.005-0.5, or 0.01-0.1 |
| CuSO₄·5H₂O | mg/L | 2.07 | 0.1-100, 0.5-50, or 1-25 |
| NiSO₄·6H₂O | mg/L | 2.07 | 0.1-100, 0.5-50, or 1-25 |

| Post autoclave (Vitamins) | | | |
|---|---|---|---|
| Thiamine∗∗ | mg/L | 9.75 | 0.1-100, 1-50, or 5-25 |
| Vitamin B12∗∗ | mg/L | 0.16 | 0.01-100, 0.05-5, or 0.1-1.0 |
| Ca½-pantothenate∗∗ | mg/L | 3.33 | 0.1-100, 0.1-50, or 1-10 |
| ∗∗ filter sterilized and | added post-autoclave | | |

| Post autoclave (Carbon) | | | |
|---|---|---|---|
| Glucose | g/L | 20.0 | 5-150, 10-100, or 20-5 |
| Nitrogen Feed: | | | |

| Ingredient | Concentration | | |
|---|---|---|---|
| NH₄OH | mL/L | 23.6 | 5-150, 10-100, 15-50 |

General cultivation conditions for producing microalgal extracellular bodies include the following:

| | |
|---|---|
| pH: | 5.5-9.5, 6.5-8.0, or 6.3-7.3 |
| temperature: | 15 °C-45 °C, 18 °C-35 °C, or 20 °C-30 °C |
| dissolved oxygen: | 0.1%-100% saturation, 5%-50% saturation, or 10%-30% saturation |
| glucose controlled: | 5 g/L-100 g/L, 10 g/L-40 g/L, or 15 g/L-35 g/L. |

In some embodiments, the microalgal extracellular body is produced from a Labyrinthulomycota host cell. In some embodiments, the microalgal extracellular body is produced from a Labyrinthulae host cell. In some embodiments, the microalgal extracellular body is produced from a thraustochytrid host cell. In some embodiments, the microalgal extracellular body is produced from a *Schizochytrium* or *Thraustochytrium.*

The present invention is also directed to a microalgal extracellular body comprising a heterologous membrane protein comprising a transmembrane domain, wherein the extracellular body is discontinuous with a plasma membrane of a microalgal host cell.

In some embodiments, a microalgal extracellular body of the invention comprises a polypeptide that is also associated with a plasma membrane of a microalgal host cell. In some embodiments, a polypeptide associated with a plasma membrane of a microalgal host cell includes a native membrane polypeptide, a heterologous polypeptide, and a combination thereof.

In some embodiments, the heterologous polypeptide is contained within a microalgal extracellular body.

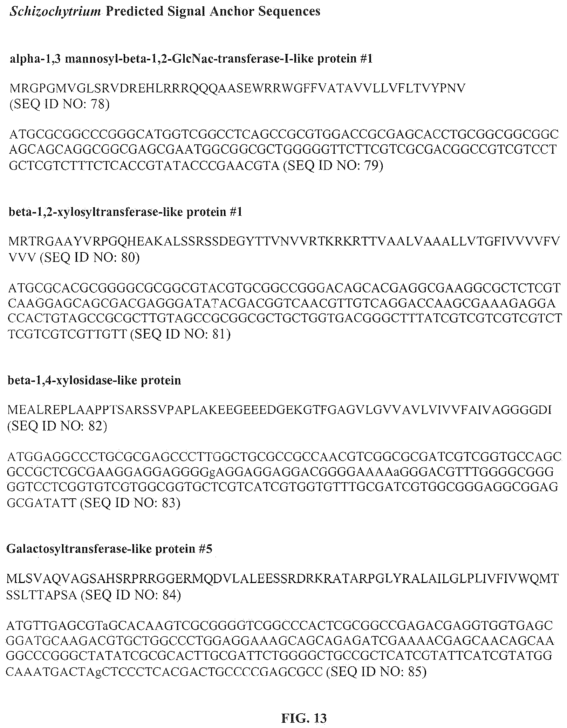

In the present invention, the heterologous polypeptide comprises a membrane domain that comprises a transmembrane domain. The term "membrane domain" as used herein refers to any domain within a polypeptide that targets the polypeptide to a membrane and/or allows the polypeptide to maintain association with a membrane and includes, but is not limited to, a transmembrane domain (e.g., a single or multiple membrane spanning region), an integral monotopic domain, a signal anchor sequence, an ER signal sequence, an N-terminal or internal or C-terminal stop transfer signal, a glycosylphosphatidylinositol anchor, and combinations thereof. A membrane domain can be located at any position in the polypeptide, including the N-terminal, C-terminal, or middle of the polypeptide. A membrane domain can be associated with permanent or temporary attachment of a polypeptide to a membrane. In some instances, a membrane domain can be cleaved from a membrane protein. In some instances, the membrane domain is a signal anchor sequence. In some instances, the membrane domain is any of the signal anchor sequences shown in **FIG. 13**, or an anchor sequence derived therefrom. In some embodiments, the membrane domain is a viral signal anchor sequence.

In some embodiments, the heterologous polypeptide is a polypeptide that naturally comprises a membrane domain. In some embodiments, the heterologous polypeptide does not naturally comprise a membrane domain but has been recombinantly fused to a membrane domain. In some embodiments, the heterologous polypeptide is an otherwise soluble protein that has been fused to a membrane domain.

In some embodiments, the membrane domain is a microalgal membrane domain. In some embodiments, the membrane domain is a Labyrinthulomycota membrane domain. In some embodiments, the membrane domain is a thraustochytrid membrane domain. In some embodiments, the membrane domain is a *Schizochytrium* or *Thraustochytrium* membrane domain. In some embodiments, the membrane domain comprises a signal anchor sequence from *Schizochytrium* alpha-1,3-mannosyl-beta-1,2-GlcNac-transferase-I-like protein #1 (SEQ ID NO:78), *Schizochytrium* beta-1,2-xylosyltransferase-like protein #1 (SEQ ID NO:80), *Schizochytrium* beta-1,4-xylosidase-like protein (SEQ ID NO:82), or *Schizochytrium* galactosyltransferase-like protein #5 (SEQ ID NO:84).

In the present invention, the heterologous polypeptide is a membrane protein. The term "membrane protein" as used herein refers to any protein associated with or bound to a cellular membrane. As described by Chou and Elrod, Proteins: Structure, Function and Genetics 34:137-153 (1999), for example, membrane proteins can be classified into various general types:
1) Type 1 membrane proteins: These proteins have a single transmembrane domain in the mature protein. The N-terminus is extracellular, and the C-terminus is cytoplasmic. The N-terminal end of the proteins characteristically has a classic signal peptide sequence that directs the protein for import to the ER. The proteins are subdivided into Type Ia (containing a cleavable signal sequence) and Type Ib (without a cleavable signal sequence). Examples of Type I membrane proteins include, but are not limited to: Influenza HA, insulin receptor, glycophorin, LDL receptor, and viral G proteins.
2) Type II membrane proteins: For these single membrane domain proteins, the C-terminus is extracellular, and the N-terminus is cytoplasmic. The N-terminus can have a signal anchor sequence. Examples of this protein type include, but are not limited to: Influenza Neuraminidase, Golgi galactosyltransferase, Golgi sialyltransferase, Sucrase-isomaltase precursor, Asialoglycoprotein receptor, and Transferrin receptor.
3) Multipass transmembrane proteins: In Type I and II membrane proteins the polypeptide crosses the lipid bilayer once, whereas in multipass membrane proteins the polypeptide crosses the membrane multiple times. Multipass transmembrane proteins are also subdivided into Types IIIa and IIIb. Type IIIa proteins have cleavable signal sequences. Type IIIb proteins have their amino termini exposed on the exterior surface of the membrane, but do not have a cleavable signal sequence. Type IIIa proteins include, but are not limited to, the M and L peptides of the photoreaction center. Type IIIb proteins include, but are not limited to, cytochrome P450 and leader peptidase of *E. coli.* Additional examples of multipass transmembrane proteins are membrane transporters, such as sugar transporters (glucose, xylose), and ion transporters.
4) Lipid chain anchored membrane proteins: These proteins are associated with the membrane bilayer by means of one or more covalently attached fatty acid chains or other types of lipid chains called prenyl groups.
5) GPI-anchored membrane proteins: These proteins are bound to the membrane by a glycosylphosphatidylinositol (GPI) anchor.
6) Peripheral membrane proteins: These proteins are bound to the membrane indirectly by noncovalent interactions with other membrane proteins.

In some embodiments, the membrane domain is the membrane domain of a HA protein.

In some embodiments, the heterologous membrane polypeptide comprises a native signal anchor sequence or a native membrane domain from a wild-type polypeptide corresponding to the heterologous polypeptide. In some embodiments, the heterologous membrane polypeptide is fused to a heterologous signal anchor sequence or a heterologous membrane domain that is different from the native signal anchor sequence or native membrane domain. In some embodiments, the heterologous membrane polypeptide comprises a heterologous signal anchor sequence or a heterologous membrane domain, while a wild-type polypeptide corresponding to the heterologous polypeptide does not comprise any signal anchor sequence or membrane domain. In some embodiments, the heterologous membrane polypeptide comprises a *Schizochytrium* signal anchor sequence. In some embodiments, the heterologous membrane polypeptide comprises a HA membrane domain. In some embodiments, the heterologous membrane polypeptide is a therapeutic polypeptide.

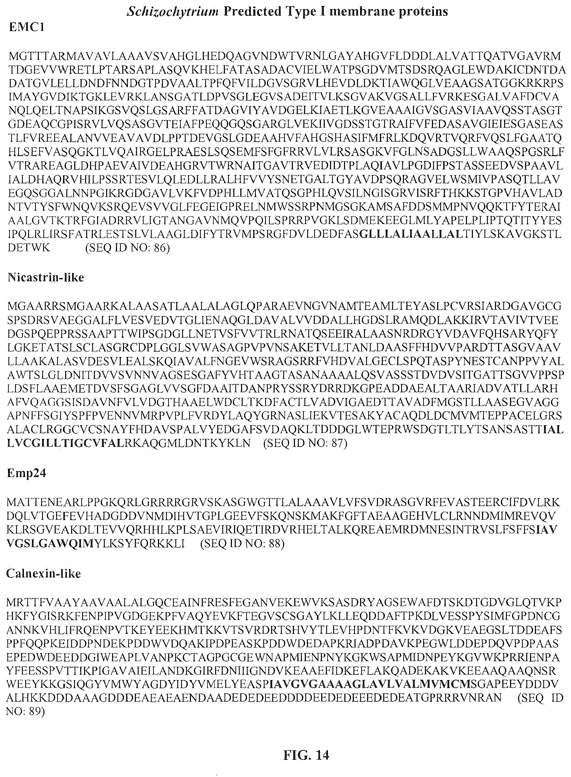

In some embodiments, the membrane domain is a membrane domain from any of the Type I membrane proteins shown in **FIG. 14**, or a membrane domain derived therefrom. In some embodiments, a heterologous polypeptide is a fusion polypeptide comprising the membrane spanning region in the C-terminus of any of the membrane proteins shown in **FIG. 14**. In some embodiments, the C-terminus side of the membrane spanning region is further modified by replacement with a similar region from a viral protein.

In some embodiments, the heterologous membrane polypeptide is a glycoprotein. In some embodiments, the heterologous membrane polypeptide has a glycosylation pattern characteristic of expression in a Labyrinthulomycota cell. In some embodiments, the heterologous membrane polypeptide has a glycosylation pattern characteristic of expression in a thraustochytrid cell. In some embodiments, a heterologous membrane polypeptide expressed in the microalgal host cell is a glycoprotein having a glycosylation pattern that more closely resembles mammalian glycosylation patterns than proteins produced in yeast or *E. coli.* In some embodiments, the glycosylation pattern comprises a N-linked glycosylation pattern. In some embodiments, the glycoprotein comprises high-mannose oligosaccharides. In some embodiments, the glycoprotein is substantially free of sialic acid. The term "substantially free of sialic acid" as used herein means less than 10%, less than 9%, less than 8%, less than 7%, less than 6%, less than 5%, less than 4%, less than 3%, less than 2%, or less than 1% of sialic acid. In some embodiments, sialic acid is absent from the glycoprotein.

In some embodiments, a microalgal extracellular body of the invention comprising a heterologous membrane polypeptide is produced at commercial or industrial scale.

The present invention is also directed to a composition comprising any of the microalgal extracellular bodies of the invention and an aqueous liquid carrier.

In some embodiments, a microalgal extracellular body of the invention comprising a heterologous polypeptide is recovered from the culture medium or fermentation medium in which the microalgal host cell is grown. In some embodiments, a microalgal extracellular body of the invention can be isolated in "substantially pure" form. As used herein, "substantially pure" refers to a purity that allows for the effective use of the microalgal extracellular body as a commercial or industrial product.

The present invention is also directed to a method of producing a microalgal extracellular body comprising a heterologous membrane polypeptide, the method comprising:
(a) expressing a heterologous membrane polypeptide in a microalgal host cell, wherein the heterologous membrane polypeptide comprises a transmembrane domain, and (b) culturing the host cell under culture conditions sufficient to produce a microalgal extracellular body comprising the heterologous membrane polypeptide, wherein the extracellular body is discontinuous with a plasma membrane of the host cell.

The present invention is also directed to a method of producing a composition comprising a microalgal extracellular body and a heterologous membrane polypeptide, the method comprising: (a) expressing a heterologous membrane polypeptide in a microalgal host cell, wherein the heterologous membrane polypeptide comprises a transmembrane domain, and (b) culturing the host cell under culture conditions sufficient to produce a microalgal extracellular body comprising the heterologous membrane polypeptide, wherein the extracellular body is discontinuous with a plasma membrane of the host cell, wherein the composition is produced as the culture supernatant comprising the extracellular body. In some embodiments, the method further comprises removing the culture supernatant and resuspending the extracellular body in an aqueous liquid carrier. In some embodiments, the composition is used as a vaccine.

### Microalgal Extracellular Bodies Comprising Viral Polypeptides

Virus envelope proteins are membrane proteins that form the outer layer of virus particles. The synthesis of these proteins utilizes membrane domains, such as cellular targeting signals, to direct the proteins to the plasma membrane. Envelope coat proteins fall into several major groups, which include but are not limited to: H or HA (hemagglutinin) proteins, N or NA (neuraminidase) proteins, F (fusion) proteins, G (glycoprotein) proteins, E or env (envelope) protein, gp120 (glycoprotein of 120 kDa), and gp41 (glycoprotein of 41 kDa). Structural proteins commonly referred to as "matrix" proteins serve to help stabilize the virus. Matrix proteins include, but are not limited to, M1, M2 (a membrane channel protein), and Gag. Both the envelope and matrix proteins can participate in the assembly and function of the virus. For example, the expression of virus envelope coat proteins alone or in conjunction with viral matrix proteins can result in the formation of virus-like particles (VLPs).

Viral vaccines are often made from inactivated or attenuated preparations of viral cultures corresponding to the disease they are intended to prevent, and generally retain viral material such as viral genetic material. Generally, a virus is cultured from the same or similar cell type as the virus might infect in the wild. Such cell culture is expensive and often difficult to scale. To address this problem, certain specific viral protein antigens are instead expressed by a transgenic host, which can be less costly to culture and more amenable to scale. However, viral proteins are typically integral membrane proteins present in the viral envelope. Since membrane proteins are very difficult to produce in large amounts, these viral proteins are usually modified to make a soluble form of the proteins. These viral envelope proteins are critical for establishing host immunity, but many attempts to express them in whole or part in heterologous systems have met with limited success, presumably because the protein must be presented to the immune system in the context of a viral envelope membrane in order to be sufficiently immunogenic. Thus, there is a need for new heterologous expression systems, such as those of the present invention, that are scalable and able to present viral antigens free or substantially free of associated viral material, such as viral genetic material, other than the desired viral antigens. The term "substantially free of associated viral material" as used herein means less than 10%, less than 9%, less than 8%, less than 7%, less than 5%, less than 4%, less than 3%, less than 2%, or less than 1% of associated viral material.

In some embodiments, a microalgal extracellular body comprises a heterologous polypeptide that is a viral glycoprotein selected from the group consisting of a H or HA (hemagglutinin) protein, a N or NA (neuraminidase) protein, a F (fusion) protein, a G (glycoprotein) protein, an E or env (envelope) protein, a gp120 (glycoprotein of 120 kDa), a gp41 (glycoprotein of 41 kDa), and combinations thereof. In some embodiments, the microalgal extracellular body comprises a heterologous polypeptide that is a viral matrix protein. In some embodiments, the microalgal extracellular body comprises a viral matrix protein selected from the group consisting of M1, M2 (a membrane channel protein), Gag, and combinations thereof. In some embodiments, the microalgal extracellular body comprises a combination of two or more viral proteins selected from the group consisting of a H or HA (hemagglutinin) protein, a N or NA (neuraminidase) protein, a F (fusion) protein, a G (glycoprotein) protein, an E or env (envelope) protein, a gp120 (glycoprotein of 120 kDa), a gp41 (glycoprotein of 41 kDa), and a viral matrix protein.

In some embodiments, the microalgal extracellular bodies of the present invention comprise viral glycoproteins lacking sialic acid that might otherwise interfere with protein accumulation or function.

In some embodiments, the microalgal extracellular body is a VLP.

The term "VLP" as used herein refers to particles that are morphologically similar to infectious virus that can be formed by spontaneous self-assembly of viral proteins when the viral proteins are over-expressed. VLPs have been produced in yeast, insect, and mammalian cells and appear to be an effective and safer type of subunit vaccine, because they mimic the overall structure of virus particles without containing infectious genetic material. This type of vaccine delivery system has been successful in stimulating the cellular and humoral responses.

Studies on Pararmyxoviruses have shown that when multiple viral proteins were co-expressed, the VLPs produced were very similar in size and density to authentic virions. Expression of the matrix protein (M) alone was necessary and sufficient for VLP formation. In Paramyxovirus, the expression of HN alone resulted in very low efficiency of VLP formation. Other proteins alone were not sufficient for NDV budding. HN is a type II membrane glycoprotein that exists on virion and infected-cell surfaces as a tetrameric spike. *See,* for example, Collins PL and Mottet G, J. Virol. 65:2362-2371 (1991); Mirza AM et al., J. Biol. Chem. 268: 21425-21431 (1993); and Ng D et al., J. Cell. Biol. 109: 3273-3289 (1989). Interactions with the M protein were responsible for incorporation of the proteins HN and NP into VLPs. *See,* for example, Pantua et. al., J. Virology 80:11062-11073 (2006).

Hepatitis B virus (HBV) or the human papillomavirus (HPV) VLPs are simple VLPs that are non-enveloped and that are produced by expressing one or two capsid proteins. More complex non-enveloped VLPs include particles such as VLPs developed for blue-tongue disease. In that case, four of the major structural proteins from the blue-tongue virus (BTV, *Reoviridae* family) were expressed simultaneously in insect cells. VLPs from viruses with lipid envelopes have also been produced (e.g., hepatitis C and influenza A). There are also VLP-like structures such as the self-assembling polypeptide nanoparticles (SAPN) that can repetitively display antigenic epitopes. These have been used to design a potential malaria vaccine. *See,* for example, Kaba SA et al., J. Immunol. 183 (11): 7268-7277 (2009).

VLPs have significant advantages in that they have the potential to generate immunity comparable to live attenuated or inactivated viruses, are believed to be highly immunogenic because of their particulate nature, and because they display surface epitopes in a dense repetitive array. For example, it has been hypothesized that B cells specifically recognize particulate antigens with epitope spacing of 50 Å to 100 Å as foreign. *See* Bachman et al., Science 262: 1448 (1993). VLPs also have a particle size that is believed to greatly facilitate uptake by dendritic cells and macrophages. In addition, particles of 20 nm to 200 nm diffuse freely to lymph nodes, while particles of 500 to 2000 nm do not. There are at least two approved VLP vaccines in humans, Hepatitis B Vaccine (HBV) and Human Papillomavirus (HPV). However, viral-based VLPs such as baculovirus-based VLPs often contain large amounts of viral material that require further purification from the VLPs.

In some embodiments, the microalgal extracellular body is a VLP comprising a viral glycoprotein selected from the group consisting of a H or HA (hemagglutinin) protein, a N or NA (neuraminidase) protein, a F (fusion) protein, a G (glycoprotein) protein, an E or env (envelope) protein, a gp120 (glycoprotein of 120 kDa), a gp41 (glycoprotein of 41 kDa), and combinations thereof. In some embodiments, the microalgal extracellular body is a VLP comprising a viral matrix protein. In some embodiments, the microalgal extracellular body is a VLP comprising a viral matrix protein selected from the group consisting of M1, M2 (a membrane channel protein), Gag, and combinations thereof. In some embodiments, the microalgal extracellular body is a VLP comprising a combination of two or more viral proteins selected from the group consisting of a H or HA (hemagglutinin) protein, a N or NA (neuraminidase) protein, a F (fusion) protein, a G (glycoprotein) protein, an E or env (envelope) protein, a gp120 (glycoprotein of 120 kDa), a gp41 (glycoprotein of 41 kDa), and a viral matrix protein.

### Methods of Using the Microalgal Extracellular Bodies

In some instances, a microalgal extracellular body of the invention is useful as a vehicle for a protein activity or function. In some instances, the protein activity or function is associated with a heterologous polypeptide present in or on the extracellular body. In some instances, the heterologous polypeptide is a membrane protein. In some instances, the protein activity or function is associated with a polypeptide that binds to a membrane protein present in the extracellular body. In some instances, the protein is not functional when soluble but is functional when part of an extracellular body of the invention. In some instances, a microalgal extracellular body containing a sugar transporter (such as, for example, a xylose, sucrose, or glucose transporter) can be used to deplete media containing mixes of sugars or other low molecular weight solutes, of trace amounts of a sugar by capturing the sugar within the vesicles that can then be separated by various methods including filtration or centrifugation.

The isolated microalgal extracellular bodies comprising a heterologous membrane protein comprising a transmembrane domain of the invention, and compositions thereof, can be used for therapeutic applications in animals or humans ranging from preventive treatments to disease.

The terms "treat" and "treatment" refer to both therapeutic treatment and prophylactic or preventative measures, wherein the object is to prevent or slow down (lessen) an undesired physiological condition, disease, or disorder, or to obtain beneficial or desired clinical results. For purposes of this invention, beneficial or desired clinical results include, but are not limited to, alleviation or elimination of the symptoms or signs associated with a condition, disease, or disorder; diminishment of the extent of a condition, disease, or disorder; stabilization of a condition, disease, or disorder, (i.e., where the condition, disease, or disorder is not worsening); delay in onset or progression of the condition, disease, or disorder; amelioration of the condition, disease, or disorder; remission (whether partial or total and whether detectable or undetectable) of the condition, disease, or disorder; or enhancement or improvement of a condition, disease, or disorder. Treatment includes eliciting a clinically significant response without excessive side effects. Treatment also includes prolonging survival as compared to expected survival if not receiving treatment.

In some embodiments, any of the microalgal extracellular bodies of the invention comprising a heterologous membrane polypeptide are recovered in the culture supernatant for direct use as animal or human vaccine.

In some embodiments, a microalgal extracellular body comprising a heterologous polypeptide is purified according to the requirements of the use of interest, e.g., administration as a vaccine. For a typical human vaccine application, the low speed supernatant would undergo an initial purification by concentration (e.g., tangential flow filtration followed by ultrafiltration), chromatographic separation (e.g., anion-exchange chromatography), size exclusion chromatography, and sterilization (e.g., 0.2 µm filtration). In some embodiments, a vaccine of the invention lacks potentially allergenic carry-over proteins such as, for example, egg protein. In some embodiments, a vaccine comprising an extracellular body of the invention lacks any viral material other than a viral polypeptide associated with the extracellular body.

According to the disclosed methods, a microalgal extracellular body comprising a heterologous polypeptide, or a composition thereof, can be administered, for example, by intramuscular (i.m.), intravenous (i.v.), subcutaneous (s.c.), or intrapulmonary routes. Other suitable routes of administration include, but are not limited to intratracheal, transdermal, intraocular, intranasal, inhalation, intracavity, intraductal (e.g., into the pancreas), and intraparenchymal (e.g., into any tissue) administration. Transdermal delivery includes, but is not limited to, intradermal (e.g., into the dermis or epidermis), transdermal (e.g., percutaneous), and transmucosal administration (e.g., into or through skin or mucosal tissue). Intracavity administration includes, but is not limited to, administration into oral, vaginal, rectal, nasal, peritoneal, and intestinal cavities, as well as, intrathecal (e.g., into spinal canal), intraventricular (e.g., into the brain ventricles or the heart ventricles), intraatrial (e.g., into the heart atrium), and subarachnoid (e.g., into the subarachnoid spaces of the brain) administration.

In some embodiments, the invention includes compositions comprising a microalgal extracellular body that comprises a heterologous membrane polypeptide. In some embodiments, the composition comprises an aqueous liquid carrier. In further embodiments, the aqueous liquid carrier is a culture supernatant. In some embodiments, the compositions of the invention include conventional pharmaceutically acceptable excipients known in the art such as, but not limited to, human serum albumin, ion exchangers, alumina, lecithin, buffer substances such as phosphates, glycine, sorbic acid, potassium sorbate, and salts or electrolytes such as protamine sulfate, as well as excipients listed in, for example, Remington: The Science and Practice of Pharmacy, 21st ed. (2005).

The most effective mode of administration and dosage regimen for the compositions of this invention depends upon the severity and course of the disease, the subject's health and response to treatment and the judgment of the treating physician. Accordingly, the dosages of the compositions should be titrated to the individual subject. Nevertheless, an effective dose of the compositions of this invention can be in the range of from 1 mg/kg to 2000 mg/kg, 1 mg/kg to 1500 mg/kg, 1 mg/kg to 1000 mg/kg, 1 mg/kg to 500 mg/kg, 1 mg/kg to 250 mg/kg, 1 mg/kg to 100 mg/kg, 1 mg/kg to 50 mg/kg, 1 mg/kg to 25 mg/kg, 1 mg/kg to 10 mg/kg, 500 mg/kg to 2000 mg/kg, 500 mg/kg to 1500 mg/kg, 500 mg/kg to 1000 mg/kg, 100 mg/kg to 2000 mg/kg, 100 mg/kg to 1500 mg/kg, 100 mg/kg to 1000 mg/kg, or 100 mg/kg to 500 mg/kg.

Having generally described this invention, a further understanding can be obtained by reference to the examples provided herein. These examples are for purposes of illustration only and are not intended to be limiting.

### EXAMPLE 1

### Construction of the pCL0143 expression vector

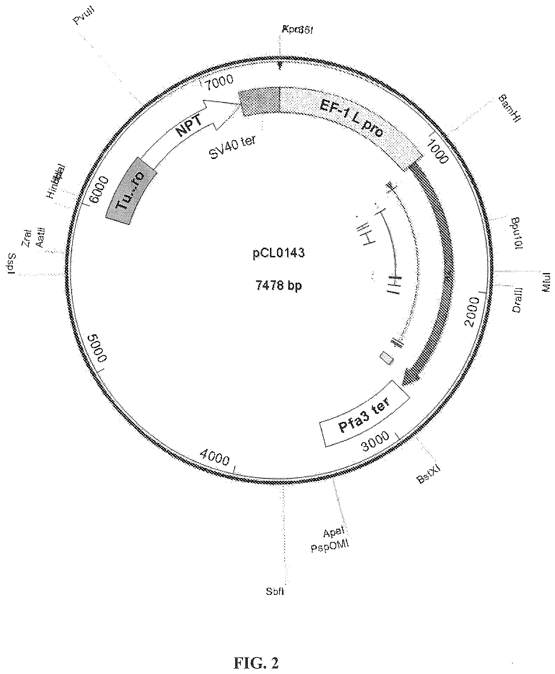

The pCL0143 expression vector **(****FIG. 2****)** was synthesized and the sequence was verified by Sanger sequencing by DNA 2.0 (Menlo Park, CA). The pCL0143 vector includes a promoter from the *Schizochytrium* elongation factor-1 gene (EF1) to drive expression of the HA transgene, the OrfC terminator (also known as the PFA3 terminator) following the HA transgene, and a selection marker cassette conferring resistance to the antibiotic paromomycin.

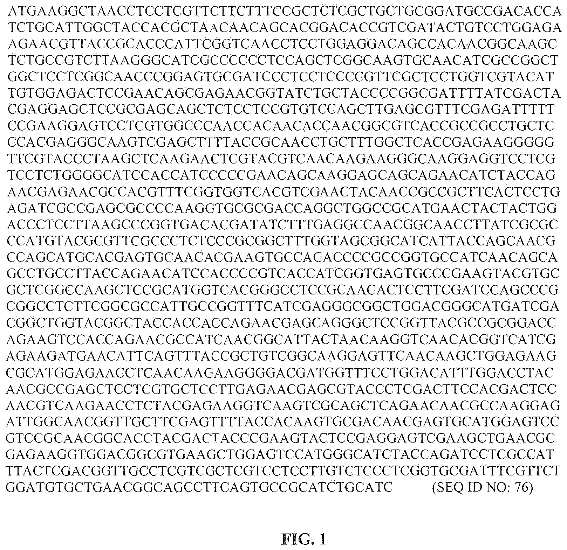

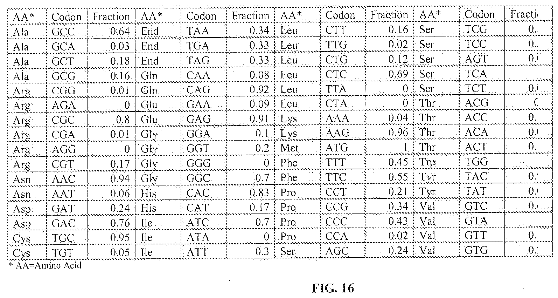

SEQ ID NO: 76 **(****FIG. 1****)** encodes the HA protein of Influenza A virus (A/Puerto Rico/8/34/Mount Sinai (H1N1)). The protein sequence matches that of GenBank Accession No. AAM75158. The specific nucleic acid sequence of SEQ ID NO: 76 was codon-optimized and synthesized for expression in *Schizochytrium* by DNA 2.0 as guided by the *Schizochytrium* codon usage table shown in **FIG. 16**. A construct was also produced using an alternative signal peptide in which the signal peptide of SEQ ID NO: 76 (first 51 nucleotides) was removed and replaced by the polynucleotide sequence encoding the *Schizochytrium* Sec1 signal peptide (SEQ ID NO: 38).

### EXAMPLE 2

### Expression and Characterization of HA Protein Produced in Schizochytrium

*Schizochytrium* sp. ATCC 20888 was used as a host cell for transformation with the vector pCL0143 with a Biolistic^{™} particle bombarder (BioRad, Hercules, CA). Briefly, cultures of *Schizochytrium* sp. ATCC number 20888 were grown in M2B medium consisting of 10 g/L glucose, 0.8 g/L (NH₄)₂SO₄, 5 g/L Na₂SO₄, 2 g/L MgSO₄●7H₂O, 0.5 g/L KH₂PO₄, 0.5 g/L KCl, 0.1 g/L CaCl₂●2H₂O, 0.1 M MES (pH 6.0), 0.1% PB26 metals, and 0.1% PB26 Vitamins (v/v). PB26 vitamins consisted of 50 mg/mL vitamin B12, 100 µg/mL thiamine, and 100 µg/mL Ca-pantothenate. PB26 metals were adjusted to pH 4.5 and consisted of 3 g/L FeSO₄•7H₂O, 1 g/L MnCl₂•4H₂O, 800 mg/mL ZnSO₄•7H₂O, 20 mg/mL CoCl₂•6H₂O, 10 mg/mL Na₂MoO₄•2H₂O, 600 mg/mL CuSO₄•5H₂O, and 800 mg/mL NiSO₄•6H₂O. PB26 stock solutions were filter-sterilized separately and added to the broth after autoclaving. Glucose, KH₂PO₄, and CaCl₂•2H₂O were each autoclaved separately from the remainder of the broth ingredients before mixing to prevent salt precipitation and carbohydrate caramelizing. All medium ingredients were purchased from Sigma Chemical (St. Louis, MO). Cultures of *Schizochytrium* were grown to log phase and transformed with a Biolistic^{™} particle bombarder (BioRad, Hercules, CA). The Biolistic^{™} transformation procedure was essentially the same as described previously (see Apt et al., J. Cell. Sci. 115(Pt 21):4061-9 (1996) and U.S. Patent No. 7,001,772). Primary transformants were selected on solid M2B media containing 20 g/L agar (VWR, West Chester, PA), 10 µg/mL Sulfometuron methyl (SMM) (Chem Service, Westchester, PA) after 2-6 days of incubation at 27°C.

gDNA from primary transformants of pCL0143 was extracted and purified and used as a template for PCR to check for the presence of the transgene.

*Genomic DNA Extraction Protocol for Schizochytrium* - The *Schizochytrium* transformants were grown in 50 ml of media. 25 ml of culture was aseptically pipetted into a 50 ml conical vial and centrifuge for 4 minutes at 3000 x g to form a pellet. The supernatant was removed and the pellet stored at -80°C until use. The pellet was resuspended in approximately 4-5 volumes of a solution consisting of 20 mM Tris pH 8, 10 mM EDTA, 50 mM NaCl, 0.5% SDS and 100 µg/ml of Proteinase K in a 50 ml conical vial. The pellet was incubated at 50°C with gentle rocking for 1 hour. Once lysed, 100 µg/ml of RNase A was added and the solution was rocked for 10 minutes at 37° C. Next, 2 volumes of phenol:chloroform:isoamyl alcohol was added and the solution was rocked at room temperature for 1 hour and then centrifuged at 8000 × g for 15 minutes. The supernatant was transferred into a clean tube. Again, 2 volumes of phenol: chloroform :isoamyl alcohol was added and the solution was rocked at room temperature for 1 hour and then centrifuged at 8000 × g for 15 minutes and the supernatant was transferred into a clean tube. An equal volume of chloroform was added to the resulting supernatant and the solution was rocked at room temperature for 30 minutes. The solution was centrifuged at 8000 × g for 15 minutes and the supernatant was transferred into a clean tube. An equal volume of chloroform was added to the resulting supernatant and the solution was rocked at room temperature for 30 minutes. The solution was centrifuged at 8000 × g for 15 minutes and the supernatant was transferred into a clean tube. 0.3 volumes of 3M NaOAc and 2 volumes of 100% EtOH were added to the supernatant, which was rocked gently for a few minutes. The DNA was spooled with a sterile glass rod and dipped into 70 % EtOH for 1-2 minutes. The DNA was transferred into a 1.7 ml microfuge tube and allowed to air dry for 10 minutes. Up to 0.5 ml of pre-warmed EB was added to the DNA and it was placed at 4° C overnight.

Cryostocks of transgenic *Schizochytrium* (transformed with pCL0143) were grown in M50-20 to confluence and then propagated in 50 mL baffled shake flasks at 27°C, 200 rpm for 48 hours (h), unless indcated otherwise, in a medium containing the following (per liter):

| | |
|---|---|
| Na₂SO₄ | 13.62g |
| K₂SO₄ | 0.72g |
| KCl | 0.56g |
| MgSO₄.7H₂O | 2.27g |
| (NH₄)2SO₄ | 3g |
| CaCl₂.2H₂O | 0.19g |
| MSG monohydrate | 3g |
| MES | 21.4g |
| KH₂PO₄ | 0.4g |

The volume was brought to 900 mL with deionized H₂O and the pH was adjusted to 6.5, unless indicated otherwise, before autoclaving for 35 min. Filter-sterilized glucose (50 g/L), vitamins (2 mL/L) and trace metals (2 mL/L) were then added to the medium and the volume was adjusted to one liter. The vitamin solution contained 0.16 g/L vitamin B12, 9.75 g/L thiamine, and 3.33 g/L Ca-pentothenate. The trace metal solution (pH 2.5) contained 1.00 g/L citric acid, 5.15 g/L FeSO₄.7H₂O, 1.55 g/L MnCl₂.4H₂O, 1.55 g/L ZnSO₄.7H₂O, 0.02 g/L CoCl₂.6H₂O, 0.02 g/L Na₂MoO₄.2H₂O, 1.035 g/L CuSO₄.5H₂O, and 1.035 g/L NiSO₄.6H₂O.

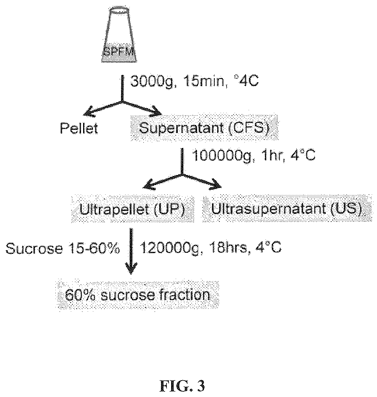

*Schizochytrium* cultures were transferred to 50 mL conical tubes and centrifugated at 3000 × g or 4500 × g for 15 min. *See* **FIG. 3****.** The supernatant resulting from this centrifugation, termed the "cell-free supernatant" (CFS), was used for a immunoblot analysis and a hemagglutination activity assay.

The cell-free supernatant (CFS) was further ultracentrifugated at 100,000 × g for 1 h. *See* **FIG. 3****.** The resulting pellet (insoluble fraction or "UP") containing the HA protein was resuspended in PBS, pH 7.4. This suspension was centrifuged (120,000 × g, 18 h, 4°C) on a discontinuous sucrose density gradient containing sucrose solutions from 15-60%. *See* **FIG. 3****.** The 60% sucrose fraction containing the HA protein was used for peptide sequence analysis, glycosylation analysis, as well as electron microscopy analysis.

### Immunoblot Analysis

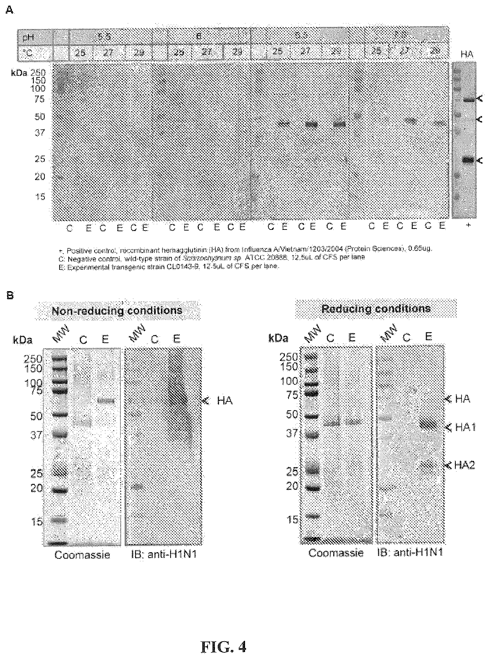

The expression of the recombinant HA protein from transgenic Schizochytrium CL0143-9 ("E") was verified by immunoblot analysis following standard immunoblotting procedure. The proteins from the cell-free supernatant (CFS) were separated by sodium dodecyl sulfate-polyacrylamide gel electrophoresis (SDS-PAGE) on a NuPAGE^{®} Novex^{®} 12% bis-tris gel (Invitrogen, Carlsbad, CA) under reducing conditions with MOPS SDS running buffer, unless indicated otherwise. The proteins were then stained with Coomassie blue (SimplyBlue Safe Stain, Invitrogen, Carlsbad, CA) or transferred onto polyvinylidene fluoride membrane and probed for the presence of HA protein with anti-Influenza A/Puerto Rico/8/34 (H1N1) virus antiserum from rabbit (1:1000 dilution, gift from Dr. Albert D.M.E. Osterhaus; Fouchier R.A.M. et al., J. Virol. 79: 2814-2822 (2005)) followed by anti-rabbit IgG (Fc) secondary antibody coupled to alkaline phosphatase (1:2000 dilution, #S3731, Promega Corporation, Madison, WI). The membrane was then treated with 5-bromo-4-chloro-3-indoyl-phosphate/nitroblue tetrazolium solution (BCIP/NBT) according to the manufacturer's instructions (KPL, Gaithersburg, MD). Anti-H1N1 immunoblots for the transgenic Schizochytrium CL0143-9 ("E") grown at various pH (5.5, 6.0, 6.5 and 7.0) and various temperatures (25°C, 27°C, 29°C) are shown in FIG. 4A. The negative control ("C") was the wild-type strain of Schizochytrium sp. ATCC 20888. The recombinant HA protein was detected in the cell-free supernatant at pH 6.5 (FIG. 4A) and hemagglutination activity detected was highest at pH 6.5, 27°C Coomassie blue-stained gels ("Coomassie") and corresponding anti-H1N1 immunoblots ("IB: anti-H1N1") for CL0143-9 ("E") grown at pH 6.5, 27°C, are shown in FIG. 4B under non-reducing and reducing conditions. The negative control ("C") was the wild-type strain of *Schizochytrium* sp. ATCC 20888.

### HA Activity

The activity of the HA protein produced in *Schizochytrium* was evaluated by a hemagglutination activity assay. The functional HA protein displays a hemagglutination activity that is readily detected by a standard hemagglutination activity assay. Briefly, 50 µL of doubling dilutions of low speed supernatant in PBS were prepared in a 96-well microtiter plate. Equal volume of an approximate 1% solution of chicken red blood cells (Fitzgerald Industries, Acton, MA) in PBS, pH 7.4, was then added to each well followed by incubation at room temperature for 30 min. The degree of agglutination was then analyzed visually. The hemagglutination activity unit (HAU) is defined as the highest dilution that causes visible hemagglutination in the well.

Typical activity was found to be in the order of 512 HAU in transgenic *Schizochytrium* CL0143-9 ("E") cell free supernatant (**FIG. 5A**). PBS ("-") or the wild-type strain of *Schizochytrium* sp. ATCC 20888 ("C"), grown and prepared in the same manner as the transgenic strains, were used as negative controls and did not show any hemagglutination activity. The recombinant HA protein from Influenza A/Vietnam/1203/2004 (H5N1) (Protein Sciences Corporation, Meriden, CT, dilution 1:1000 in PBS) was used as a positive control ("+").

Analysis of the soluble and insoluble fractions of the cell-free supernatant of the transgenic *Schizochytrium* CL0143-9 strain by hemagglutination assay a indicated that the HA protein is found predominantly in the insoluble fraction (**FIG. 5B**). Typical activity was found to be in the order of 16HAU in the soluble fraction ("US") and 256 HAU in the insoluble fraction ("UP").

Activity levels of HA protein in 2 L cultures demonstrated similar activity as in shake flask cultures when cultured in the same media at a constant pH of 6.5.

In a separate experiment, the native signal peptide of HA was removed and replaced by the *Schizochytrium* Sec1 signal peptide (SEQ ID NO: 37, encoded by SEQ ID NO: 38). Transgenic *Schizochytrium* obtained with this alternative construct displayed similar hemagglutin activity and recombinant protein distribution as observed with transgenic *Schizochytrium* containing the pCL0143 construct (data not shown).

### Peptide Sequence Analysis

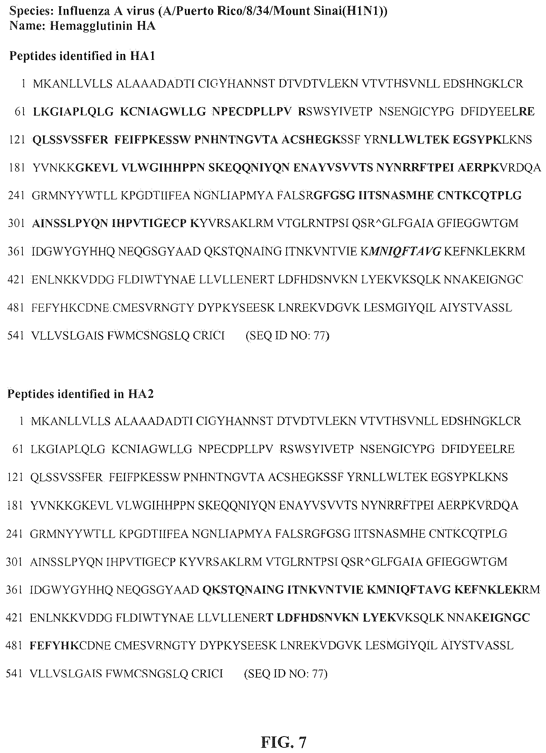

The insoluble fraction ("UP") resulting from 100,000 × g centrifugation of the cell-free supernatant was further fractionated on sucrose density gradient and the fractions containing the HA protein, as indicated by hemagglutination activity assay (FIG. 6B), was separated by SDS-PAGE and stained with Coomassie blue or transferred to PVDF and immunoblotted with anti-H1N1 antiserum from rabbit (FIG. 6A), as described above. The bands corresponding to the cross-reaction in immunoblot (HA1 and HA2) were excised from the Coomassie blue-stained gel and peptide sequence analysis was performed. Briefly, the bands of interest were washed/destained in 50% ethanol, 5% acetic acid. The gel pieces were then dehydrated in acetonitrile, dried in a SpeedVac^{®} (Thermo Fisher Scientific, Inc., Waltham, MA), and digested with trypsin by adding 5 µL of 10 ng/µL trypsin in 50 mM ammonium bicarbonate and incubating overnight at room temperature. The peptides that were formed were extracted from the polyacrylamide in two aliquots of 30 µL 50% acetonitrile with 5% formic acid. These extracts were combined and evaporated to <10 µL in a SpeedVac^{®} and then resuspended in 1% acetic acid to make up a final volume of approximately 30 µL for LC-MS analysis. The LC-MS system was a FinniganTM LTQTM Linear Ion Trap Mass Spectrometer (Thermo Electron Corporation, Waltham, MA). The HPLC column was a self-packed 9 cm × 75 µm Phenomenex JupiterTM C18 reversed-phase capillary chromatography column (Phenomenex, Torrance, CA). Then, µL volumes of the extract were injected and the peptides were eluted from the column by an acetonitrile/0.1% formic acid gradient at a flow rate of 0.25 µL/min and were introduced into the source of the mass spectrometer on-line. The microelectrospray ion source was operated at 2.5 kV. The digest was analyzed using a selective reaction (SRM) experiment in which the mass spectrometer fragments a series of m/z ratios over the entire course of the LC experiment. The fragmentation pattern of the peptides of interest was then used to produce chromatograms. The peak areas for each peptide was determined and normalized to an internal standard. The internal standards used in this analysis were proteins that have an unchanging abundance between the samples being studied. The final comparison between the two systems was determined by comparing the normalized peak ratios for each protein. The collision-induced dissociation spectra were then searched against the NCBI database. The HA protein was identified by a total of 27 peptides covering over 42% of the protein sequence. The specific peptides that were sequenced are highlighted in bold font in FIG. 7. More specifically, HA1 was identified by a total of 17 peptides and HA2 was identified by a total of 9 peptides. This is consistent with the HA N-terminal polypeptide being truncated prior to position 397. The placement of the identified peptides for HA1 and HA2 are shown within the entire amino acid sequence of the HA protein. The putative cleavage site within HA is located between amino acids 343 and 344 (shown as R^G). The italicized peptide sequence beginning at amino acid 402 is associated with the HA2 polypeptide but appeared in the peptides identified in HA1, likely due to trace carryover of HA2 peptides in the excised band for HA1. See, for example, Figure 3 of Wright et al., BMC Genomics 10:61 (2009).

### Glycosylation Analysis

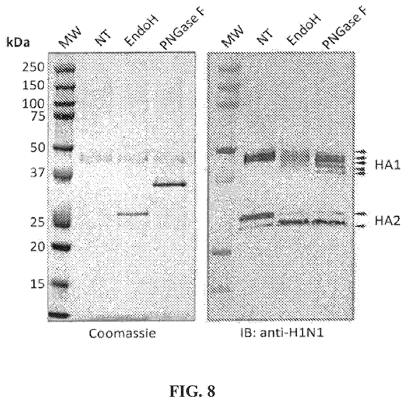

The presence of glycans on the HA protein was evaluated by enzymatic treatment. The 60% sucrose fraction of the transgenic *Schizochytrium* "CL0143-9" was digested with EndoH or PNGase F according to manufacturer's instructions (New England Biolabs, Ipswich, MA). Removal of glycans was then identified by the expected shift in mobility when separating the proteins by SDS-PAGE on NuPAGE^{®} Novex^{®} 12% bis-tris gels (Invitrogen, Carlsbad, CA) with MOPS SDS running buffer followed by staining with Coomassie blue ("Coomassie") or by immunoblotting with anti-H1N1 antiserum ("IB: anti-H1N1") (**FIG. 8**). The negative control for the enzymatic treatment was the transgenic *Schizochytrium* "CL0143-9" incubated without enzymes ("NT" = non-treated). At least five different species can be identified on the immunoblot at the level of HA1 and two different species can be identified on the immunoblot at the level of HA2. This is consistent with multiple glycosylation sites on HA1 and a single glycosylation site on HA2, as reported in the literature.

### EXAMPLE 3

### Characterization of Proteins from Schizochytrium Culture Supernatants

*Schizochytrium* sp. ATCC 20888 was grown under typical fermentation conditions as described above. Samples of culture supernatant were collected in 4 hour intervals from 20 h to 52 h of culture, with a final collection at 68 h.

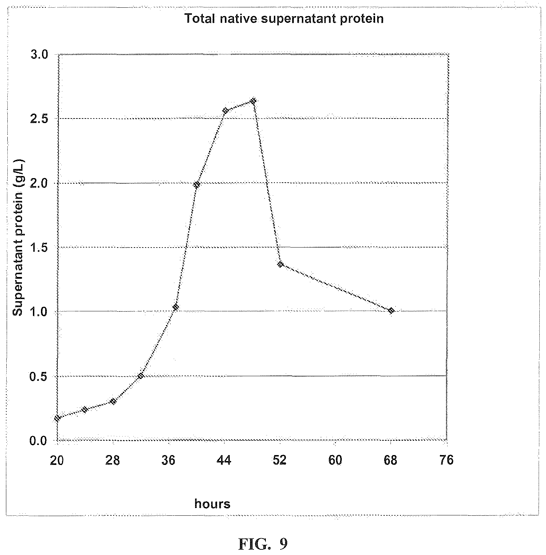

Total protein in the culture supernatant based on each sample was determined by a standard Bradford Assay. *See* **FIG. 9****.**

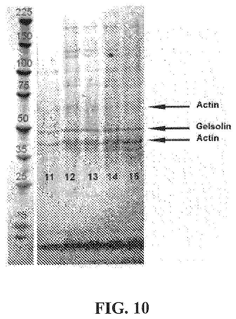

Proteins were isolated from the samples of culture supernatant at 37 h, 40 h, 44 h, 48 h, and 68 h using the method of **FIG. 3****.** A SDS-PAGE gel of the proteins is shown in **FIG. 10****.** Lane 11 was loaded with 2.4 µg of total protein, the remaining lanes were loaded with 5 µg total protein. Abundant bands identified as actin or gelsolin (by mass spectral peptide sequencing) are marked with arrows in **FIG. 10****.**

### EXAMPLE 4

### Negative-Staining and Electron Microscopy of Culture Supernatant Materials

*Schizochytrium* sp. ATCC 20888 (control) and transgenic *Schizochytrium* CL0143-9 (experimental) were grown under typical flask conditions as described above. Cultures were transferred to 50 mL conical tubes and centrifugated at 3000 × g or 4500 × g for 15 min. This cell-free supernatant was further ultracentrifugated at 100,000 × g for 1 h and the pellet obtained was resuspended in PBS, pH 7.4. This suspension was centrifuged on a discontinuous 15% to 60% sucrose gradient (120,000 × g, 18 h, 4°C), and the 60% fraction was used for negative-staining and examination by electron microscopy.

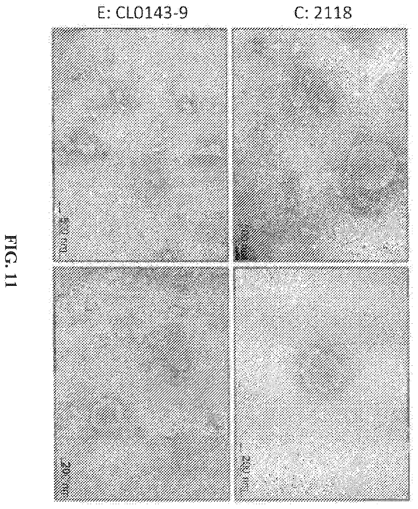

Electron microscope observations of control material negative-stained material contained a mixture of membrane fragments, membrane aggregates and vesicles (collectively "extracellular bodies") ranging from hundreds of nanometers in diameter to <50 nm. *See* **FIG. 11****.** Vesicle shape ranged from circular to elongated (tubular), and the margins of the vesicles were smooth or irregular. The interior of the vesicles appeared to stain lightly, suggesting that organic material was present. The larger vesicles had thickened membranes, suggesting that edges of the vesicles overlapped during preparation. Membrane aggregates and fragments were highly irregular in shape and size. The membrane material likely originated from the ectoplasmic net, as indicated by a strong correlation with actin in membranes purified by ultracentrifugation.

Similarly, electron microscope observations of negative-stained material from cell-free supernatants of culture of transgenic *Schizochytrium* CL0143-9 expressing heterologous protein indicated that the material was a mixture of membrane fragments, membrane aggregates and vesicles ranging from hundreds of nanometers in diameter to <50 nm. *See* **FIG. 11****.**

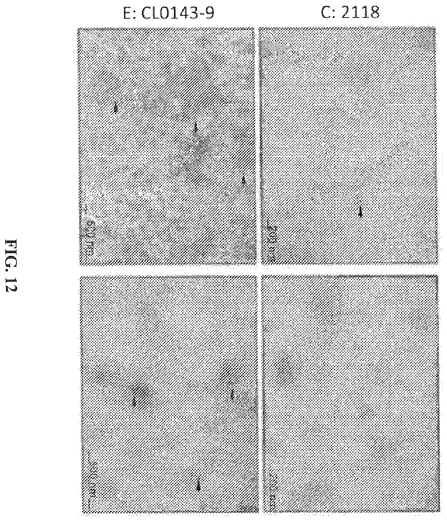

Immunolocalization was also conducted on this material as described in Perkins et al., J. Virol. 82:7201-7211 (2008), using the H1N1 antiserum described for the immunoblot analysis in Example 22 and 12 nm gold particles. Extracellular membrane bodies isolated from transgenic *Schizochytrium* CL0143-9 were highly decorated by gold particles attached to the antiserum (**FIG. 12**), indicating that the antibody recognized HA protein present in the extracellular bodies. Minimal background was observed in areas absent of membrane material. There were few or no gold particles bound to extracellular bodies isolated from control material (**FIG. 12**).

### EXAMPLE 5

### Construction of Xylose Transporter, Xylose Isomerase and Xylulose Kinase Expression Vectors

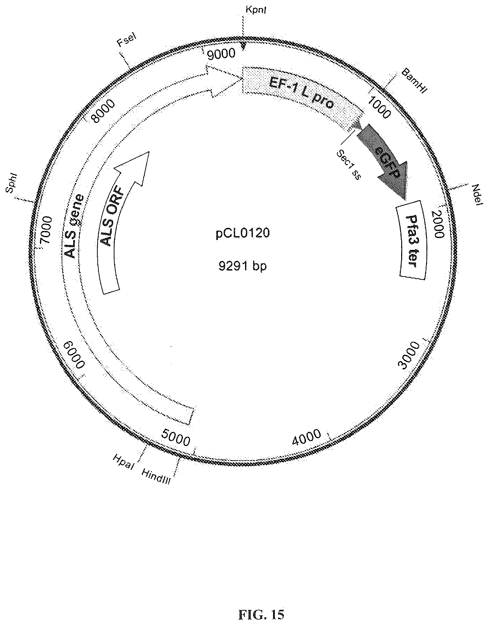

The vector pAB0018 (ATCC Accession No. PTA-9616) was digested with HindIII, treated with mung bean nuclease, purified, and then further digested with KpnI generating four fragments of various sizes. A fragment of 2552 bp was isolated by standard electrophoretic techniques in an agar gel and purified using commercial DNA purification kits. A second digest of pAB0018 with Pmel and Kpn was then performed. A fragment of 6732 bp was isolated and purified from this digest and ligated to the 2552 bp fragment. The ligation product was then used to transform commercially supplied strains of competent DH5-a *E. coli* cells (Invitrogen) using the manufacturer's protocol. Plasmids from ampicillin-resistant clones were propagated, purified, and then screened by restriction digests or PCR to confirm that the ligation generated the expected plasmid structures. One verified plasmid was designated pCL0120. *See* **FIG. 15****.**

Sequences encoding the *Candida intermedia* xylose transporter protein GXS1 (GenBank Accession No. AJ875406) and the *Arabidopsis thaliana* xylose transporter protein At5g17010 (GenBank Accession No. BT015128) were codon-optimized and synthesized (Blue Heron Biotechnology, Bothell, WA) as guided by the *Schizochytrium* codon usage table shown in **FIG. 16****.** SEQ ID NO: 94 is the codon-optimized nucleic acid sequence of GSX1, while SEQ ID NO: 95 is the codon-optimized nucleic acid sequence of At5g17010.

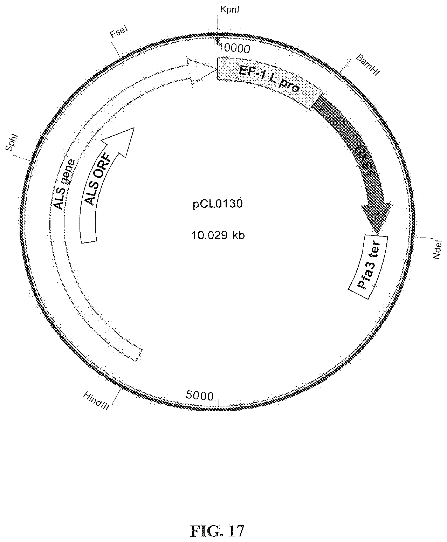

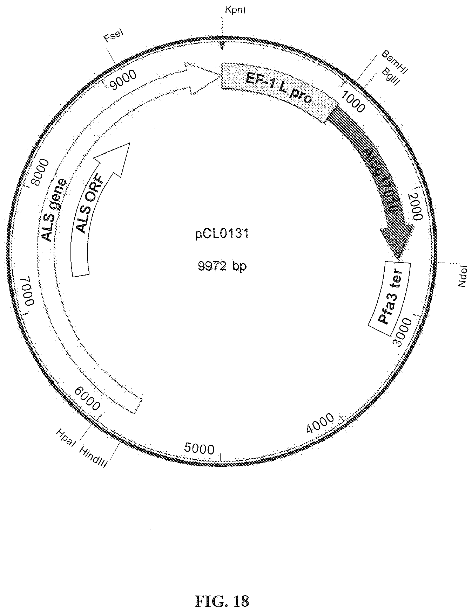

SEQ ID NO: 94 and SEQ ID NO: 95 were respectively cloned into pCL0120 using the 5' and 3' restriction sites BamHI and NdeI for insertion and ligation according to standard techniques. Maps of the resulting vectors, pCL0130 and pCL0131 are shown in **FIG. 17** and **FIG. 18****,** respectively.

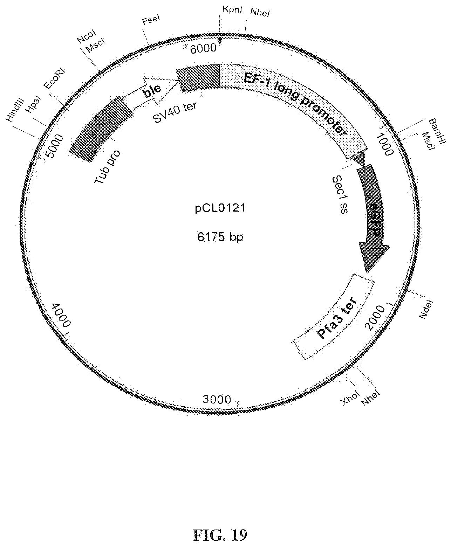

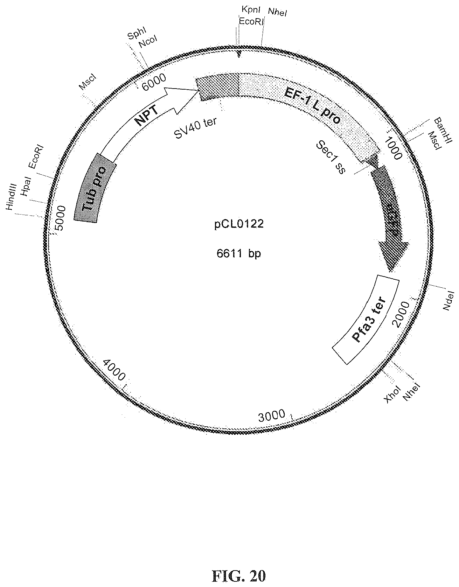

Vectors pCL0121 and pCL0122 were created by ligating a 5095 bp fragment which had been liberated from pCL0120 by digestion with HindIII and KpnI to synthetic selectable marker cassettes designed to confer resistance to either zeocin or paromomycin. These cassettes were comprised of an alpha tubulin promoter to drive expression of either the sh ble gene (for zeocin) or the npt gene (for paromomycin). The transcripts of both selectable marker genes were terminated by an SV40 terminator. The full sequence of vectors pCL0121 and pCL0122 are provided as SEQ ID NO: 90 and SEQ ID NO: 91, respectively. Maps of vectors pCL0121 and pCL0122 are shown in **FIGs. 19** and **20**, respectively.

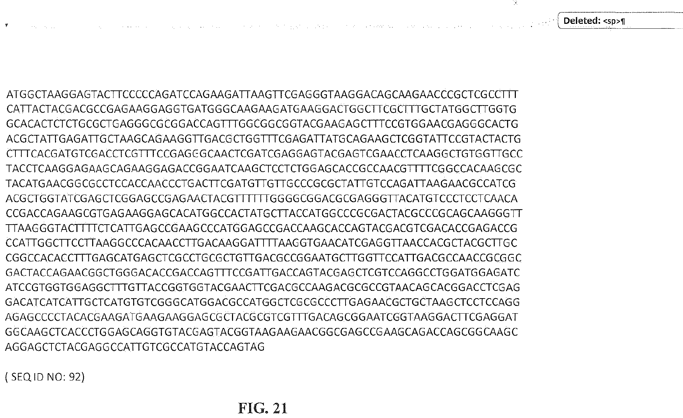

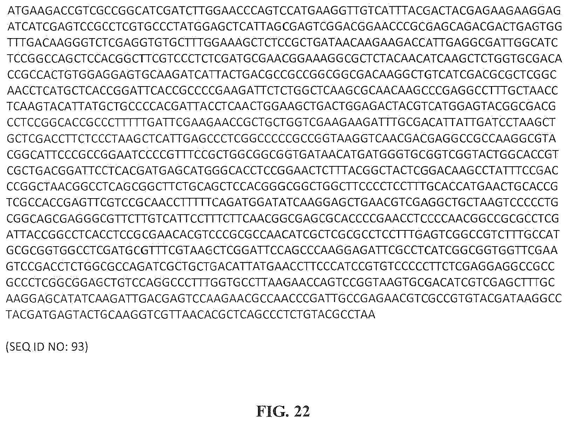

Sequences encoding the *Piromyces* sp. E2 xylose isomerase (CAB76571) and *Piromyces* sp. E2 xylulose kinase (AJ249910) were codon-optimized and synthesized (Blue Heron Biotechnology, Bothell, WA) as guided by the *Schizochytrium* codon usage table shown in **FIG. 16****.** "XylA" (SEQ ID NO: 92) is the codon-optimized nucleic acid sequence of CAB76571 (**FIG. 21**), while "XylB" (SEQ ID NO: 93) is the codon-optimized nucleic acid sequence of AJ249910 (**FIG. 22**).

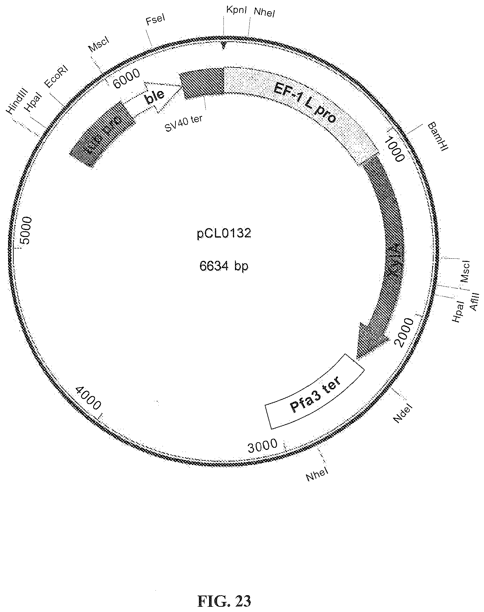

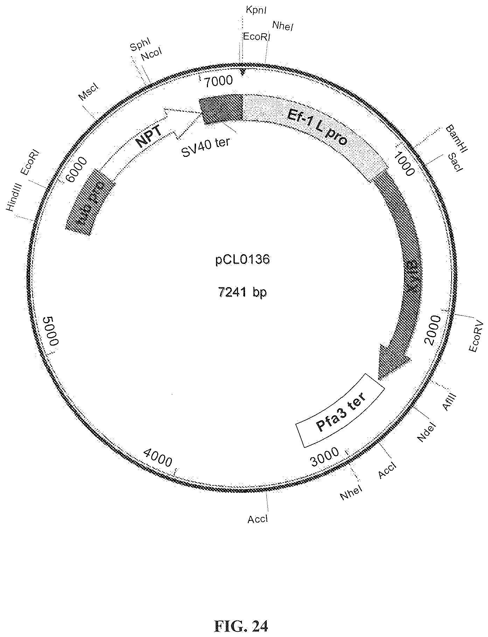

SEQ ID NO: 92 was cloned into the vector pCL0121 resulting in the vector designated pCL0132 (**FIG. 23**) and SEQ ID NO: 21 was cloned into the vector pCL0122 by insertion into the BamHI and NdeI sites, resulting in the vector designated pCL0136 (**FIG. 24**).

### EXAMPLE 6

### Expression and Characterization of Xylose Transporter, Xylose Isomerase and Xylulose Kinase Proteins Produced in Schizochytrium

*Schizochytrium* sp. ATCC 20888 was used as a host cell for transformation with vector pCL0130, pCL0131, pCL0132 or pCL0136 individually.

*Electroporation with enzyme pretreatment* - Cells were grown in 50 mL of M50-20 media *(see* U.S. Publ. No. 2008/0022422) on a shaker at 200 rpm for 2 days at 30°C. The cells were diluted at 1:100 into M2B media (see following paragraph) and grown overnight (16-24 h), attempting to reach mid-log phase growth (OD₆₀₀ of 1.5-2.5). The cells were centrifuged in a 50 mL conical tube for 5 min at 3000 × g. The supernatant was removed and the cells were resuspended in 1 M mannitol, pH 5.5, in a suitable volume to reach a final concentration of 2 OD₆₀₀ units. 5 mL of cells were aliquoted into a 25 mL shaker flask and amended with 10 mM CaCl₂ (1.0 M stock, filter sterilized) and 0.25 mg/mL Protease XIV (10 mg/mL stock, filter sterilized; Sigma-Aldrich, St. Louis, MO). Flasks were incubated on a shaker at 30°C and 100 rpm for 4 h. Cells were monitored under the microscope to determine the degree of protoplasting, with single cells desired. The cells were centrifuged for 5 min at 2500 × g in round-bottom tubes (i.e., 14 mL Falcon^{™} tubes, BD Biosciences, San Jose, CA). The supernatant was removed and the cells were gently resuspended with 5 mL of ice cold 10% glycerol. The cells were re-centrifuged for 5 min at 2500 × g in round-bottom tubes. The supernatant was removed and the cells were gently resuspended with 500 µL of ice cold 10% glycerol, using wide-bore pipette tips. 90 µL of cells were aliquoted into a prechilled electro-cuvette (Gene Pulser^{®} cuvette - 0.2 cm gap, Bio-Rad, Hercules, CA). 1 µg to 5 µg of DNA (in less than or equal to a 10 µL volume) was added to the cuvette, mixed gently with a pipette tip, and placed on ice for 5 min. Cells were electroporated at 200 ohms (resistance), 25 µF (capacitance), and 500V. 0. 5 mL of M50-20 media was added immediately to the cuvette. The cells were then transferred to 4.5 mL of M50-20 media in a 25 mL shaker flask and incubated for 2-3 h at 30°C and 100 rpm on a shaker. The cells were centrifuged for 5 min at 2500 × g in round bottom tubes. The supernatant was removed and the cell pellet was resuspended in 0.5 mL of M50-20 media. Cells were plated onto an appropriate number (2 to 5) of M2B plates with appropriate selection (if needed) and incubated at 30°C.

M2B media consisted of 10 g/L glucose, 0.8 g/L (NH4)2SO4, 5 g/L Na2SO4, 2 g/L MgSO4•7H2O, 0.5 g/L KH2PO4, 0.5 g/L KCl, 0.1 g/L CaCl2•2H2O, 0.1 M MES (pH 6.0), 0.1% PB26 metals, and 0.1% PB26 Vitamins (v/v). PB26 vitamins consisted of 50 mg/mL vitamin B12, 100 µg/mL thiamine, and 100 µg/mL Ca-pantothenate. PB26 metals were adjusted to pH 4.5 and consisted of 3 g/L FeSO4•7H2O, 1 g/L MnCl2•4H2O, 800 mg/mL ZnSO4•7H2O, 20 mg/mL CoCl2•6H2O, 10 mg/mL Na2MoO4•2H2O, 600 mg/mL CuSO4•5H2O, and 800 mg/mL NiSO4•6H2O. PB26 stock solutions were filter-sterilized separately and added to the broth after autoclaving. Glucose, KH2PO4, and CaCl2•2H2O were each autoclaved separately from the remainder of the broth ingredients before mixing to prevent salt precipitation and carbohydrate caramelizing. All medium ingredients were purchased from Sigma Chemical (St. Louis, MO).

The transformants were selected for growth on solid media containing the appropriate antibiotic. Between 20 and 100 primary transformants of each vector were re-plated to "xylose-SSFM" solid media which is the same as SSFM (described below) except that it contains xylose instead of glucose as a sole carbon source, and no antibiotics were added. No growth was observed for any clones under these conditions.

SSFM media: 50 g/L glucose, 13.6 g/L Na₂SO₄, 0.7 g/L K₂SO₄, 0.36 g/L KCl, 2.3 g/L MgSO₄•7H₂O, 0.1M MES (pH 6.0), 1.2 g/L (NH₄)₂SO₄, 0.13 g/L monosodium glutamate, 0.056 g/L KH₂PO₄, and 0.2 g/L CaCl₂•2H₂O. Vitamins were added at 1 mL/L from a stock consisting of 0.16 g/L vitamin B12, 9.7 g/L thiamine, and 3.3 g/L Ca-pantothenate. Trace metals were added at 2 mL/L from a stock consisting of 1 g/L citric acid, 5.2 g/L FeSO₄•7H₂O, 1 .5 g/L MnCl₂•₄H₂O, 1.5 g/L ZnSO₄•7H₂O, 0.02 g/L CaCl₂•6H₂O, 0.02 g/L Na₂MoO₄•2H₂O, 1.0 g/L CuSO₄•5H₂O, and 1.0 g/L NiSO₄•6H₂O, adjusted to pH 2.5.

gDNA from primary transformants of pCL0130 and pCL0131 was extracted and purified and used as a template for PCR to check for the presence of the transgene.

Genomic DNA Extraction was performed as described in Example 2.

Alternatively, after the RNase A incubation, the DNA was further purified using a Qiagen Genomic tip 500/G column (Qiagen, Inc USA, Valencia, CA), following the manufacturers protocol.

*PCR* - The primers used for detecting the GXS1 transgene were 5'CL0130 (CCTCGGGCGGCGTCCTCTT) (SEQ ID NO: 96) and 3'CL0130 (GGCGGCCTTCTCCTGGTTGC) (SEQ ID NO: 97). The primers used for detecting the At5g17010 transgene were 5'CL0131 (CTACTCCGTTGTTGCCGCCATCCT) (SEQ ID NO: 98) and 3'CL0131 (CCGCCGACCATACCGAGAACGA) (SEQ ID NO: 99).

Combinations of pCL0130, pCL0132, and pCL0136 together (the "pCL01310 series") or pCL0131, pCL0132, and pCL0136 together (the "pCL0131 series") were used for co-transformations of *Schizochytrium* wild type strain (ATCC 20888). Transformants were plated directly on solid xylose SSFM media and after 3-5 weeks, colonies were picked and further propagated in liquid xylose-SSFM. Several rounds of serial transfers in xylose-containing liquid media improved growth rates of the transformants. Co-transformants of the pCL0130 series or the pCL0131 series were also plated to solid SSFM media containing either SMM, zeocin, or paromomycin. All transformants plated to these media were resistant to each antibiotic tested, indicating that transformants harbored all three of their respective vectors. The *Schizochytrium* transformed with a xylose transporter, a xylose isomerase and a xylulose kinase were able to grow in media containing xylose as a sole carbon source.

In a future experiment, Western blots of both cell-free extract and cell-free supernatant from shake flask cultures of selected SMM-resistant transformant clones (pCL0130 or pCL0131 transformants alone, or the pCL0130 series co-transformants, or the pCL0131 series co-transformants) are performed and show that both transporters are expressed and found in both fractions, indicating that these membrane-bound proteins are associated with extracellular vesicles in a manner similar to that observed with other membrane proteins described herein. Additionally, Western blots are performed that show expression of the xylose isomerase and xylulose kinase in the cell-free extracts of all clones where their presence is expected. Extracellular bodies such as vesicles containing xylose transporters can be used to deplete media containing mixes of sugars or other low molecular weight solutes, of trace amounts of xylose by capturing the sugar within the vesicles that can then be separated by various methods including filtration or centrifugation.

### EXAMPLE 7

### Construction of the pCL0140 and pCL0149 expression vectors

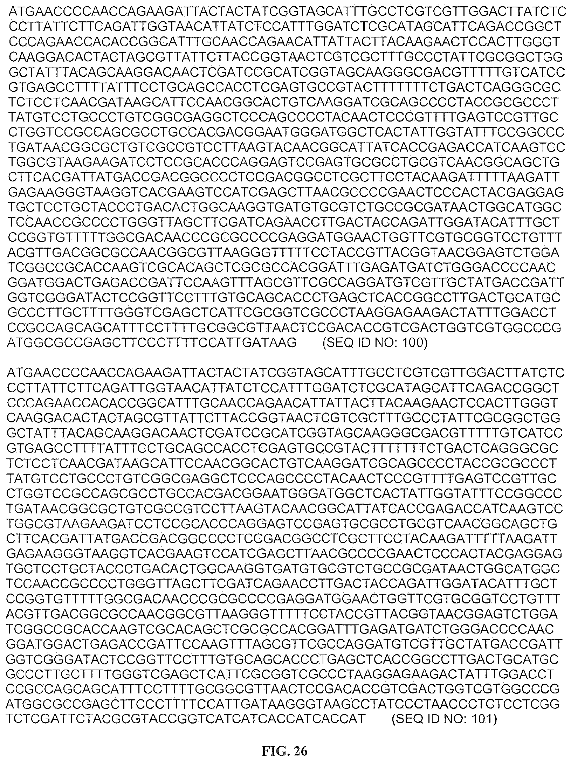

The vector pCL0120 was digested with BamHI and NdeI resulting in two fragments of 837 base pairs (bp) and 8454 bp in length. The 8454 bp fragment was fractionated by standard electrophoretic techniques in an agar gel, purified using commercial DNA purification kits, and ligated to a synthetic sequence (SEQ ID NO: 100 or SEQ ID NO: 101; *see* **FIG. 26**) that had also been previously digested with BamHI and NdeI. SEQ ID NO: 100 (**FIG. 26**) encodes the NA protein of Influenza A virus (A/Puerto Rico/8/34/Mount Sinai(H1N1)). The protein sequence matches that of GenBank Accession No. NP_040981. The specific nucleic acid sequence of SEQ ID NO: 100 was codon-optimized and synthesized for expression in *Schizochytrium* by DNA 2.0 as guided by the *Schizochytrium* codon usage table shown in **FIG. 16****.** SEQ ID NO: 101 (**FIG. 26**) encodes the same NA protein as SEQ ID NO: 100, but includes a V5 tag sequence as well as a polyhistidine sequence at the C-terminal end of the coding region.

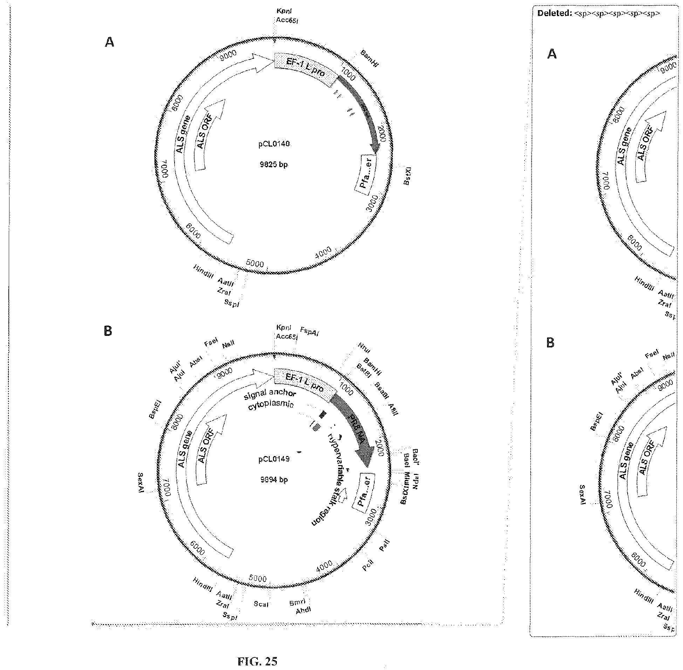

The ligation product was then used to transform commercially supplied strains of competent DH5-a *E*. *coli* cells (Invitrogen, Carlsbad, CA) using the manufacturer's protocol. These plasmids were then screened by restriction digests or PCR to confirm that the ligation generated the expected plasmid structures. Plasmid vectors resulting from the procedure were verified using Sanger sequencing by DNA 2.0 (Menlo Park, CA) and designated pCL0140 (**FIG. 25A**), containing SEQ ID NO: 100, and pCL0149 (**FIG. 25B**), containing SEQ ID NO: 101. The pCL0140 and pCL0149 vectors include a promoter from the *Schizochytrium* elongation factor-1 gene (EF1) to drive expression of the NA transgene, the OrfC terminator (also known as the PFA3 terminator) following the NA transgene, and a selection marker cassette conferring resistance to sulfometuron methyl.

### EXAMPLE 8

### Expression and Characterization of NA Protein Produced in Schizochytrium

*Schizochytrium* sp. ATCC 20888 was used as a host cell for transformation with the vectors pCL0140 and pCL0149 with a Biolistic^{™} particle bombarder (BioRad, Hercules, CA), as described in Example 2. The transformants were selected for growth on solid media containing the appropriate antibiotic. gDNA from primary transformants was extracted and purified and used as a template for PCR to check for the presence of the transgene, as described earlier (Example 2).

Cryostocks of transgenic *Schizochytrium* (transformed with pCL0140 and pCL0149) were grown in M50-20 to confluence and then propagated in 50 mL baffled shake flasks as described in Example 2.

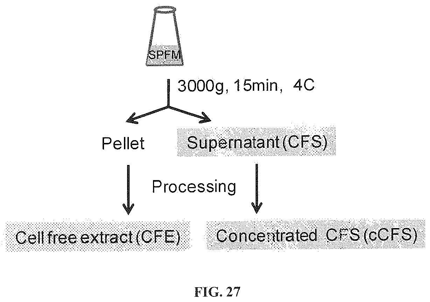

*Schizochytrium* cultures were transferred to 50 mL conical tubes and centrifugated at 3000 × g for 15 min. *See* **FIG. 27****.** The supernatant resulting from this centrifugation, was termed the "cell-free supernatant" (CFS). The CFS fraction was concentrated 50-100 fold using Centriprep^{™} gravity concentrators (Millipore, Billerica, MA) and termed the "concentrated cell-free supernatant" (cCFS). The cell pellet resulting from the centrifugation was washed in water and frozen in liquid nitrogen before being resuspended in twice the pellet weight of lysis buffer (consisting of 50 mM sodium phosphate (pH 7.4), 1 mM EDTA, 5% glycerol, and 1 mM fresh phenylmethylsulphonylfluoride) and twice the pellet weight of 0.5 mm glass beads (Sigma, St. Louis, MO)). The cell pellet mixture was then lysed by vortexing at 4°C in a multi-tube vortexer (VWR, Westchester, PA) at maximum speed for 3 hours. The resulting cell lysate was then centrifuged at 5500 × g for 10 minutes at 4°C. The resulting supernatant was retained and re-centrifuged at 5500 × g for 10 minutes at 4°C. The resulting supernatant is defined herein as "cell-free extract" (CFE). Protein concentration was determined in cCFS and CFE by a standard Bradford assay (Bio-Rad, Hercules, CA). These fractions were used for neuramidase activity assays as well as immunoblot analysis.

A functional influenza NA protein displays neuraminidase activity that can be detected by a standard fluorometric NA activity assay based on the hydrolysis of a sodium (4-Methylumbelliferyl)-α-D-*N*-Acetylneuraminate (4-MUNANA) substrate (Sigma-Aldrich, St. Louis, MO) by sialidases to give free 4-methylumbelliferone which has a fluorescence emission at 450 nm following an excitation at 365 nm. Briefly, the CFS, cCFS or CFE of transgenic *Schizochytrium* strains were assayed following the procedure described by Potier et al., Anal. Biochem. 94: 287-296 (1979), using 25 µL of CFS and 75 µL of 40 µM 4-MUNANA or 75 µL ddH2O for controls. Reactions were incubated for 30 minutes at 37°C and fluorescence was measured with a FLUOstar Omega multimode microplate reader (BMG LABTECH, Offenburg, Germany).

Typical activities observed in concentrated cell-free supernatants (cCFSs) and cell-free extracts (CFEs) from 9 transgenic strains of *Schizochytrium* transformed with CL0140 are presented in **FIG. 28****.** The wild-type strain of *Schizochytrium* sp. ATCC 20888 ("-") and a PCR-negative strain of *Schizochytrium* transformed with pCL0140 ("27"), grown and prepared in the same manner as the transgenic strains, were used as negative controls. The majority of the activity was found in the concentrated cell-free supernatant, indicating the successful expression and secretion of a functional influenza neuraminidase to the outer milieu by *Schizochytrium.*

### Peptide Sequence Analysis

Transgenic *Schizochytrium* strain CL0140-26 was used for partial purification of the influenza NA protein to confirm its successful expression and secretion by peptide sequence analysis. The purification procedure was adapted from Tarigan et al., JITV 14(1): 75-82 (2008), and followed by measuring the NA activity (**FIG. 29A**), as described above. Briefly, the cell-free supernatant of the transgenic strain CL0140-26 was further centrifugated at 100,000 × g for 1 hour at 4°C. The resulting supernatant was concentrated 100 fold (fraction "cCFS" in **FIG. 29A**) using Centriprep^{™} gravity concentrators (Millipore, Billerica, MA) and diluted back to the original volume (fraction "D" in **FIG. 29A**) with 0.1M sodium bicarbonate buffer (pH 9.1) containing 0.1% Triton X-100. This diluted sample was used for purification by affinity chromatography. N-(p-aminophenyl) oxamic acid agarose (Sigma-Aldrich, St. Louis, MO) was packed into a PD-10 column (BioRad, Hercules, CA), activated by washing with 6 column volumes (CV) of 0.1 M sodium bicarbonate buffer (pH 9.1) containing 0.1% Triton X-100 followed by 5 CV of 0.05 M sodium acetate buffer pH 5.5 containing 0.1% Triton X-100. The diluted sample (fraction "D") was loaded into the column; unbound materials were removed by washing the column with 10 CV of 0.15 M sodium acetate buffer containing 0.1% Triton X-100 (fraction "W" in **FIG. 29A**). Bound NA was eluted from the column with 5CV of 0.1 M sodium bicarbonate buffer containing 0.1% Triton X-100 and 2 mM CaCl2 (fraction "E" in **FIG. 29A**). The NA-rich solution of fraction E was concentrated to about 10% original volume using a 10-kDa-molecular-cut-off-spin concentrator to produce fraction cE.

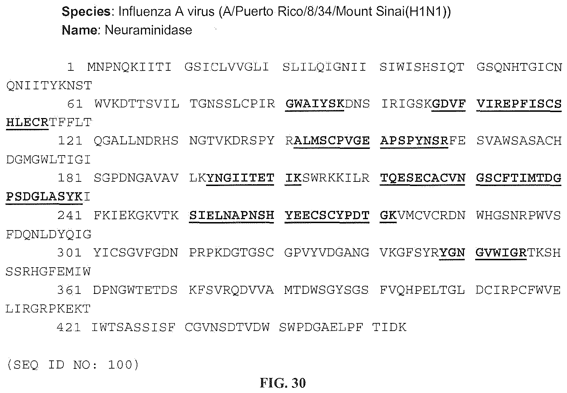

The proteins from each fraction were separated by sodium dodecyl sulfate-polyacrylamide gel electrophoresis (SDS-PAGE) on a NuPAGE^{®} Novex^{®} 12% bis-tris gel (Invitrogen, Carlsbad, CA) under reducing conditions with MOPS SDS running buffer. The proteins were then stained with Coomassie blue (SimplyBlue Safe Stain, Invitrogen, Carlsbad, CA). The proteins bands visible in lane "cE" (**FIG. 29B**) were excised from the Coomassie blue-stained gel and peptide sequence analysis was performed as described in Example 2. The protein band containing NA protein (indicated by the arrow in lane "cE") was identified by a total of 9 peptides (113 amino acids) covering 25% of the protein sequence. The specific peptides that were sequenced are highlighted in bold font in **FIG. 30****.**

### Immunoblot Analysis

The expression of the recombinant NA protein from transgenic *Schizochytrium* CL0149 (clones 10, 11, 12) was tested by immunoblot analysis following standard immunoblotting procedure (**FIG. 31B**)**.** The proteins from the cell-free supernatant (CFS) were separated by sodium dodecyl sulfate-polyacrylamide gel electrophoresis (SDS-PAGE) on a NuPAGE^{®} Novex^{®} 12% bis-tris gel (Invitrogen, Carlsbad, CA) under reducing conditions with MOPS SDS running buffer. The proteins were then stained with Coomassie blue (SimplyBlue Safe Stain, Invitrogen, Carlsbad, CA) or transferred onto polyvinylidene fluoride membrane and probed for the presence of NA protein with anti-V5-AP conjugated mouse monoclonal antibody (1:1000 dilution, #962-25, Invitrogen, Carlsbad, CA). The membrane was then treated with 5-bromo-4-chloro-3-indoyl-phosphate/nitroblue tetrazolium solution (BCIP/NBT) according to the manufacturer's instructions (KPL, Gaithersburg, MD). The recombinant NA protein was detected in the cell-free supernatant of clone 11 (**FIG. 31B**). The negative control ("-") was the wild-type strain of *Schizochytrium* sp. ATCC 20888. The positive control ("+") was the Positope^{™} antibody control protein (#R900-50, Invitrogen, Carlsbad, CA). The corresponding neuraminidase activity is presented in **FIG. 31A.**

### EXAMPLE 9

### Simultaneous expression of Influenza HA and NA in Schizochytrium

*Schizochytrium* sp. ATCC 20888 was used as a host cell for simultaneous transformation with the vectors pCL0140 (**FIG. 25A**) and pCL0143 (**FIG. 2**) with a Biolistic^{™} particle bombarder (BioRad, Hercules, CA), as described in Example 2.

Cryostocks of transgenic *Schizochytrium* (transformed with pCL0140 and pCL0143) were cultivated and processed as described in Example 2. The hemagglutination and neuraminidase activities were measured as described in Examples 2 and 7, respectively, and are shown in FIG. 32. Transgenic *Schizochytrium* transformed with pCL0140 and pCL0143 demonstrated activities associated with HA and NA.

### EXAMPLE 10

### Expression and Characterization of Extracellular Bodies Comprising Parainfluenza F Protein Produced in Schizochytrium

*Schizochytrium* sp. ATCC 20888 is used as a host cell for transformation with a vector comprising a sequence that encodes the F protein of human parainfluenza 3 virus strain NIH 47885, (GenBank Accession No. P06828). A representative sequence for the F protein is provided as SEQ ID NO: 102. Some cells are transformed with a vector comprising a sequence encoding the native signal peptide sequence associated with the F protein. Other cells are transformed with a vector comprising a sequence encoding a different signal peptide sequence (such as, for example, a *Schizochytrium* signal anchor sequence) that is fused to the sequence encoding the F protein, such that the F protein is expressed with a heterologous signal peptide sequence. Other cells are transformed with a vector comprising a sequence encoding a different membrane domain (such as, for example, a HA membrane domain) that is fused to the sequence encoding the F protein, such that the F protein is expressed with a heterologous membrane domain. The F protein comprises a single-pass transmembrane domain near the C-terminus. The F protein can be split into two peptides at the Furin cleavage site (amino acid 109). The first portion of the protein designated F2 contains the N-terminal portion of the complete F protein. The F2 region can be fused individually to sequences encoding heterologous signal peptides. The remainder of the viral F protein containing the C-terminal portion of the F protein is designated F1. The F1 region can be fused individually to sequences encoding heterologous signal peptides. Vectors containing the F1 and F2 portions of the viral F protein can be expressed individually or in combination. A vector expressing the complete F protein can be co-expressed with the furin enzyme that will cleave the protein at the furin cleavage site. Alternatively, the sequence encoding the furin cleavage site of the F protein can be replaced with a sequence encoding an alternate protease cleavage site that is recognized and cleaved by a different protease. The F protein containing an alternate protease cleavage site can be co-expressed with a corresponding protease that recognizes and cleaves the alternate protease cleavage site.

Transformation is performed, and cryostocks are grown and propagated according to any of the methods described herein. *Schizochytrium* cultures are transferred to 50 mL conical tubes and centrifugated at 3000 × g or 4500 × g for 15 min to yield a low-speed supernatant. The low-speed supernatant is further ultracentrifugated at 100,000 × g for 1 h. *See* FIG. 3. The resulting pellet of the insoluble fraction containing the F protein is resuspended in phosphate buffer saline (PBS) and used for peptide sequence analysis as well as glycosylation analysis as described in Example 2.

The expression of the F protein from transgenic *Schizochytrium* is verified by immunoblot analysis following standard immunoblotting procedure as described in Example 2, using anti-F antiserum and a secondary antibody at appropriate dilutions. The recombinant F protein is detected in the low-speed supernatant and the insoluble fraction. Additionally, the recombinant F protein is detected in cell-free extracts from transgenic *Schizochytrium* expressing the F protein.

The activity of the F protein produced in *Schizochytrium* is evaluated by a F activity assay. A functional F protein displays an F activity that is readily detected by a standard F activity assay.

Electron microscopy, using negative-stained material produced according to Example 4, is performed to confirm the presence of extracellular bodies. Immunogold labeling is performed to confirm the association of protein with extracellular membrane bodies.

### EXAMPLE 11

### Expression and Characterization of Extracellular Bodies Comprising G Vesicular Stomatitus Virus G Protein Produced in Schizochytrium

*Schizochytrium* sp. ATCC 20888 is used as a host cell for transformation with a vector comprising a sequence that encodes the Vesicular Stomatitis virus G (VSV-G) protein. A representative sequence for the VSV-G protein is provided as SEQ ID NO: 103 (from GenBank Accession No. M35214). Some cells are transformed with a vector comprising a sequence encoding the native signal peptide sequence associated with the VSV-G protein. Other cells are transformed with a vector comprising a sequence encoding a different signal peptide sequence (such as, for example, a *Schizochytrium* signal anchor sequence) that is fused to the sequence encoding the VSV-G protein, such that the VSV-G protein is expressed with a heterologous signal peptide sequence. Other cells are transformed with a vector comprising a sequence encoding a different membrane domain (such as, for example, a HA membrane domain) that is fused to the sequence encoding the VSV-G protein, such that the VSV-G protein is expressed with a heterologous membrane domain. Transformation is performed, and cryostocks are grown and propogated according to any of the methods described herein. *Schizochytrium* cultures are transferred to 50 mL conical tubes and centrifugated at 3000 × g or 4500 × g for 15 min to yield a low-speed supernatant. The low-speed supernatant is further ultracentrifugated at 100,000 × g for 1 h. *See* **FIG. 3****.** The resulting pellet of the insoluble fraction containing the VSV-G protein is resuspended in phosphate buffer saline (PBS) and used for peptide sequence analysis as well as glycosylation analysis as described in Example 2.

The expression of the VSV-G protein from transgenic *Schizochytrium* is verified by immunoblot analysis following standard immunoblotting procedure as described in Example 2, using anti- VSV-G antiserum and a secondary antibody at appropriate dilutions. The recombinant VSV-G protein is detected in the low-speed supernatant and the insoluble fraction. Additionally, the recombinant VSV-G protein is detected in cell-free extracts from transgenic *Schizochytrium* exprsesing the VSV-G protein.

The activity of the VSV-G protein produced in *Schizochytrium* is evaluated by a VSV-G activity assay. A functional VSV-G protein displays an VSV-G activity that is readily detected by a standard VSV-G activity assay.

Electron microscopy, using negative-stained material produced according to Example 4, is performed to confirm the presence of extracellular bodies. Immunogold labeling is performed to confirm the association of protein with extracellular membrane bodies.

### EXAMPLE 12

### Expression and Characterization of Extracellular Bodies Comprising eGFP Fusion Proteins Produced in Schizochytrium

Transformation of *Schizochytrium* sp. ATCC 20888 with vectors comprising a polynucleotide sequence encoding eGFP and expression of eGFP in transformed *Schizochytrium* has been described. *See* U.S. Publ. No. 2010/0233760 and WO 2010/107709.

In a future experiment, *Schizochytrium* sp. ATCC 20888 is used as a host cell for transformation with a vector comprising a sequence that encodes a fusion protein between eGFP and a membrane domain, such as, for example, a membrane domain from *Schizochytrium* or a viral membrane domain such as the HA membrane domain. Representative *Schizochytrium* membrane domains are provided in **FIG. 13** and **FIG. 14****.** Transformation is performed, and cryostocks are grown and propogated according to any of the methods described herein. *Schizochytrium* cultures are transferred to 50 mL conical tubes and centrifugated at 3000 × g or 4500 × g for 15 min to yield a low-speed supernatant. The low-speed supernatant is further ultracentrifugated at 100,000 × g for 1 h. *See* **FIG. 3****.** The resulting pellet of the insoluble fraction containing the eGFP fusion protein from transgenic *Schizochytrium* is resuspended in phosphate buffer saline (PBS) and used for peptide sequence analysis as well as glycosylation analysis as described in Example 2.

The expression of the eGFP fusion protein from transgenic *Schizochytrium* is verified by immunoblot analysis following standard immunoblotting procedure as described in Example 2, using anti-eGFP fusion protein antiserum and a secondary antibody at appropriate dilutions. The recombinant eGFP fusion protein is detected in the low-speed supernatant and the insoluble fraction. Additionally, the recombinant eGFP fusion protein is detected in cell-free extracts from transgenic *Schizochytrium* expressing the eGFP fusion protein.

The activity of the eGFP fusion protein produced in *Schizochytrium* is evaluated by a eGFP fusion protein activity assay. A functional eGFP fusion protein displays an eGFP fusion protein activity that is readily detected by a standard eGFP fusion protein activity assay.

Electron microscopy, using negative-stained material produced according to Example 4, is performed to confirm the presence of extracellular bodies. Immunogold labeling is performed to confirm the association of protein with extracellular membrane bodies.

### EXAMPLE 13

### Detection of Heterologous Polypeptides Produced in Thraustochytrid Cultures

A culture of a thraustochytrid host cell is prepared comprising at least one heterologous polypeptide in a fermentor under appropriate fermentation conditions. The fermentor is batched with a media containing, for example, carbon (glucose), nitrogen, phosphorus, salts, trace metals, and vitamins. The fermentor is inoculated with a typical seed culture, then cultivated for 72 - 120 hours, and fed a carbon (e.g., glucose) feed. The carbon feed is fed and consumed throughout the fermentation. After 72 - 120 hours, the fermentor is harvested and the broth is centrifuged to separate the biomass from the supernatant.

The protein content is determined for the biomass and the cell-free supernatant by standard assays such as Bradford or BCA. Proteins are further analyzed by standard SDS-PAGE and Western blotting to determine the expression of the heterologous polypeptide(s) in the respective biomass and cell-free supernatant fractions. The heterologous polypeptide(s) comprising membrane domains are shown to be associated with microalgal extracellular bodies by routine staining procedures (e.g., negative staining and immunogold labeling) and subsequent electron microscope observations.

### EXAMPLE 14

### Preparation of Virus-Like Particles from Microalgal Cultures

One or more viral envelope polypeptides are heterologously expressed in a microalgal host cell under conditions described above, such that the viral polypeptides are localized to microalgal extracellular bodies produced under the culture conditions. When overexpressed using appropriate culture conditions and regulatory control elements, the viral envelope polypeptides in the microalgal extracellular bodies spontaneously self-assemble into particles that are morphologically similar to infectious virus.

Similarly, one or more viral envelope polypeptides and one or more viral matrix polypeptides are heterologously expressed in a microalgal host cell under conditions described above, such that the viral polypeptides are localized to microalgal extracellular bodies produced under the culture conditions. When overexpressed using appropriate culture conditions and regulatory control elements, the viral polypeptides in the microalgal extracellular bodies spontaneously self-assemble into particles that are morphologically similar to infectious virus.

All of the various aspects, embodiments, and options described herein can be combined in any and all variations.

## Claims

1. A method of producing a heterologous membrane protein comprising a transmembrane domain, comprising:
providing a culture comprising a recombinant microalgal host cell comprising a heterologous nucleic acid encoding a heterologous membrane protein comprising a transmembrane domain;
culturing the recombinant microalgal host cell under conditions sufficient to produce a microalgal extracellular body comprising the heterologous membrane protein comprising a transmembrane domain; and
recovering the microalgal extracellular body comprising the heterologous membrane protein comprising a transmembrane domain from the culture medium in which the microalgal host cell is grown;
wherein the extracellular body is discontinuous with a plasma membrane of the host cell.

2. The method of claim 1, wherein the microalgal host cell is a thraustochytrid cell, and wherein the produced heterologous membrane protein comprising a transmembrane domain has a glycosylation pattern characteristic of expression in a thraustochytrid cell.

3. The method of claim 1 or claim 2, wherein the membrane protein is a viral envelope protein selected from a hemagglutinin (HA) protein, neuraminidase (NA) protein, a fusion (F) protein, a glycoprotein (G) protein, an envelope (Env) protein, a glycoprotein of 120 kDa (gp120), a glycoprotein of 41 kDa (gp41), and combinations thereof.

4. The method of claim 3, wherein the viral envelope protein is an influenza HA protein.

5. An isolated microalgal extracellular body comprising a heterologous membrane protein comprising a transmembrane domain.

6. The isolated microalgal extracellular body of claim 5, wherein the microalgal extracellular body is a thraustochytrid extracellular body, and wherein the heterologous membrane protein comprising a transmembrane domain has a thraustochytrid glycosylation pattern.

7. The isolated microalgal extracellular body of claim 5 or claim 6, wherein the membrane protein is a viral envelope protein selected from a hemagglutinin (HA) protein, neuraminidase (NA) protein, a fusion (F) protein, a glycoprotein (G) protein, an envelope (Env) protein, a glycoprotein of 120 kDa (gp120), a glycoprotein of 41 kDa (gp41), and combinations thereof.

8. The isolated microalgal extracellular body of claim 7, wherein the viral envelope protein is an influenza HA protein.

9. A composition comprising microalgal extracellular bodies according to any one of claims 5 to 8.

10. A vaccine composition comprising microalgal extracellular bodies according to any one of claims 5 to 8, wherein said microalgal extracellular bodies are:
(i) recovered from the culture medium; or
(ii) purified.

11. The vaccine composition according to claim 10, further comprising a pharmaceutically acceptable excipient and/or aqueous liquid carrier.

12. The vaccine composition according to claim 10 or claim 11 for use in vaccinating a subject.

## Patentansprüche

1. Verfahren zur Erzeugung eines eine Transmembrandomäne umfassenden heterologen Membranproteins, umfassend:
Bereitstellen einer Kultur, die eine rekombinante Mikroalgenwirtszelle umfasst, welche eine heterologe Nukleinsäure umfasst, die ein eine Transmembrandomäne umfassendes heterologes Membranprotein codiert;
Kultivieren der rekombinanten Mikroalgenwirtszelle unter hinreichenden Bedingungen, so dass ein extrazelluläres Mikroalgenkörperchen erzeugt wird, das das eine Transmembrandomäne umfassende heterologe Membranprotein umfasst; und
Gewinnen des extrazellulären Mikroalgenkörperchens, das das eine Transmembrandomäne umfassende heterologe Membranprotein umfasst, aus dem Kulturmedium, in dem die Mikroalgenwirtszelle kultiviert wird;
wobei das extrazelluläre Körperchen keine Fortsetzung einer Plasmamembran der Wirtszelle darstellt.

2. Verfahren nach Anspruch 1, wobei es sich bei der Mikroalgenwirtszelle um eine Thraustochytrium-Zelle handelt und wobei das erzeugte eine Transmembrandomäne umfassende heterologe Membranprotein ein Glykosylierungsmuster aufweist, das für die Expression in einer Thraustochytrium-Zelle charakteristisch ist.

3. Verfahren nach Anspruch 1 oder Anspruch 2, wobei es sich bei dem Membranprotein um ein Virushüllprotein ausgewählt aus einem Hämagglutinin(HA)-Protein, Neuraminidase(NA)-Protein, einem Fusion(F)-Protein, einem Glycoprotein(G)-Protein, einem Env(Envelope)-Protein, einem Glycoprotein von 120 kDa (gp120), einem Glycoprotein von 41 kDa (gp41) und Kombinationen davon handelt.

4. Verfahren nach Anspruch 3, wobei es sich bei dem Virushüllprotein um ein Influenza-HA-Protein handelt.

5. Isoliertes extrazelluläres Mikroalgenkörperchen, umfassend ein eine Transmembrandomäne umfassendes heterologes Membranprotein.

6. Isoliertes extrazelluläres Mikroalgenkörperchen nach Anspruch 5, wobei es sich bei dem extrazellulären Mikroalgenkörperchen um ein extrazelluläres Thraustochytrium-Körperchen handelt und wobei das eine Transmembrandomäne umfassende heterologe Membranprotein ein Thraustochytrium-Glykosylierungsmuster aufweist.

7. Isoliertes extrazelluläres Mikroalgenkörperchen nach Anspruch 5 oder Anspruch 6, wobei es sich bei dem Membranprotein um ein Virushüllprotein ausgewählt aus einem Hämagglutinin(HA)-Protein, Neuraminidase(NA)-Protein, einem Fusion(F)-Protein, einem Glycoprotein(G)-Protein, einem Env(Envelope)-Protein, einem Glycoprotein von 120 kDa (gp120), einem Glycoprotein von 41 kDa (gp41) und Kombinationen davon handelt.

8. Isoliertes extrazelluläres Mikroalgenkörperchen nach Anspruch 7, wobei es sich bei dem Virushüllprotein um ein Influenza-HA-Protein handelt.

9. Zusammensetzung, umfassend extrazelluläre Mikroalgenkörperchen gemäß einem der Ansprüche 5 bis 8.

10. Impfstoffzusammensetzung, umfassend extrazelluläre Mikroalgenkörperchen gemäß einem der Ansprüche 5 bis 8, wobei die extrazellulären Mikroalgenkörperchen:
(i) aus dem Kulturmedium gewonnen sind; oder
(ii) aufgereinigt sind.

11. Impfstoffzusammensetzung gemäß Anspruch 10, ferner umfassend einen pharmazeutisch akzeptablen Exzipienten und/oder wässrigen flüssigen Träger.

12. Impfstoffzusammensetzung gemäß Anspruch 10 oder Anspruch 11 zur Verwendung beim Impfen eines Individuums.

## Revendications

1. Procédé de production d'une protéine membranaire hétérologue comprenant un domaine transmembranaire, comprenant :
la mise à disposition d'une culture comprenant une cellule hôte de microalgue recombinante comprenant un acide nucléique hétérologue codant pour une protéine membranaire hétérologue comprenant un domaine transmembranaire ;
la culture de la cellule hôte de microalgue recombinante dans des conditions suffisantes pour produire un corps extracellulaire de microalgue comprenant la protéine membranaire hétérologue comprenant un domaine transmembranaire ; et
la récupération du corps extracellulaire de microalgue comprenant la protéine membranaire hétérologue comprenant un domaine transmembranaire depuis le milieu de culture dans lequel la cellule hôte de microalgue est cultivée ;
le corps extracellulaire étant discontinu avec une membrane plasmatique de la cellule hôte.

2. Procédé selon la revendication 1, la cellule hôte de microalgue étant une cellule de thraustochytride et la protéine membranaire hétérologue produite comprenant un domaine transmembranaire possédant un motif de glycosylation caractéristique d'une expression dans une cellule de thraustochytride.

3. Procédé selon la revendication 1 ou la revendication 2, la protéine membranaire étant une protéine d'enveloppe virale choisie parmi une protéine de type hémagglutinine (HA), une protéine de type neuraminidase (NA), une protéine de fusion (F), une protéine de type glycoprotéine (G), une protéine d'enveloppe (Env), une glycoprotéine de 120 kDa (gp120), une glycoprotéine de 41 kDa (gp41), et des combinaisons correspondantes.

4. Procédé selon la revendication 3, la protéine d'enveloppe virale étant une protéine HA de grippe.

5. Corps extracellulaire de microalgue isolé comprenant une protéine membranaire hétérologue comprenant un domaine transmembranaire.

6. Corps extracellulaire de microalgue isolé selon la revendication 5, le corps extracellulaire de microalgue étant un corps extracellulaire de thraustochytride, et la protéine membranaire hétérologue comprenant un domaine transmembranaire possédant un motif de glycosylation de thraustochytride.

7. Corps extracellulaire de microalgue isolé selon la revendication 5 ou la revendication 6, la protéine membranaire étant une protéine d'enveloppe virale choisie parmi une protéine de type hémagglutinine (HA), une protéine de type neuraminidase (NA), une protéine de fusion (F), une protéine de type glycoprotéine (G), une protéine d'enveloppe (Env), une glycoprotéine de 120 kDa (gp120),une glycoprotéine de 41 kDa (gp41), et des combinaisons correspondantes.

8. Corps extracellulaire de microalgue isolé selon la revendication 7, la protéine d'enveloppe virale étant une protéine HA de grippe.

9. Composition comprenant des corps extracellulaires de microalgue selon l'une quelconque des revendications 5 à 8.

10. Composition de vaccin comprenant des corps extracellulaires de microalgue selon l'une quelconque des revendications 5 à 8, lesdits corps extracellulaires de microalgue étant :
(i) récupérés depuis le milieu de culture ; ou
(ii) purifiés.

11. Composition de vaccin selon la revendication 10, comprenant en outre un excipient et/ou support liquide aqueux pharmaceutiquement acceptable.

12. Composition de vaccin selon la revendication 10 ou la revendication 11 pour une utilisation dans la vaccination d'un sujet.
